Europäisches Patentamt

European Patent Office

Office européen des brevets

(11) Publication number: **0 209 568 B1**

# EUROPEAN PATENT SPECIFICATION

(45) Date of publication of patent specification: **20.07.94**　(51) Int. Cl.5: **C12P 21/00**, C12N 15/12

(21) Application number: **86900900.1**

(22) Date of filing: **10.01.86**

(86) International application number:
**PCT/US86/00027**

(87) International publication number:
**WO 86/04094 (17.07.86 86/17)**

(54) DNA SEOUENCES, RECOMBINANT DNA MOLECULES AND PROCESSES FOR PRODUCING HUMAN LIPOCORTIN-LIKE POLYPEPTIDES.

(30) Priority: **10.01.85 US 690146**
**15.03.85 US 712376**
**14.08.85 US 765877**
**05.09.85 US 772892**

(43) Date of publication of application:
**28.01.87 Bulletin 87/05**

(45) Publication of the grant of the patent:
**20.07.94 Bulletin 94/29**

(84) Designated Contracting States:
**AT BE CH DE FR GB IT LI LU NL SE**

(56) References cited:
**WO-A-86/06100**
**US-A- 4 239 780**
**US-A- 4 537 858**

**Genetic Engineering News, vol. 5, no. 8, September 1985, page 12, New York, US; P. Dimond**

(73) Proprietor: **BIOGEN, INC.**
**14 Cambridge Center**
**Cambridge Massachusetts 02142(US)**

(72) Inventor: **WALLNER, Barbara, P.**
**7 Centre Street**
**Cambridge, MA 02139(US)**
Inventor: **PEPINSKY, Blake, R.**
**69 Parker Street, Apt. 2**
**Watertown, MA 02172(US)**
Inventor: **GARWIN, Jeffrey, L.**
**76 Fletcher Road**
**Bedford, MA 01730(US)**
Inventor: **SCHINDLER, Daniel, G.**
**36 Monastery Road**
**Brighton, MA 02135(US)**
Inventor: **HUANG, Kuo-Seng**
**12 Stevens Road**
**Lexington, MA 02173(US)**

Hattori et al. (1983), Biochem. Biophys. Res. Commun. III, pp 551-559

Huang, K-S et al. (1986), Cell, vol. 46, pp 191-199

Twigg, A.J. & Sherratt D. (1980), Nature, vol 283, pp 216-218

Errasfa et al., Biochimica et Biophysica Acta, vol. 847, pp. 247-254, 1985

Hirata et al., Biochemical and Biophysical Res. Comm., vol. 118, pp 682-690, 1984

Mancheva, et al., Hoppe-Seyler's Z. Physiol. Chem., vol. 365 (8), pp 885-894, 1984

Morrow, et al., Methods in Enzymology, vol. 68, pp 3-24, Academic Press, 1979

Maniatis, et al., Molecular Cloning, pp 405-414, Cold Spring Harbor Laboratory, 1982

Genetic Engineering Letter, 10th June 1985, p. 4

DiRosa et al., Prostaglandins, vol. 28, pp. 441-442

(74) Representative: **Bannerman, David Gardner et al**
**Withers & Rogers**
**4 Dyer's Buildings**
**Holborn**
**London, EC1N 2JT (GB)**

**Description**

TECHNICAL FIELD OF THE INVENTION

This invention relates to DNA sequences, recombinant DNA molecules and processes for producing at least one human lipocortin.* More particularly, the invention relates to DNA sequences and recombinant DNA molecules that are characterized in that they code for at least one human lipocortin-like polypeptide. Accordingly, hosts transformed with these sequences may be employed in the processes of this invention to produce the human lipocortin-like polypeptides of this invention. These polypeptides possess anti-inflammatory activity and are useful in the treatment of arthritic, allergic, dermatologic, ophthalmic and collagen diseases.

BACKGROUND OF THE INVENTION

Arachidonic acid is an unsaturated fatty acid that is a precursor in the synthesis of compounds, such as prostaglandins, hydroxy-acids and leukotrienes, that are involved in inflammation reac tions. It is released from membrane phospholipids by phospholipase $A_2$ activity. In response to anti-inflammatory agents, such as glucocorticoids, certain cells release proteins that have been characterized in vitro by their ability to inhibit phospholipase $A_2$. Accordingly, by inhibiting arachidonic acid production, lipocortins block the synthesis of prostaglandins and other inflammatory substances, thereby reducing inflammation [F. Hirata et al., "A Phospholipase $A_2$ Inhibitory Protein In Rabbit Neutrophils Induced By Glucocorticoids", Proc. Natl. Acad. Sci. USA, 77, No. 5, pp. 2533-36 (1980)].

To date, several phospholipase $A_2$ inhibitory proteins have been studied. One of them --lipomodulin -- has been characterized as an about 40,000 molecular weight protein that is probably degraded by proteases in the cell to two smaller active species of about 30,000 and 15,000 molecular weight [F. Hirata et al., "Identification Of Several Species Of Phospholipase Inhibitory Protein(s) By Radioimmunoassay For Lipomodulin", Biochem. Biophys. Res. Commun., 109, No. 1, pp. 223-30 (1982)]. Other experimental evidence suggests that two other phospholipase $A_2$ inhibitors, macrocortin (about 15,000 molecular weight) and renocortin (two species with molecular weights of about 15,000 and 30,000 respectively) may also be cleavage products of larger inhibitory proteins such as lipomodulin [J. F. Cloix et al., "Characterization And Partial Purification Of Renocortins: Two Polypeptides Formed In Renal Cells Causing The Anti-Phospholipase-like Action Of Glucocorticoids", Br. J. Pharmac., 79, pp. 313-21 (1983); G. J. Blackwell et al., "Macrocortin: A Polypeptide Causing The Anti-Phospholipase Effect Of Glucocorticoids", Nature, 287, pp. 147-49 (1980)].

Although lipomodulin has been isolated from rabbit neutrophil cells, macrocortin from rat macrophages and renocortin from rat renomedullary interstitial cells, the three proteins exhibit similar biological activities, molecular weights and cross-reactivity with monoclonal antibodies against lipomodulin or macrocortin. Moreover, all are induced by glucocorticoids. Thus, it has been suggested that these phospholipase inhibitory proteins, or lipocortins, are closely related to each other and are produced by cells as a general physiological mechanism of steroid action [B. Rothhut et al., "Further Characterization Of The Glucocorticoid-Induced Anti-phospholipase Protein 'Renocortin'", Biochem. Biophys. Res. Commun., 117, No. 3, pp. 878-84 (1983)].

Recent data have also indicated that the 15,000 molecular weight species of lipomodulin is produced by lymphocytes in response to immunogens and acts as a glycosylation-inhibiting factor, inhibiting the glycosylation of IgE-binding factors and leading to the suppression of the IgE response [T. Uede et al., "Modulation Of The Biologic Activities Of IgE-Binding Factors: I. Identification Of Glycosylation-Inhibitory Factor As A Fragment Of Lipomodulin", J. Immunol., 130, No. 2, pp. 878-84 (1983)].

As a result of their anti-inflammatory activities, lipocortins are useful for the treatment of disorders involving inflammatory processes. Such disorders include arthritic, allergic, dermatologic, ophthalmic and collagen diseases. Furthermore, the use of these proteins to treat inflammation might avoid the disadvantages now associated with present anti-inflammatory compounds.

At present two classes of compounds are being used for anti-inflammatory therapy: corticosteroids and nonsteroidal anti-inflammatory drugs. Corticosteroids are generally disfavored due to the severe side effects that may be associated with their use. These effects include hypertension, gastrointestinal bleeding, muscle weakness, cataracts and convulsions. Thus, nonsteroidal anti-inflammatory compounds are preferred.

---

* Lipocortin is also referred to as phospholipase inhibitor protein. Applicants used the term phospholipase inhibitor protein to refer to lipocortin in patent applications 712,376 and 690,146.

However, these non-steroids may also produce side effects, such as adverse effects on gastric and platelet physiology and on the central nervous system and hematopoiesis. In addition, most non-steroidal anti-inflammatory agents inhibit the production of inflammatory substances via their effect on only one of the two pathways for production of those substances, i.e., either the cyclooxygenase pathway or the lipoxygenase pathway.

In contrast, lipocortins inhibit the production of inflammatory substances via both pathways. Furthermore, because lipocortins are only mediators of steroid action, it is unlikely that they will produce the side effects often associated with the use of corticosteroids. And because these inhibitor proteins are natural mediators produced by the cell, they are unlikely to have the side effects usually associated with many non-steroid anti-inflammatories.

In addition, lipocortin may affect inflammation through an alternate pathway by blocking the chemotactic response of leukocytes. It has been shown that certain peptides whose sequences code for the tyrosine phosphorylation sites found within pp60 src and MT antigen, i.e., the src peptide and the MT peptide, inhibit chemotaxis of rabbit neutrophils stimulated by a chemoattractant [F. Hirata et al., "Inhibition Of Leukocyte Chemotaxis By Glu-Glu-Glu-Glu-Tyr-Pro-Met-Glu And Leu-Ile-Glu-Asp-Asn-Glu-Tyr-Thr-Ala-Arg-Gln-Gly", Biochem. Biophys. Res. Comm., 18, pp. 682-90 (1984)]. Lipocortin contains a similar sequence within its amino acid sequence (GluAsnGluGlu GlnGluTyr, residue number 15-21). Therefore, lipocortin may exert its anti-inflammatory effect via this sequence by inhibiting or blocking the movement of neutrophils and macrophages into inflamed tissue.

To date, however, human lipocortins have not been purified from cells. Furthermore, even if a procedure could be developed for the purification of lipocortins, it is doubtful that sufficient quantities of them could be produced for their many clinical and commercial applications. Accordingly, processes enabling the production of human lipocortins in clinically useful amounts would be highly advantageous in anti-inflammatory therapy.

According to the invention there is provided a recombinant DNA sequence as claimed in any one of claims 1 to 9.

In another aspect the invention provides a transformed unicellular host as claimed in claim 10 or 11.

In a further aspect the invention provides a polypeptide as claimed in any one of claims 12 to 16.

The invention also provides a DNA sequence as claimed in claim 17 or 18.

In addition, the invention provides a method as claimed in claim 19 and a pharmaceutical composition as claimed in claim 20 or 21.

The DNA sequences of this invention are selected from the group consisting of the cDNA inserts of λLC (ATCC Deposit No. 68812), DNA sequences which hybridize to this cDNA insert and which code on expression for a human lipocortin-like polypeptide, and DNA sequences which encode a human polypeptide having phospholipase $A_2$ inhibitory activity and an immunological activity of the human lipocortin encoded by λLC insert (ATCC Deposit No. 68812) and DNA sequences that are degenerate as a result of the genetic code as to the DNA insert and sequences described above. Recombinant DNA molecules containing these DNA sequences, hosts transformed with them and human lipocortin-like polypeptides coded for on expression by them are also part of this invention.

The DNA sequences, recombinant DNA molecules, hosts and processes of this invention enable the production of human lipocortin-like polypeptides for use in the treatment of arthritic, allergic, dermatologic, ophthalmic and collagen diseases, as well as other diseases, involving inflammation processes.

BRIEF DESCRIPTION OF THE DRAWINGS

Figure 1 depicts the amino acid sequences of fragments obtained from a cyanogen bromide digestion of rat phospholipase $A_2$ inhibitor protein.

Figure 2 depicts the amino acid sequences of fragments obtained from tryptic digestion of rat phospholipase $A_2$ inhibitor protein.

Figure 3 shows the four pools of chemically synthesized oligonucleotide DNA probes used to screen for the lipocortin DNA sequences of the invention.

Figure 4 displays the nucleotide sequence of the cDNA insert of λLC (ATCC Deposit No.68812) which codes for human lipocortin. The figure also depicts the predicted amino acid sequence of the lipocortin protein. The numbers indicated below the amino acid sequence (e.g., 1, 30 and 346) represent the first, thirtieth and last amino acids of the protein based upon the coding sequence of the gene.

Figure 5 depicts in schematic outline the construction of plasmid pKK233.LIP.1 used to express in one embodiment the DNA sequences of the invention.

4

Figure 6 depicts in schematic outline the construction of plasmid pLiptrc155A (ATCC Deposit No. 68763 ) used to express in one embodiment the DNA sequences of the invention.

Figures 7-9 depict the construction of plasmid pBg120 (ATCC Deposit No.74106), a yeast expression vector for production of human lipocortin according to one embodiment of this invention.

Figure 10 depicts an SDS-polyacrylamide gel analysis of crude yeast lysates. Lane a contains purified human lipocortin made in E.coli according to this invention. Lane b contains extracts from yeast with no plasmid. Lane d contains extracts from yeast containing pBgl20. Lanes c and e contain extracts from yeast containing other plasmids without DNA sequences encoding human lipocortin.

Figures 11-13 depict the construction of plasmid pSVL9109, a mammalian expression vector for production of human lipocortin according to one embodiment of this invention.

Figure 14 depicts an SDS-polyacrylamide gel analysis of plasmin digested human lipocortinlike polypeptide. Lanes a-f represent samples taken at 0, 5, 10, 20, 40 and 120 minutes, respectively, after addition of plasmin.

Figure 15 depicts the phospholipase $A_2$ inhibitory activity of the plasmin fragments of human lipocortin-like polypeptide after fractionation by Biogel P-60 column chromatography.

Figure 16 depicts the amino acid sequences of fragments obtained from tryptic digestion of human lipocortin-like polypeptide.

Figure 17 depicts the amino acid sequences of fragments obtained from tryptic digestion of Lipo-S and Lipo-L.

Figure 18 depicts the phospholipase $A_2$ inhibitory activity of the elastase fragments of human lipocortin-like polypeptide after separation by SDS polyacrylamide gel electrophoresis.

Figure 19 depicts the amino acid sequences of fragments obtained by tryptic digestion of e-1, e-4 and e-5.

Figure 20 depicts the mutagenic oligonucleotides utilized according to the methods of this invention for the production of biologically active lipocortin-like polypeptide fragments by recombinant DNA techniques.

Figure 21 is a schematic line map of the lipocortin amino acid sequence indicating by numbers the sites along the sequence where methionines are located. The bracketed numbers indicate sites where methionines can be introduced to yield fragments according to this invention. Several fragments of this invention and their molecular weights are depicted below the map.

Figure 22 depicts an SDS-polyacrylamide gel analysis of untreated and cyanogen bromide-treated human lipocortin-like polypeptide extracted from Lipo 8, showing a 26 Kd fragment.

Figure 23 depicts, in Panel A, the absorbance profile at 280 nm of lipocortin (peak I) and N-lipocortin (peak II) after fractionation via Mono S high resolution cation exchange chromatography. Panel B depicts the phospholipase $A_2$ inhibitory activity of the eluted lipocortin and N-lipocortin of the invention in % inhibition.

Figure 24 depicts the tryptic maps of the lipocortin and N-lipocortin proteins isolated according to the methods of this invention.

Figure 25 depicts the tryptic map of the N-lipocortin of the invention and correlates the peaks of the map with the amino acid sequences of peptide fragments contained in those peaks.

Figure 26 depicts the four pools of chemically synthesized oligonucleotide DNA probes used to screen for the N-lipocortin DNA sequences of the invention.

Figure 27 displays the nucleotide sequence of the cDNA insert of pNLipl.

Figure 28 compares the nucleotide sequence of lipocortin and the N-lipocortin cDNA sequences of this invention. The "X" on the figure indicates the end of the coding sequence and the start of the 3' non-coding sequence of the genes.

DETAILED DESCRIPTION OF THE INVENTION

In order that the invention herein described may be more fully understood, the following detailed description is set forth.

In the description the following terms are employed:

Nucleotide--A monomeric unit of DNA or RNA consisting of a sugar moiety (pentose), a phosphate, and a nitrogenous heterocyclic base. The base is linked to the sugar moiety via the glycosidic carbon (1' carbon of the pentose) and that combination of base and sugar is called a nucleoside. The base characterizes the nucleotide. The four DNA bases are adenine ("A"), guanine ("G"), cytosine ("C"), and thymine ("T"). The four RNA bases are A, G, C, and uracil ("U").

DNA Sequence--A linear array of nucleotides connected one to the other by phosphodiester bonds between the 3' and 5' carbons of adjacent pentoses.

5

Codon--A DNA sequence of three nucleotides (a triplet) which encodes through mRNA an amino acid, a translation start signal or a translation termination signal. For example, the nucleotide triplets TTA, TTG, CTT, CTC, CTA and CTG encode for the amino acid leucine ("Leu"), TAG, TAA and TGA are translation stop signals and ATG is a translation start signal.

Reading Frame--The grouping of codons during the translation of mRNA into amino acid sequences. During translation the proper reading frame must be maintained. For example, the DNA sequence GCTGGTTGTAAG may be expressed in three reading frames or phases, each of which affords a different amino acid sequence:

$$\underline{GCT}\ \underline{GGT}\ \underline{TGT}\ \underline{AAG}\text{--Ala-Gly-Cys-Lys}$$

$$G\ \underline{CTG}\ \underline{GTT}\ \underline{GTA}\ AG\text{--Leu-Val-Val}$$

$$GC\ \underline{TGG}\ \underline{TTG}\ \underline{TAA}\ G\text{--Trp-Leu-(STOP)}$$

Polypeptide--A linear array of amino acids connected one to the other by peptide bonds between the $\alpha$-amino and carboxy groups of adjacent amino acids.

Peptidase--An enzyme which hydrolyzes peptide bonds.

Genome--The entire DNA of a cell or a virus. It includes inter alia the structural gene coding for the polypeptides of the substance, as well as operator, promoter and ribosome binding and interaction sequences, including sequences such as the Shine-Dalgarno sequences.

Gene--A DNA sequence which encodes through its template or messenger RNA ("mRNA") a sequence of amino acids characteristic of a specific polypeptide.

Transcription--The process of producing mRNA from a gene or DNA sequence.

Translation--The process of producing a polypeptide from mRNA.

Expression--The process undergone by a gene or DNA sequence to produce a polypeptide. It is a combination of transcription and translation.

Plasmid--A nonchromosomal double-stranded DNA sequence comprising an intact "replicon" such that the plasmid is replicated in a host cell. When the plasmid is placed within a unicellular organism, the characteristics of that organism may be changed or transformed as a result of the DNA of the plasmid. For example, a plasmid carrying the gene for tetracycline resistance (TET$^R$) transforms a cell previously sensitive to tetracycline into one which is resistant to it. A cell transformed by a plasmid is called a "transformant".

Phage or Bacteriophage--Bacterial virus many of which consist of DNA sequences encapsidated in a protein envelope or coat ("capsid").

Cosmid--A plasmid containing the cohesive end ("cos") site of bacteriophage λ. Cosmids may, because of the presence of the cos site, be packaged into λ coat protein and used to infect an appropriate host. Because of their capacity for large fragments of foreign DNA, cosmids are useful as cloning vehicles.

Cloning Vehicle--A plasmid, phage DNA, cosmid or other DNA sequence which is able to replicate in a host cell, characterized by one or a small number of endonuclease recognition sites at which such DNA sequences may be cut in a determinable fashion without attendant loss of an essential biological function of the DNA, e.g., replication, production of coat proteins or loss of promoter or binding sites, and which contain a marker suitable for use in the identification of transformed cells, e.g., tetracycline resistance or ampicillin resistance. A cloning vehicle is often called a vector.

Cloning--The process of obtaining a population of organisms or DNA sequences derived from one such organism or sequence by asexual reproduction.

Recombinant DNA Molecule or Hybrid DNA--A molecule consisting of segments of DNA from different genomes which have been joined end-to-end outside of living cells and able to be maintained in living cells.

Expression Control Sequence--A sequence of nucleotides that controls and regulates expression of genes when operatively linked to those genes. They include the lac system, the β-lactamase system, the trp system, the tac and trc systems, the major operator and promoter regions of phage λ, the control region of fd coat protein, the early and late promoters of SV40, promoters derived from polyoma virus and adenovirus, metallothionine promoters, the promoter for 3-phosphoglycerate kinase or other glycolytic enzymes, the promoters of acid phosphatase, e.g., Pho5, the promoters of the yeast α-mating factors, and other sequences known to control the expression of genes of prokaryotic or eukaryotic cells and their viruses or combinations thereof. For mammalian cells the gene can be linked to a eukaryotic promoter such as that for the SV40 early region coupled to the gene encoding dihydrofolate reductase and selectively

6

amplified in Chinese hamster ovary cells to produce a cell line containing many copies of actively transcribed eukaryotic genes.

Lipocortin-Like Polypeptide--A polypeptide displaying a biological or immunological activity of a lipocortin. This polypeptide may include amino acids in addition to those of a native lipocortin or it may not include all of the amino acids of native lipocortin. Finally, it may include an N-terminal methionine. Lipocortin is also referred to as phospholipase inhibitor protein.

The present invention relates to DNA sequences and recombinant DNA molecules coding for human lipocortin-like polypeptides according to claims 1-9 and processes for the production of those polypeptides.

Although a variety of selection and DNA cloning techniques might potentially have been employed in our isolating and cloning of a DNA sequence of this invention, in one embodiment of the invention, we adopted a selection strategy based upon rat phospholipase $A_2$ inhibitor protein. Accordingly, we purified a rat phospholipase $A_2$ inhibitor protein from the extracellular supernatant of rat peritoneal exudate cells and determined the amino acid sequence of various fragments of that protein. Based on those protein sequences, we then synthesized several antisense oligonucleotide DNA probes corresponding to those regions of purified rat protein which had minimal nucleotide degeneracy. We then used these probes to screen a human cDNA library comprising E.coli cells containing human macrophage cDNA sequences inserted into a phage cloning vector.

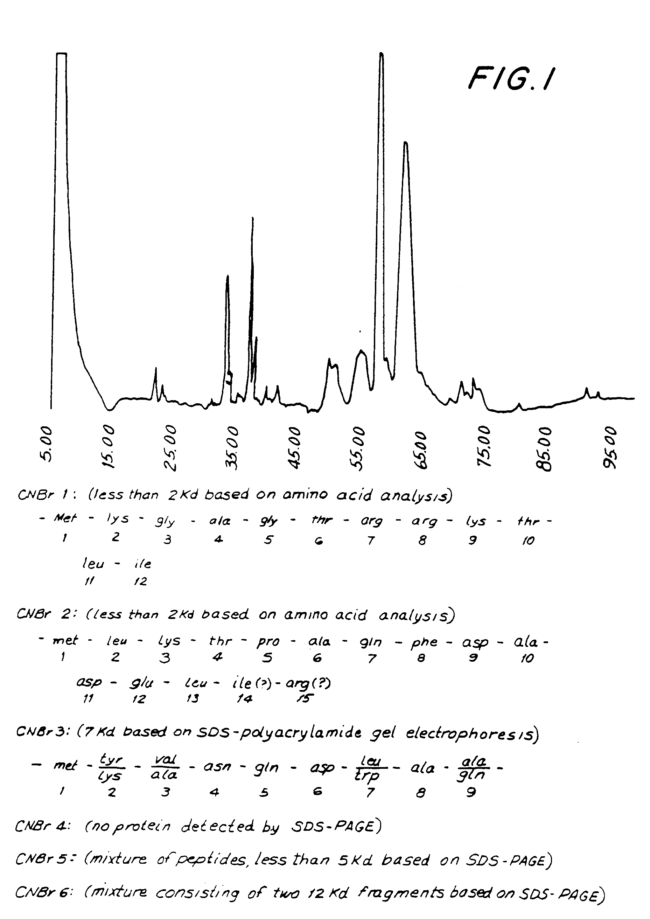

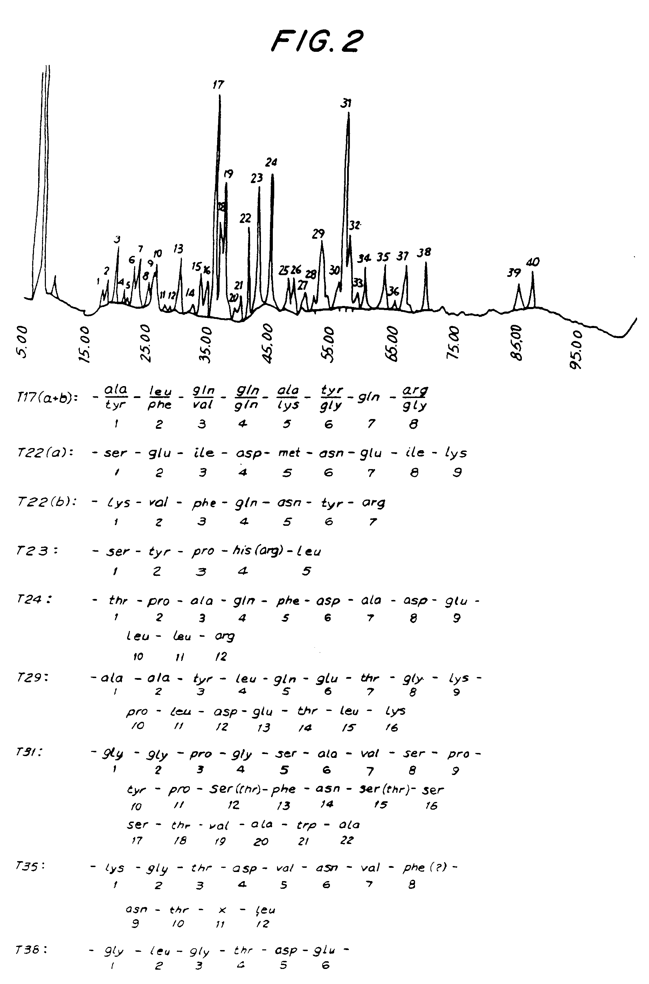

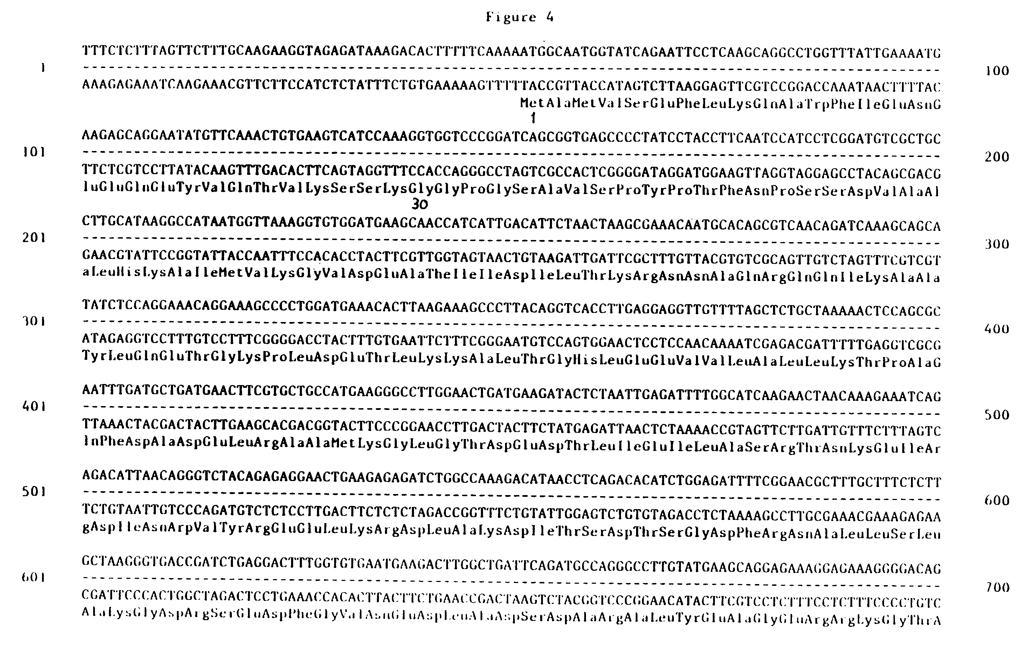

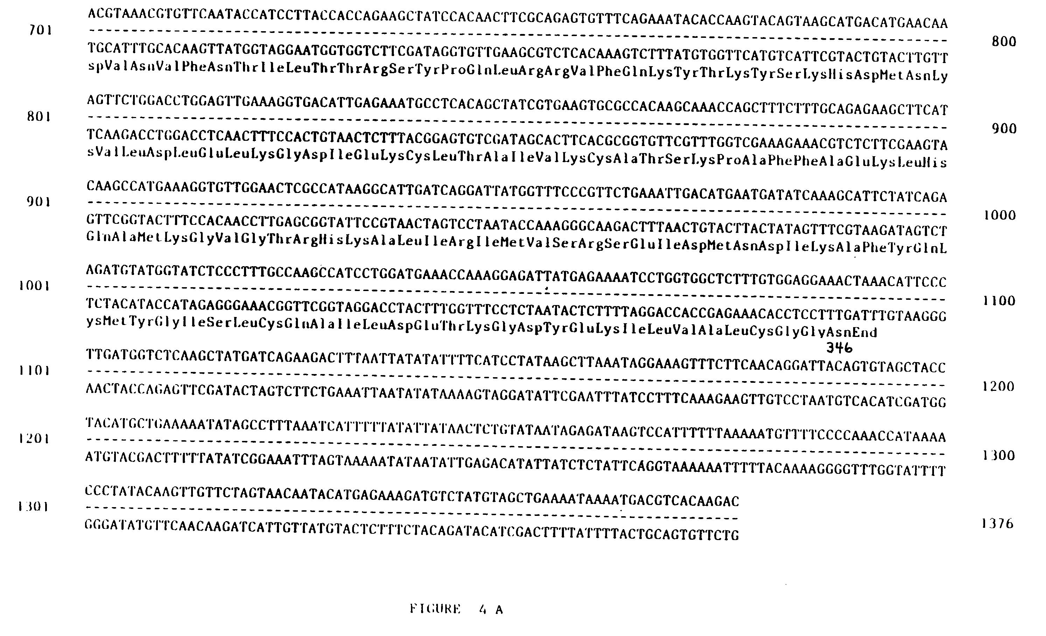

For screening, we hybridized the oligonucleotide probes to the human cDNA library utilizing a plaque hybridization screening assay and we selected clones hybridizing to a number of our probes. After isolating and subcloning the selected human cDNA inserts into plasmids, we determined their nucleotide sequences and compared them to our amino acid sequences from peptides of purified rat lipocortin. As a result of this comparison, we found that the nucleotide sequences of all clones isolated coded for amino acid sequences that had a marked homology to the amino acid sequences of our purified rat lipocortin. (Compare Figures 1 and 2 with Figure 4.) We confirmed that at least one of the clones isolated contained the full length sequence encoding human lipocortin.

The cDNA sequences of this invention can be operatively linked to expression control sequences and used in various mammalian or other eukaryotic or prokaryotic host cells to produce the human lipocortinlike polypeptides coded for by them. For example, we have constructed high level expression vectors for the production of a 37 Kd human lipocortin.

In addition, the cDNA sequences of this invention are useful as probes to screen human cDNA libraries for other sequences coding for lipocortin-like polypeptides. The cDNA sequences of this invention are also useful as probes to screen human genomic DNA libraries to select human genomic DNA sequences coding for lipocortin-like polypeptides. These genomic sequences, like the above cDNA sequences of this invention, are then useful to produce the lipocortin-like polypeptides coded for by them. The genomic sequences are particularly useful in transforming mammalian cells to produce human lipocortin-like polypeptides.

According to a second embodiment of the invention, we isolated from human placenta a human lipocortin-like polypeptide which shows structural similarity, i.e., amino acid homology, to the 37 Kd recombinant lipocortin of this invention. This protein also displays phospholipase $A_2$ inhibitory activity. We have designated the protein, N-lipocortin. Lipocortin and N-lipocortin are discrete proteins that have been defined chemically by tryptic mapping. We determined the amino acid sequences of various fragments of N-lipocortin and based on these sequences, we synthesized antisense oligonucleotide DNA probes. We used these probes to screen a human cDNA library comprising E.coli cells containing human placenta cDNA sequences inserted into a phage cloning vector. We isolated several clones which hybridized to the probes. After introducing the cDNA insert of one of these clones into a plasmid, we determined the nucleotide sequence of the cDNA insert. It codes for a portion of N-lipocortin.

This cDNA sequence is useful as a probe to screen human cDNA libraries for other sequences coding for N-lipocortin-like polypeptides. For example, this cDNA insert can be used to screen the human placenta cDNA library of this invention for the full length sequence encoding N-lipocortin. Alternatively, the oligonucleotide probes that were used to obtain the partial N-lipocortin cDNA sequence of this invention are useful as probes to rescreen the placenta library for the full length sequence encoding N-lipocortin. In addition, the N-lipocortin cDNA sequences obtained or the probes described herein may be used to isolate other portions of the N-lipocortin gene and the full length gene reconstructed by standard techniques in the art. Finally, the probes or cDNA sequences may be used as primers to synthesize full length coding sequences.

The N-lipocortin cDNA sequences of this invention are also useful as probes to screen human genomic DNA libraries to select human genomic DNA sequences coding for N-lipocortin-like polypeptides. These genomic sequences, like the N-lipocortin cDNA sequences, are then useful to produce N-lipocortin-like polypeptides in hosts transformed with those sequences.

Another embodiment of the present invention relates to the production of biologically active human lipocortin-like polypeptide fragments which allow better characterization of the active site of the lipocortin protein and the design of molecules having optimal therapeutic value. Accordingly, we have generated such fragments by treatment of the lipocortin-like polypeptides of this invention with various proteases. We have also generated such biologically active fragments by recombinant DNA techniques.

The human lipocortin-like polypeptides produced by the methods of this invention are useful as anti-inflammatory agents and in anti-inflammatory methods and therapies. For example, such compositions may comprise an amount of a lipocortin-like polypeptide of this invention which is pharmaceutically effective to reduce inflammation and a pharmaceutically acceptable carrier. Such therapies generally comprise a method of treating patients in a pharmaceutically acceptable manner with those compositions.

## METHODS AND MATERIALS

A wide variety of host/cloning vehicle combinations may be employed in cloning or expressing the human lipocortin-like polypeptide DNA sequences prepared in accordance with this invention. For example, useful cloning or expression vehicles may consist of segments of chromosomal, non-chromosomal and synthetic DNA sequences, such as various known derivatives of SV40 and known bacterial plasmids, e.g., plasmids from E.coli including colE1, pCR1, pBR322, pMB9 and their derivatives; wider host range plasmids, e.g., RP4, phage DNAs, e.g., the numerous derivatives of phage λ, e.g., NM 989; and other DNA phages, e.g., M13 and filamentous single-stranded DNA phages and vectors derived from combinations of plasmids and phage DNAs such as plasmids which have been modified to employ phage DNA or other expression control sequences or yeast plasmids such as the 2μ plasmid or derivatives thereof.

Within each specific cloning or expression vehicle, various sites may be selected for insertion of the human lipocortin-like polypeptide DNA sequences of this invention. These sites are usually designated by the restriction endonuclease which cuts them and are well recognized by those of skill in the art. Various methods for inserting DNA sequences into these sites to form recombinant DNA molecules are also well known. These include, for example, dG-dC or dA-dT tailing, direct ligation, synthetic linkers, exonuclease and polymerase-linked repair reactions followed by ligation, or extension of the DNA strand with DNA polymerase and an appropriate single-stranded template followed by ligation. It is, of course, to be understood that a cloning or expression vehicle useful in this invention need not have a restriction endonuclease site for insertion of the chosen DNA fragment. Instead, the vehicle could be joined to the fragment by alternative means.

Various expression control sequences may also be chosen to effect the expression of the DNA sequences of this invention. These expression control sequences include, for example, the lac system, the β-lactamase system, the trp system, the tac system, the trc system, the major operator and promoter regions of phage λ, the control regions of fd coat protein, the promoter for 3-phosphoglycerate kinase or other glycolytic enzymes, the promoters of acid phosphatase, e.g., Pho5, the promoters of the yeast α-mating factors, promoters for mammalian cells such as the SV40 early promoter, adenovirus late promoter and metallothionine promoter, and other sequences known to control the expression of genes of prokaryotic or eukaryotic cells or their viruses and various combinations thereof. In mammalian cells, it is additionally possible to amplify the expression units by linking the gene to that for dihydrofolate reductase and applying a selection to host Chinese hamster ovary cells.

For expression of the DNA sequences of this invention, these DNA sequences are operatively-linked to one or more of the above-described expression control sequences in the expression vector. Such operative linking, which may be effected before or after the chosen human lipocortin DNA sequence is inserted into a cloning vehicle, enables the expression control sequences to control and promote the expression of the DNA sequence.

The vector or expression vehicle and, in particular, the sites chosen therein for insertion of the selected DNA fragment and the expression control sequence employed in this invention, are determined by a variety of factors, e.g., number of sites susceptible to a particular restriction enzyme, size of the protein to be expressed, expression characteristics such as the location of start and stop codons relative to the vector sequences, and other factors recognized by those of skill in the art. The choice of a vector, expression control sequence, and insertion site for a particular lipocortin sequence is determined by a balance of these factors, not all selections being equally effective for a given case.

It should also be understood that the DNA sequences coding for the lipocortin-like polypeptides of this invention which are inserted at the selected site of a cloning or expression vehicle may include nucleotides which are not part of the actual gene coding for the desired lipocortin or may include only a fragment of the entire gene for that protein. It is only required that whatever DNA sequence is employed, a transformed host will produce a lipocortin-like polypeptide. For example, the lipocortin-related DNA sequences of this invention may be fused in the same reading frame in an expression vector of this invention to at least a portion of a DNA sequence coding for at least one eukaryotic or prokaryotic carrier protein or a DNA sequence coding for at least one eukaryotic or prokaryotic signal sequence, or combinations thereof. Such constructions may aid in expression of the desired lipocortin-related DNA sequence, improve purification or permit secretion, and preferably maturation, of the lipocortin-like polypeptide from the host cell. The lipocortin-related DNA sequence may alternatively include an ATG start codon, alone or together with other codons, fused directly to the sequence encoding the first amino acid of a mature native lipocortin-like polypeptide. Such constructions enable the production of, for example, a methionyl or other peptidyl-lipocortin-like polypeptide that is part of this invention. This N-terminal methionine or peptide may then be cleaved intra- or extra-cellularly by a variety of known processes or the polypeptide used together with the methionine attached to the peptide in the anti-inflammatory compositions and methods of this invention.

The cloning vehicle or expression vector containing the lipocortin-like polypeptide coding sequences of this invention is employed in accordance with this invention to transform an appropriate host so as to permit that host to express the lipocortin-like polypeptide for which the DNA sequence codes.

Useful cloning or expression hosts include strains of E.coli, such as E.coli W3110I$^Q$, E.coli JA221, E.coli C600, E.coli ED8767, E.coli DH1, E.coli LE392, E.coli HB101, E.coli X1776, E.coli X2282, E.coli MRCI, and strains of Pseudomonas, Bacillus, and Streptomyces, yeasts and other fungi, animal hosts, such as CHO cells or mouse cells, other animal (including human) hosts, plant cells in culture or other hosts.

The selection of an appropriate host is also controlled by a number of factors recognized by the art. These include, for example, compatibility with the chosen vector, toxicity of proteins encoded by the hybrid plasmid, susceptibility of the desired protein to proteolytic degradation by host cell enzymes, contamination or binding of the protein to be expressed by host cell proteins difficult to remove during purification, ease of recovery of the desired protein, expression characteristics, bio-safety and cost. A balance of these factors must be struck with the understanding that not all host vector combinations may be equally effective for either the cloning or expression of a particular recombinant DNA molecule.

It should be understood that the human lipocortin-like polypeptides (prepared in accordance with this invention in those hosts) may include polypeptides in the form of fused proteins (e.g., linked to a prokaryotic, eukaryotic or combination N-terminal segment to direct excretion, improve stability, improve purification or improve possible cleavage of the N-terminal segment), in the form of a precursor of lipocortin-like polypeptides (e.g., starting with all or parts of a lipocortin-like polypeptide signal sequence or other eukaryotic or prokaryotic signal sequences), in the form of a mature lipocortin-like polypeptide, or in the form of a met-lipocortin-like polypeptide.

One particularly useful form of a polypeptide in accordance with this invention, or at least a precursor thereof, is a mature lipocortin-like polypeptide with an easily cleaved amino acid or series of amino acids attached to the amino terminus. Such construction allows synthesis of the protein in an appropriate host, where a start signal that may not be present in the mature lipocortin is needed, and then cleavage in vivo or in vitro of the extra amino acids to produce mature lipocortin-like polypeptides. Such methods exist in the art. See, e.g., United States patents 4,332,892, 4,338,397, and 4,425,437. The polypeptides may also be glycosylated, like some native lipocortins, unglycosylated, or have a glycosylation pattern different than that of native lipocortins. Such glycosylation will result from the host cell or post-expression treatment chosen for the particular lipocortin.

The polypeptides of this invention also include biologically active polypeptide fragments derived from lipocortin-like polypeptides by treatment with proteases or by expression of a fragment of the DNA sequence which codes for a lipocortin-like polypeptide. The polypeptides of the invention also include lipocortin-like polypeptides that are coded for on expression by DNA sequences characterized by different codons for some or all of the codons of the present DNA sequences. These substituted codons may code for amino acids identical to those coded for by the codons replaced but result in higher yield of the polypeptide. Alternatively, the replacement of one or a combination of codons leading to amino acid replacement or to a longer or shorter lipocortin-like polypeptide may alter its properties in a useful way (e.g., increase the stability, increase the solubility or increase the therapeutic activity).

In order that this invention may be better understood, the following examples are set forth. These examples are for purposes of illustration only and are not to be construed as limiting the scope of the invention in any manner.

EXAMPLES

A. PURIFICATION OF A RAT PHOSPHOLIPASE $A_2$ INHIBITOR PROTEIN

We injected male Wistar rats (200-250 kg) subcutaneously with 0.1 ml of the glucocorticoid, dexamethasone phosphate (1.25 mg/kg rat) in 0.9% NaCl to induce production of phospholipase $A_2$ inhibitor protein. We then sacrificed the rats one hour after injection by intra-cardiac injection with Euthasate and washed the peritoneal cavities with 10 ml of phosphate buffered saline (50 mM $KH_2PO_4$, pH 7.3, 150 mM NaCl containing 2 U/ml heparin and 50 $\mu$M phenylmethylsulfonylfluoride). After we cleared the lavages of cells and other particulate matter by centrifugation in an International centrifuge at top speed for 30 min, we assayed the combined supernatants for lipocortin by measuring the inhibition of release of labeled oleic acid from autoclaved E.coli membranes in the presence of the supernatant and porcine pancreatic phospholipase $A_2$.

We performed this in vitro assay as follows: We mixed 200 $\mu$l samples from the peritoneal exudate supernatant in 1.5 ml Eppendorf tubes with 50 $\mu$l of 0.7 M Tris-HCl (pH 8.0), 60 mM $CaCl_2$ buffer on ice. We then added 50 $\mu$l of diluted porcine pancreatic phospholipase $A_2$ (Catalogue no. P9139, Sigma Chemicals) and mixed and incubated the solutions on ice for 1 h. Dilutions of the phospholipase $A_2$ suspension into buffer (70 mM Tris-HCl (pH 8.0), 6 mM $CaCl_2$) containing 2.5 mg/ml bovine serum albumin (BSA) were such that the final concentrations of phospholipase and BSA were l00 ng/50 $\mu$l and 125 $\mu$g/50 $\mu$l, respectively. We then added 25 $\mu$l of autoclaved $^3$H-oleic acid-labeled E.coli as substrate and incubated the mixtures at 6 °C for 8 min (both the temperature and length of incubation must be determined for each batch of E.coli utilized).

We prepared the substrate $^3$H-oleic acid-labeled E.coli as follows: We grew an overnight culture of E.coli in tryptone medium (l% bactotryptone, 0.5% NaCl), diluted it 1:20 with fresh broth and monitored cell growth with a Klett meter. At a reading of 40 (i.e., when cells were growing well), we added a l:l00 dilution of Brij 35 (polyoxyethylene-23-ether, Sigma Chemicals, l0% solution in water) and a l:200 dilution of $^3$H-oleic acid (9,l0-$^3$H-[N]-oleic acid, New England Nuclear) at l0 mCi/ml. After 5 h, when cell growth leveled off, we autoclaved the suspension for 20 min at l20 °C and stored the flask overnight at 4 °C. We then pelleted the bacteria by centrifugation for 30 min at l6,000 rpm in an SS34 rotor at 4 °C and combined the loose pellets into a single tube. We washed the bacteria four times, or until counts in the supernatant were low, with suspension buffer (0.7 M Tris-HCl (pH 8.0), l0 mM $CaCl_2$) plus 0.1% BSA. We stored the bacteria at 4 °C in suspension buffer containing 0.2% sodium azide. Typically, we prepared a 400 ml culture labeled with 20 mCi of $^3$H-oleic acid. This yielded about $7 \times l0^8$ cpm or about 10% of the input counts in labeled bacteria. For each point in an assay, we used l00,000 cpm, which was added in a volume of 25 $\mu$l. Immediately prior to use, we washed our aliquots first in 200 mM Tris-HCl (pH 8.0), l2 mM EDTA (left on ice 30 min) and then in 25 mM Tris-HCl (pH 8.0).

After the brief incubation of substrate (autoclaved labeled E.coli) with inhibitor plus phospholipase $A_2$, the reaction was stopped immediately by adding 100 $\mu$l of 2 N HCl to each tube followed by the addition of 100 $\mu$l of 20 mg/ml delipidated BSA (99% albumin, Sigma Chemicals). Tubes were vortexed and incubated on ice for 30 min. The latter step was crucial for extracting the lipase digestion products from the particulate membranes.

We then pelleted the E.coli in an Eppendorf centrifuge for 5 min at 10,000 g and counted 250 $\mu$l of each supernatant in 4 ml of a scintillation cocktail compatible with aqueous solutions. In this assay, we tested each sample in duplicate using an internal control in which the sample plus E.coli substrate was incubated both in the presence and absence of added phospholipase. This in vitro assay demonstrated that our peritoneal exudates contained phospholipase inhibitory activity.

To purify the lipocortin from the above-described peritoneal exudate supernatant, we first added additional protease inhibitors to the supernatant. These typically included aprotinin (20 $\mu$g/ml), soybean trypsin inhibitor (20 $\mu$g/ml) and EGTA (ethyleneglycol-bis-(aminoethyl ether) N,N'-tetraacetic acid) (0.5 mM). We incubated the exudate at 37 °C for 1 h in the presence of 0.1 U/ml calf intestinal alkaline phosphatase and concentrated it two-fold by ultrafiltration to a final protein concentration of 5 mg/ml using an Amicon apparatus (PM10 membrane). We next dialyzed the supernatant overnight at 4 °C against 40 volumes of 20 mM Tris-HCl (pH 8.1) and subjected it to DE52 ion exchange column chromatography (Whatman Ltd., column dimensions: 1 cm dia. x 17 cm). Prior to use, we had equilibrated the DE52 resin with 25 mM Tris-HCl (pH 8.1). We collected the flow-through fractions and concentrated them an additional 25-fold by Amicon ultrafiltration (PM10 membrane). We then subjected the concentrate to a gel filtration column (P150 resin) in 25 mM Tris-HCl (column dimensions: 2.5 cm dia. x 40 cm) and monitored the column fractions for protein using absorbance at 280 nm and using the phospholipase inhibitory activity assay described above.

We detected peak activity at 35-40,000 molecular weight.

We lyophilized these high activity fractions, dialyzed them against 25 mM Tris-HCl (pH 6.8) containing 0.2% SDS and analyzed them using a preparative SDS-polyacrylamide gel (main gel: 15% acrylamide, 0.08% methylene bisacrylamide; stacking gel: 7.6% acrylamide, 0.21% methylene bisacrylamide). The gel analysis yielded four major protein bands. According to a modification of the Western blotting technique [H. Towbin et al., "Electrophoretic Transfer Of Proteins From Polyacrylamide Gels To Nitrocellulose Sheets", Proc. Natl. Acad. Sci. USA, 76, pp. 4350-54 (1979)] using a horse radish peroxidase antibody conjugate to visualize the immunoreactive species, we found that only one of the four major bands cross reacted with a neutralizing antibody which we prepared against a snake venom lipocortin. Accordingly, we excised this region of the gel, electroeluted and precipitated the contained protein from it with 20% trichloroacetic acid and pelleted the protein by centrifugation for 20 min at 10,000 g. After washing the pellets twice with 5 ml of -20°C acetone, each washing being followed by a centrifugation step, we dried the pellets under vacuum.

We then digested the protein either with cyanogen bromide or with trypsin. When utilizing cyanogen bromide digestion, we digested the pellets containing approximately 100 $\mu$g protein with 200 mg/ml of cyanogen bromide in the dark for 16 h at 25°C in 0.5 ml of 70% formic acid. We then diluted the reaction mixture 15-fold with water and lyophilized it. When utilizing tryptic digestion, we first resuspended the pellets in 0.1 M $NH_4HCO_3$ plus 0.1 mM $CaCl_2$, carboxymethylated the mixture with iodoacetic acid and then incubated it with trypsin for 24 h at 37°C. During this incubation, we added trypsin three times to a final concentration of 1.5% of total protein at time zero, 2.5% after 4 h and 3.5% after 19 h.

We resolved the cleavage fragments from these digestions by high pressure liquid chromatography using a C8 column (Brownlee RP-3) for the cyanogen bromide digestion products and using a C18 column (Spectraphysics) for the tryptic digestion products, utilizing in both cases a gradient of acetonitrile from 0-75% in 0.l% trifluoroacetic acid to elute bound fragments. We then subjected the peak fractions to sequence analysis using a gas phase sequencer (Applied Biosystems 470A). PITH-amino acids were analyzed by high pressure liquid chromatography on a 5 $\mu$m cyanocolumn (Hypersil), using a gradient of acetonitrile:methanol (4:1) from 15-55% in 0.02 M sodium acetate (pH 5.7).

Figure 1 shows the amino acid sequences of the fragments produced by cyanogen bromide digestion of our purified rat lipocortin. Of six major peaks, only three yielded unique sequences (CNBr l, 2 and 3). These sequences are shown at the bottom of Figure l. Of the remaining peaks, two (CNBr 5 and 6) contained mixtures of fragments and thus could not be sequenced, and peak 4 was a column artifact from which no protein was detected.

Figure 2 shows the amino acid sequences of fragments from tryptic digestion. Although tryptic digestion produced over forty peaks, the amino acid sequences of only nine fractions are shown in Figure 2. In instances where peaks contained more than one peptide, the appropriate fractions were subjected to a second chromatography step. T22a and T22b are sequences derived from the two components of peak 22 which were resolved when peak 22 was rechromatographed on the same column but at a neutral pH.

## B. SYNTHESIS OF OLIGONUCLEOTIDE DNA PROBES FOR LIPOCORTIN PROTEIN SEQUENCES

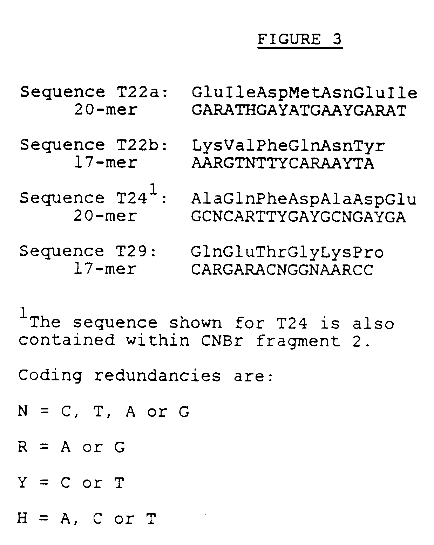

Having determined the amino acid sequences of various regions of a rat lipocortin (see Figures 1 and 2), we chemically synthesized four pools of antisense oligonucleotide DNA probes that coded for some of those protein sequences (see Figure 3). We decided to synthesize the four pools shown in Figure 3 because they corresponded to regions of the rat lipocortin that have minimal nucleic acid degeneracy. For each amino acid sequence, we synthesized mixtures of probes complementary to all possible codons. Furthermore, we synthesized the probes such that they were complementary to the DNA sequences which code for the amino acid sequence, i.e., the probes were antisense, to enable the probes to recognize the corresponding sequences in mRNA as well as in DNA. The amino acid sequences of the four selected regions of the rat lipocortin and all the possible nucleotide codon combinations that encode them are shown in Figure 3. Coding degeneracies are indicated as follows: N = C, T, A, or G; R = A or G; Y = C or T; and H = A, C, or T.

Two pools of the probes, derived from sequences in the tryptic fragments T22a and T24 of Figure 2, are 20-mers with 48 and 256 fold degeneracies, respectively. The other two probe pools are l7-mers with 64 and l28 fold degeneracies. To reduce further the degeneracies in the probes, we also prepared each pool in subpools, e.g., we prepared the 48 fold degenerate 20-mer of T22a in three subpools of l6 and synthesized the other probes in four subpools. The probes in each pool were end-labeled with $^{32}$P using [$\gamma$]-$^{32}$P-ATP and T4 polynucleotide kinase.

To test if our synthetic probes actually recognized human sequences, we hybridized the four subpools of T24 to GeneScreen filters containing poly (A)$^+$ mRNA from the human macrophage cell line U937, which had been induced with $10^{-7}$M PMA[$4\beta$-phorbol $12\beta$ myristate $13\alpha$-acetate] and $10^{-5}$ dexamethasone, utilizing the Northern blotting technique [H. Lehrach et al., Biochemistry, 10, pp. 4743-51 (1977)]. Subpools 2 and 3 of T24 hybridized to the mRNA and were detected as an 1800 base pair band upon autoradiography.

## C. CONSTRUCTION AND SCREENING OF A HUMAN cDNA LIBRARY

We constructed a human cDNA library from poly (A)$^+$ mRNA isolated from human macrophage cell line U937. The cDNA sequences were inserted into λgtl0 and amplified in E.coli C600 hfl cells.

## 1. EXTRACTION OF RNA FROM HUMAN U937 CELLS

We induced human macrophage U937 cells in culture with dexamethasone ($10^{-5}$ M) and phorbol ester ($10^{-7}$ M) and resuspended pellets containing 1.2 x 10$^9$ cells in 48 ml lysis buffer (0.2 M Tris-HCl (pH 8.0), 0.l M LiCl, 25 mM EDTA, l% SDS) plus 5 mM vanadyl complex (Bethesda Research Labs) by vortexing. We lysed the cells by addition of 24 ml phenol and vortexed for 5 min. We added 24 ml chloroform to the lysis mixture which was then shaken for l0 min. We separated the organic and aqueous phases by centrifugation in a clinical centrifuge at room temperature for l0 min. We reextracted the aqueous phase two times with phenol:chloroform (l:l), then two times with chloroform only. We next ethanol-precipitated the nucleic acids in 0.3 M sodium acetate at -20°C overnight and pelleted the nucleic acid at l4k rpm in a Sorvall RC2B centrifuge (SS34 rotor) at 4°C for 20 min. We resuspended the pellets in 5 ml of 0.3 M sodium acetate buffer, and ethanol-precipitated the nucleic acid again as described above. We resuspended the final pellet in 300 $\mu$l H$_2$O and stored it at -20°C. This RNA preparation was enriched for poly(A)$^+$ RNA by passage over an oligo(dT)-cellulose column (PL Biochem).

## 2. CONSTRUCTION OF A U937 cDNA-λgtl0 LIBRARY

cDNA Synthesis

We synthesized cDNA from 20 $\mu$g poly (A)$^+$ mRNA isolated as described above. We diluted the poly (A)$^+$ mRNA to 500 $\mu$g/ml in H$_2$O, heated it to 65°C for 3 min, quick cooled it in a dry ice-propanol bath and then thawed it. The RNA was then added to a reaction mixture composed of 0.l M Tris-HCl (pH 8.3) at 42°C, 0.0l M MgCl$_2$, 0.0l M DTT, l mM dCTP, 1 mM dGTP, 1 mM dTTP, 0.5 mM dATP and l00 $\mu$Ci $\alpha$-$^{32}$P-ATP (3000 Ci/mmole, Amersham or New England Nuclear), 20 $\mu$g oligo (dT)$_{12-18}$ (PL Biochem), 0.03 M $\beta$-mercaptoethanol, 5 mM Vanadyl Ribonucleoside Complex (Bethesda Research Labs), l69 U AMV Reverse Transcriptase (Seikagaku America). Final volume of the reaction mixture was 200 $\mu$l. We incubated this mixture for 2 min at room temperature and 6 h at 44°C. We terminated the reaction by addition of l/l0 vol 0.5 M Na$_2$EDTA (pH 8.0).

We adjusted the reaction mixture to 0.l5 M NaOH and incubated the mixture at 37°C for l2 h followed by neutralization with l/l0 vol l M Tris-HCl (pH 8.0) and HCl. This was extracted with phenol: chloroform saturated TE buffer (10 mM Tris-HCl (pH 7.0) and 1 mM Na$_2$EDTA). The aqueous phase was chromatographed through a 5 ml sterile plastic pipet containing a 7 x 29 cm bed of Sephadex Gl50 in 0.0l M (pH 7.4), 0.4 M NaCl, 0.0l M Na$_2$EDTA, 0.05% SDS. We pooled the front peak minus tail and precipitated the cDNA with 2.5 vol 95% ethanol at -20°C. This reaction yielded l $\mu$g of single-stranded cDNA.

Double Strand Synthesis

We resuspended the single-stranded cDNA in 200 $\mu$l (final vol) 0.l M Hepes (pH 6.9), 0.0l M MgCl$_2$, 0.0025 M DTT, 0.07 M KCl, l mM dXTPs and 75 U Klenow fragment DNA polymerase I (Boehringer-Mannheim) and incubated the reaction mixture at l4°C for 21 h. Reaction was terminated by addition of Na$_2$EDTA (pH 8.0) to 0.0l25 M, extracted with phenol:chloroform, as in the first cDNA step, and the aqueous phase was chromatographed on a G150 column in 0.0l M Tris-HCl (pH 7.4), 0.l M NaCl, 0.0l M Na$_2$EDTA, 0.05% SDS. We again pooled the radioactive peak minus the tail and ethanol-precipitated the DNA.

We then incubated the DNA obtained with 42 U reverse transcriptase in 50 $\mu$l 0.l M Tris-HCl (pH 8.3), 0.0l M MgCl$_2$, 0.0l M DTT, 0.l M KCl, l mM dXTPS, 0.03 M $\beta$-mercaptoethanol for l h at 37°C to complete double-strand synthesis. The reaction was terminated and processed as described above.

We cleaved the hairpin loop formed during double strand synthesis as follows: We redissolved the pellet in 50 $\mu$l 0.03 M sodium acetate (pH 4.5), 0.3 M NaCl, 0.003 M $ZnCl_2$ and treated it with l00 U $S_1$ nuclease (Sigma) for 30 min at room temperature. Reaction was terminated by addition of EDTA and processed as described above. The yield after $S_1$ treatment was 900 ng dsDNA.

To assure blunt ends following $S_1$ nuclease digestion, we treated the DNA with Klenow in 0.0l M Tris-HCl (pH 7.4), 0.0l M $MgCl_2$, l mM DTT, 0.05 M NaCl, 80 $\mu$M dXTP and l2.5 U Klenow in 60 $\mu$l for 90 min at l4°C, extracted with 50:50 phenol:chloroform, and chromatographed the DNA on a G50 spin column (l ml syringe) in 0.0l M Tris-HCl (pH 7.4), 0.l M NaCl, 0.0l M EDTA, 0.05% SDS.

We next methylated the dsDNA by treating the DNA with EcoRI methylase in 30 $\mu$l final vol 0.l M Tris-HCl (pH 8.0), 0.0l M $Na_2$EDTA, 24 $\mu$g BSA, 0.005 M DTT, 30 $\mu$M S-adenosylmethionine and 5 U EcoRI Methylase for 20 min at 37°C. The reaction was heated to 70°C for l0 min, cooled, extracted with 50:50 phenol: chloroform and chromatographed on a G50 spin column as described above.

We ligated the 900 ng cDNA to phosphorylated EcoRI linkers (New England Biolabs) using the following conditions: 0.05 M Tris-HCl (pH 7.8), 0.01 M $MgCl_2$, 0.02 M DTT, l mM ATP, 50 $\mu$g/ml BSA, 0.5 $\mu$g linker, 300 U T4 DNA ligase in 7.5 $\mu$l final volume for 32 h at l4°C.

We adjusted the reaction to 0.l M Tris-HCl (pH 7.5), 0.05 M NaCl, 5 mM $MgCl_2$, l00 $\mu$g/ml BSA, l25 U EcoRI (New England Biolabs), incubated the mixture for 2 h at 37°C, extracted with 50:50 phenol: chloroform and chromatographed the DNA on a G50 spin column as described earlier.

We redissolved cDNA in l00 $\mu$l 0.0l M Tris-HCl (pH 7.5), 0.l M NaCl, l mM EDTA and chromatographed it on a l x 50 cm Biogel A50 (BIORAD) column which had been extensively washed in the same buffer (to remove ligation inhibitors). Aliquots of fractions were run on a l% agarose gel in TBE buffer (0.089 M Tris-HCl, 0.089 M boric acid and 2.5 mM $Na_2$EDTA), dried and exposed at -70°C overnight. We pooled all fractions that were larger than 500 base pairs and ethanol-precipitated the DNA for cloning into an EcoRI-cut λgtl0 cloning vector. The size fractionation column yielded l26 ng of cDNA, average size approximately l500 bp.

Library Construction

We incubated 5 $\mu$g EcoRI-cut λgtl0 with 20 ng cDNA and T4 DNA ligase buffer at 42°C for l5 min to anneal cos sites, followed by centrifugation for 5 sec in an Eppendorf centrifuge and addition of ATP to l mM and 2400 U T4 DNA ligase (New England Biolabs) in a final vol of 50 $\mu$l. [See Huynh, Young and Davis, "Constructing And Screening cDNA Libraries in λgtl0 And λgtll", in DNA Cloning: A Practical Approach (D. Glover, ed.), IRL Press (Oxford l984).] The ligation was incubated at l4°C overnight. We packaged the λgt10 cDNA ligation mixture into phage particles using an Amersham packaging mix [Amersham packaging protocol] and diluted with 0.5 ml SM buffer (100 mM NaCl, 10 mM $MgSO_4$, 50 mM Tris-HCl (pH 7.5) and 0.01% gelatin).

We next infected E.coli C600 hfl cells with these phage particles to form a cDNA library of l x l0[7] independent recombinants [see T. Maniatis, et al., Molecular Cloning, p. 235 (Cold Spring Harbor l982)].

For plating and amplification of the library, 1 ml of cells plus 250 $\mu$l packaging mix was incubated at room temperature for 15 min, diluted to 50 ml in LB plus $MgSO_4$ top agarose at 50°C and plated on LB Mg Nunc plates. This represented a plaque density of 2 x 10[5] plate. The plates were incubated at 37°C for approximately 8 h until plaques were nearly touching.

We flooded the plates with 50 ml of cold SM buffer (0.0l M Tris-HCl (pH 7.5), 0.0l M $MgCl_2$, 0.l mM $Na_2$EDTA) and eluted on a gyro-rotary shaker overnight at 4°C. We pooled the eluants into 250 ml bottles and spun at 6k for l0 min in a Sorvall GSA rotor. We treated the supernatants with an equal volume of cold 20%, PEG 4000-2 M NaCl in ice for 3 h and pelleted the phages by centrifugation at 4k for 30 min in an H4000 rotor in an RC-3B Sorvall centrifuge. The phage pellets were thoroughly drained, resuspended in 60 ml SM, and spun at l0,000 rpm in a SS34 rotor to remove debris. The supernatants were adjusted to 3.5 M CsCl by addition of 7 g CsCl to l0 ml supernatant. We obtained phage bands by centrifugation in a 70.l Beckman rotor at 50,000 rpm for l8 h at l5°C. We pooled the phage bands and stored them at 4°C for library stock. The titer obtained was 2.2 x l0[13] PFU/ml.

Screening Of The Library

We screened the library with our labeled oligonucleotide probes, pools 2 and 3, for lipocortin sequences using the plaque hydridization screening technique of Woo [S. L. C. Woo, "A Sensitive And Rapid Method For Recombinant Phage Screening", in Methods In Enzymology, 68, pp. 389-96 (Academic Press l979)].

An overnight culture of C600 hfl cells in L broth and 0.2% maltose was pelleted and resuspended in an equal volume of SM buffer. We pre-adsorbed 0.9 ml of cells with $2 \times 10^5$ phage particles at room temperature for l5 min. We diluted the suspension to 50 ml in LB plus l0 mM $MgSO_4$ and 0.7% agarose at 55°C and plated it on LB Mg Nunc plates. We screened 10 such plates. We incubated the plates at 37°C for approximately 8 h until plaques were nearly touching. We then chilled the plates at 4°C for 1 h to allow the agarose to harden. We presoaked GeneScreen Plus filters in a 1:10 dilution of the overnight E.coli C600 hfl cells for 10 min at room temperature so that a lawn of E.coli cells covered each filter. We then transferred the λ phage particles from the plaque library plates to these bacteria-coated filters as follows:

We placed the filters onto the plates containing the recombinant plaques for 5 min, and then lifted and incubated the filters with the phagecontaining side up on LB + l0 mM $MgSO_4$ plates at 37°C for 5 h.

These filters were then lysed by placing them onto a pool of 0.5 N NaOH for 5 min, then neutralized on l M Tris-HCl (pH 7.0), submerged into l M Tris-HCl (pH 7.0) and scrubbed clean of cell debris.

We prehybridized and hybridized the filters to the oligonucleotide probes 2 and 3 in 0.2% polyvinyl-pyrrolidone (M.W. 40,000), 0.2% ficoll (M.W. 40,000), 0.2% bovine serum albumin, 0.05 M Tris-HCl (pH 7.5), l M sodium chloride, 0.l% sodium pyrophosphate, l% SDS, l0% dextran sulfate (M.W. 500,000) and denatured salmon sperm DNA (≥ l00 μg/ml) according to manufacturer's specifications (New England Nuclear) for plaque screen membranes). We detected hybridizing λ-cDNA sequences by autoradiography.

By means of this technique, we picked 20 positive plaques and rescreened at lower density using the same probes.

We isolated the DNA of these clones, digested with EcoRI, and hybridized them with the four pools of rat lipocortin probes using the Southern blot technique [E. M. Southern, "Detection Of Specific Sequences Among DNA Fragments Separated By Gel Electrophoresis", J. Mol. Biol., 98, pp. 503-l8 (l975)]. Two of the clones, λ9-lll and λ4-2ll, contained inserted cDNA which hybridized not only to the T24 probe but to the T22a and T29 probes as well.

We restricted the DNAs of these phages with EcoRI and isolated the cDNA inserts. By restricting Clone 9-111 with EcoRI we obtained a l400 base pair fragment while restriction of Clone 4-2ll gave three EcoRI fragments, l300, 300 and 75 base pairs in length. We subcloned some of these fragments into plasmid pUCl3 to produce recombinant plasmids pL9/20 (9-lll), pL4/l0 large (4-2ll, l300 bp), and pL4/l0 small (4-2ll, 300 bp). We then sequenced these plasmids by the method of Maxam and Gilbert (A. M. Maxam and W. Gilbert, "A New Method For Sequencing DNA", Proc. Natl. Acad. Sci. USA, 74, pp. 560-64 (1977)]. This sequencing analysis demonstrated that the clones contained nucleotide sequences which corresponded to the amino acid sequences of the purified rat lipocortin but seemed to be lacking the most 5' sequence.

A 480 base pair EcoRI-BglII fragment of pL9/20 was used as a probe to rescreen the U937-λgt10 library. Seventy-two positives were isolated and partially plaque purified by rescreening at lower density. The DNA of each of these positives was digested with HhaI and analyzed by the Southern blotting technique (E. M. Southern, supra] using a 30 oligonucleotide sequence (lipo 16) as a probe. Lipo 16 corresponds to the sequence starting at base pair 81-111 of the sequence presented in Figure 4. Fourteen of these clones showed a positive signal and were further analyzed by genomic sequencing [G. Church and W. Gilbert, Proc. Natl. Acad. Sci. USA, 81, p. 1991 (1984)] by digesting DNA with MspI and using lipo 16 as probe. Seven clones, λL110, λL106, λL112, λLC, λLH, λLN, and λLDD, contained an 81 base pair sequence 5' to the lipo 16 probe sequence.

These clones contain cDNA sequences having an uninterrupted open reading frame that can code for 363 amino acids (see Figure 4). We believe that the initiating ATG codon for lipocortin may be the ATG located at nucleotides 52-54 of Figure 4. However, the DNA sequence of our clone, reported in Figure 4, may be lacking one or more codons coding for amino acids at the N-terminal end of native lipocortin. These potential missing codons may be isolated, if necessary, by one of skill in the art using conventional hybridization conditions from our libraries, or other libraries, of genomic DNA and cDNA using as probes our clones, or more preferably portions of the 5' terminal end of those clones. Full length clones may then be prepared using conventional ligation techniques and our lipocortin coding clones.

We confirmed that clone λLC of Figure 4 contains the full length gene for lipocortin. To confirm that the ATG at nucleotides 52-54 in the λLC cDNA (Figure 4) is the first in-frame methionine codon and thus the initiating methionine, we determined the 5' sequence of the lipocortin mRNA by primer extension. A 27 oligonucleotide (lipo 18) homologous to the sequence 10 to 37 of λLC was labelled with $^{32}P$-($\gamma$)-ATP and hybridized to human placental poly (A)$^+$ RNA. Using this oligonucleotide as a primer and AMV reverse transcriptase, we transcribed a 60 base pair fragment of the most 5' end of the lipocortin mRNA. This fragment was gel purified and sequenced by the Maxam and Gilbert sequencing technique (supra). The resulting sequence showed 37 base pairs homologous to sequence 1 to 37 of λLC and 23 additional nucleotides that represented the 5' end of the lipocortin mRNA.

14

To exclude the possibility that the mRNA was in fact longer than our primer extension indicated, but instead had a strong stop signal for reverse transcriptase which we mistook for the 5' end, we determined the exact size of the mRNA. An oligonucleotide (lipo 17) that is homologous to sequence 94 to 128 of λLC was hybridized to placental poly (A)$^+$ RNA and digested with RNase H. RNase H digests RNA only when in a hybrid with DNA and thus it introduced a defined cleavage in the mRNA at the site where lipo 17 hybridised. This RNA was then separated on a sequencing gel, blotted onto Gene-Screen and probed with $^{32}$P-labelled lipo 18. This enabled us to determine the exact size of the 5' end of the lipocortin mRNA, which agreed with the size obtained by primer extension.

The cDNA sequences of this invention can be further utilized to screen human genomic cosmid or phage libraries to isolate human genomic sequences encoding human lipocortin-like polypeptides.*

These human cDNA and genomic sequences can be used to transform eukaryotic and prokaryotic host cells by techniques well known in the art to produce human lipocortin-like polypeptides in clinically and commercially useful amounts.

It should also be understood that the cDNA sequences of the invention may be contained in larger mRNA species which result from alternate splicing. Such mRNAs may encode a signal sequence for lipocortin in addition to the mature protein.

## D. EXPRESSION OF A LIPOCORTIN PROTEIN IN E.COLI

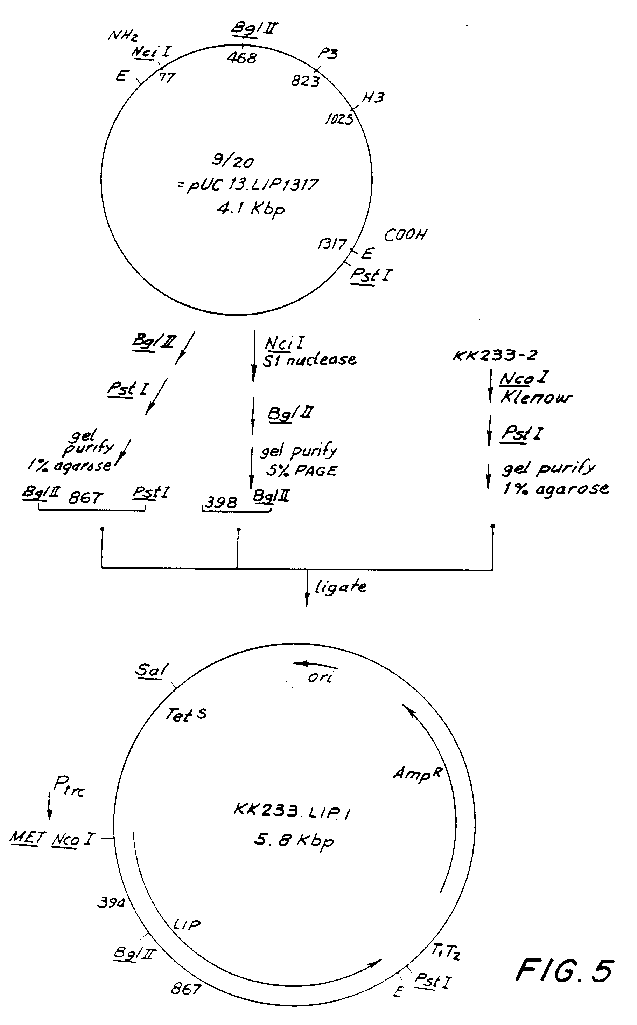

Plasmid pKK233.LIP.1 (which contains a partial sequence of the lipocortin coding region) was constructed by a three part ligation using NcoI-PstI-cut pKK233-2 [E. Amann et al., "Vectors Bearing A Hybrid Trp-Lac Promoter Useful For Regulated Expression Of Cloned Genes In Escherichia coli", Gene, 25, pp. 167-78 (1983)] and the BglII-PstI and NciI-BglII fragments from pL9/20 (see Figure 5). Plasmid pL9/20 contains the DNA sequence of nucleotides 67-1376 of the cDNA insert of λLC shown in Figure 4 inserted into the EcoRI site of pUC13.

Transformants resulting from this ligation and subsequent transformation into E.coli strain HB101 I$^Q$ were picked into microtiter wells containing L broth plus ampicillin and grown overnight. The overnight cultures were then replicated onto nitrocellulose filters on L broth agar plus ampicillin plates in quadruplicate and incubated for approximately 4 h at 37°C. The nitrocellulose filters were then transferred to L broth plates containing IPTG (10 μg/ml) and incubated for 0, 30, 60, or 120 min, followed by lysozyme-detergent treatment to lyse the colonies and finally by Western blot analysis with a cross reactive antiserum that was prepared against the rat lipocortin. Transformants were also analyzed by plasmid restriction mapping. All the Western positive colonies contained plasmids carrying the predicted restriction fragments. Preparations of E.coli from the positive colonies were also analyzed by SDS polyacrylamide gel electrophoresis. With Western blot analysis of these preparations using the antibody against rat lipocortin, we detected a 31,000 molecular weight truncated protein.

We have also constructed various expression vectors in E.coli for the production of the full length human lipocortin. All are perfect constructs starting with the first methionine in the sequence depicted in Figure 4. We confirmed expression by the procedure described above for the truncated protein, using an antiserum prepared against rat lipocortin.

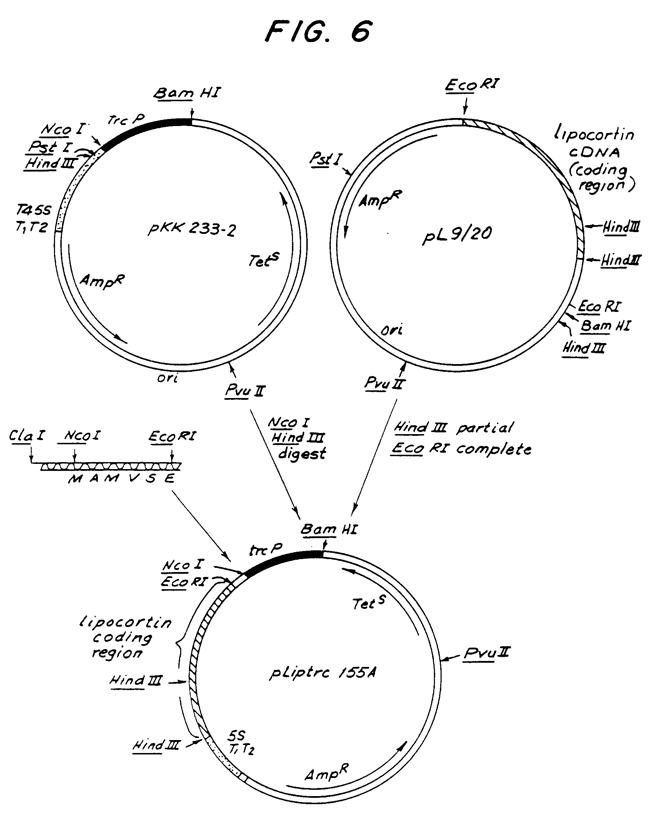

For example, Figure 6 depicts plasmid pLiptrc155A, a trc expression vector derived from plasmid pKK233-2 [E. Amann et al., supra]. pLiptrc155A has a hybrid promoter which contains the -10 region from lac and the -35 region from trp. It also contains the 5S RNA $T_1 T_2$ terminators and the β-lactamase gene which confers ampicillin resistance.

pLiptrc155A (ATCC Deposit No. 68763) was constructed as follows:
Plasmid pKK233-2 was restricted with NcoI and HindIII, yielding a linear fragment. Plasmid pL9/20 was partially digested with HindIII and then completely digested with EcoRI and the 1090 fragment was isolated by agarose gel electrophoresis. These two fragments were then completely digested ligated in the presence of a NcoI-EcoRI linker containing the initiation ATG and the sequence coding for five amino acids 5' to the EcoRI site in the human lipocortin cDNA.

The resulting pLiptrc155A expression vector was then used to transform E.coli strains JA221 and W3110I$^Q$ and expression was induced by growth of the transformed strains for 4 h in LB medium containing 1mM IPTG and 35 μg/ml ampicillin. SDS polyacrylamide gel analysis of crude lysates of the transformed host cells showed a single new protein band at an apparent molecular weight of 37 Kd. Control extracts

---

* For example, an EcoRI fragment of plasmid pL9/20 was used to screen a partial HaeIII-AluI human liver library (R. Lawn et al., "The Isolation And Characterization of Linked δ and β Globin Genes From A Cloned Library of Human DNA", Cell, 15, pp. 1157-74 (1978)), and positive clones were obtained.

from the strains not transformed with pLiptrc155A, or strains transformed with the plasmid but suppressed for production of the protein, did not show this 37 Kd protein. We found, for example, that when the JA221 host was transformed with pLiptrc155A, the 37 Kd protein accounted for as much as 2% of the total protein.

To further verify that we were expressing the human lipocortin, the same lysates were also subjected to Western blot analysis using antibody raised against the rat lipocortin. Only the 37 Kd protein was immunoreactive with the antibody against the rat protein. We have also shown by Western blot analysis that the natural human lipocortin from U937 cells (detected by its immunoreactivity with the anti-rat protein antibody) is virtually identical in size with the 37 Kd protein we expressed, banding in the same place on the gel.

Finally, a small amount of the expressed protein was electroeluted out of an SDS polyacrylamide gel and subjected to N-terminal sequence analysis. The amino acid sequence obtained was consistent with the predicted amino acid sequence of the λLC cDNA sequence of Figure 4.

The human lipocortin which we expressed inhibited exogenous phospholipase A2 in the in vitro assay described in Example A above. This inhibition was detected first using crude lysates and later with a more purified preparation of expressed protein. When the soluble fraction of crude lysates prepared with a french pressure cell was assayed for phospholipase inhibitory activity, we obtained the results shown in Table I below. Inhibitory activity was detected in E.coli lysates containing plasmid pLiptrc155A, while no activity was detected in lysates from E.coli that did not contain the plasmid. As determined by gel analysis, the only difference between these two extracts was the presence of the 37 Kd protein in the inhibitory fraction. We found that the 37 Kd protein accounted for less than 1% of the total protein in the lysate. We also obtained similar results when sonicated lysates were assayed.

Although inhibitory activity could be detected directly in the soluble lysate, most of the 37 Kd protein in E.coli was insoluble and hence removed by low speed centrifugation after the cells were lysed with the french press. The insoluble protein was extracted from particulate matter with guanidine hydrochloride, dialysed against 1M urea, and then assayed for phospholipase inhibitory activity. The results of this assay are shown in Table II below. The dialysate contained approximately 200 U of inhibitory activity per ml (1 U inhibits 15 ng A2). To insure that this activity was the result of a protein, and not some other component in the extract such as lipid, 25 $\mu$l of the lysate used in Table II were incubated with trypsin. As shown in Table III below, the inhibitory activity was very trypsin-sensitive.

Table I.    Phospholipase Inhibitory Activity In
            Crude E.coli Lysates.

    Cultures of the W3110I$^Q$ strain of E.coli,
which either did or did not contain the plasmid
pLiptrc155A, were induced with IPTG and lysed with a
french pressure cell.  Particulate matter was removed
by centrifugation at 10,000xg for 20 min.  The soluble
fraction was assayed for phospholipase inhibitory
activity.  The numbers shown are the averages from
several assays in which 50 μl of extracts were assayed
with 100 ng of porcine pancreatic phospholipase A$_2$.

| Sample | Percent Inhibition |
|---|---|
| A$_2$ alone | 0 |
| A$_2$ + E.coli, no plasmid | 0 |
| A$_2$ + E.coli containing trc plasmid | 24 |

Table II.   Dose-Response Curve Of Partially Purified
            Inhibitor.

    The insoluble preparation, which was recov-
ered from JA221 cells transformed with pLiptrc155A
(using the lysis treatment described above) was ex-
posed to 6M guanidine hydrochloride in 25 mM sodium
acetate (pH 6.0).  Particulate matter was removed by
centrifugation (100,000xg for 1 h).  Extracted protein,
which was highly enriched for the human 37 Kd protein,
was dialyzed against 1M urea in 25 mM sodium acetate

(pH 6.0) and then assayed for phospholipase $A_2$ inhibitory activity.

| μl extract assayed | Percent Inhibition |
|---|---|
| 0 | 0 |
| 3 | 12 |
| 10 | 26 |
| 30 | 58 |

Table III. Trypsin Sensitivity Of Inhibitor.

The partially purified preparation described in Table II was exposed to trypsin for 15 min at room temperature and then assayed with 100 ng of porcine pancreatic phospholipase $A_2$. Under the conditions used, the trypsin treatment did not alter the phospholipase $A_2$ activity. For each sample, 25 μl of inhibitor were assayed.

| Sample | Trypsin μg/ml | Percent Inhibition |
|---|---|---|
| $A_2$ alone | 0 | 0 |
| $A_2$ + inhibitor | 0 | 54 |
| $A_2$ + inhibitor | 1 | 3 |
| $A_2$ + inhibitor | 3 | 4 |

In addition to pLiptrc155A, we constructed other high level expression vectors of this invention. For example, plasmid pLipPLT4A was constructed as follows: plasmid pPLT4HTNF, a gift from Walter Fiers, (this plasmid is identical to the plasmid deposited in the culture collection of the Deutsche Sammlung Von Mikroorganismen, in Gottingen, West Germany, on December 27, 1984 under DSM No. 3175 and which was deposited within E.coli strain C600 and designated as pBR322-pL-T4-hTNF) was digested with restriction enzymes ClaI and HindIII and a linear fragment was obtained. Plasmid pL9/20 was partially digested with HindIII and then completely with EcoRI, and the 1350 bp fragment was isolated from an agarose gel. These two fragments were ligated in the presence of a ClaI-EcoRI linker containing an initiation ATG and the sequence coding for five amino acids 5' to the EcoRI site in the lipocortin cDNA. In the resulting expression vector, the $P_L$ promoter directs the transcription of a hybrid mRNA including sequences of $P_L$, $T_4$ and the lipocortin mRNA. Translation of this mRNA initiates at the first ATG of the human lipocortin coding sequence, resulting in a 37 Kd protein. A second tetracycline resistant plasmid pLipPLT4T was constructed by inserting the tetracycline resistance gene of pBR322 into the ScaI site of pLipPLT4A.

We transformed E.coli strains MC1061 and C600pCi 857 with pLipPLT4A and determined expression by SDS polyacrylamide electrophoresis and Western blot analysis as described above. The E.coli extracts showed a 37 Kd protein reactive with antibody to rat lipocortin.

## E. EXPRESSION OF HUMAN LIPOCORTIN PROTEIN IN YEAST

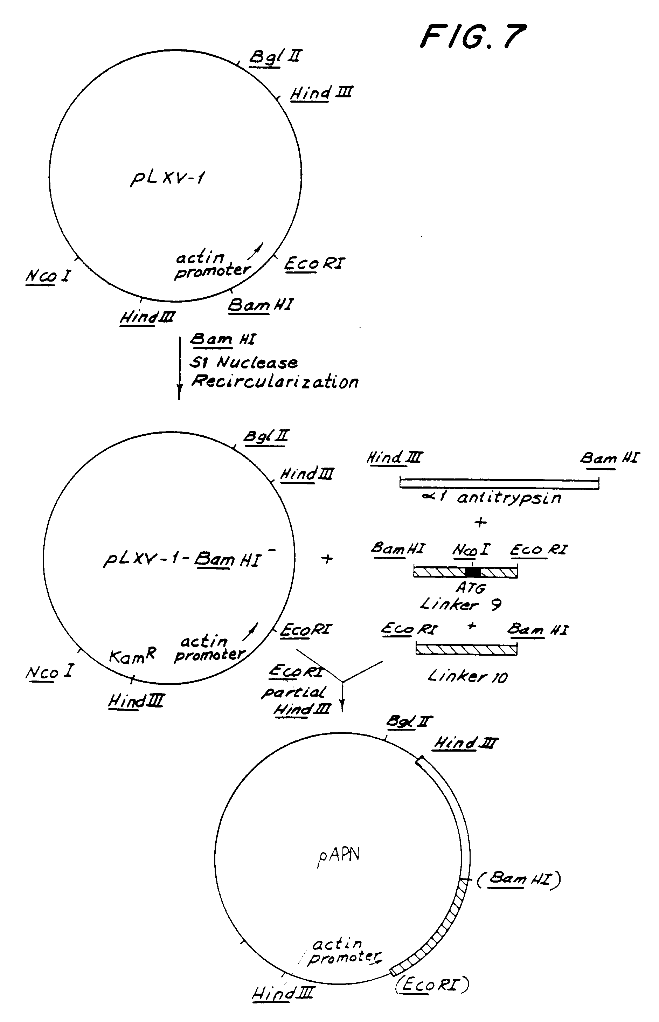

We have also constructed expression vectors for the production of human lipocortin in yeast. Figures 7-9 show the construction of pBgl20 which, when used to transform yeast cells, expressed human lipocortin as detected by Western blot analysis with the anti-rat lipocortin antibody.

We constructed pBgl20 as follows:

Plasmid pLXV-l contains the origin of DNA replication from E.coli plasmid pBR322 and from yeast plasmid 2μ DNA. It can therefore replicate in both E.coli and yeast (J. Ernst, personal communication). We removed the BamHI site from plasmid pLXV-l by digestion with BamHI, treatment with SI nuclease at 37°C for 30 minutes and recircularization with T4 DNA ligase (Figure 7). E.coli JA221 was transformed with the ligation mixture and plasmid pLXV-1-BamHI⁻ was isolated.

In order to reconstitute the ATG initiation codon of the actin gene on pLXV-l, we digested pLXV-l-BamHI⁻ with EcoRI and with HindIII. We isolated the large fragment containing the actin promoter. We ligated together this fragment with a DNA fragment containing the human α1 antitrypsin gene containing a BamHI end and a HindIII linker*and two synthetic linkers (linkers 9 & 10).**Linkers 9 and 10 both contained EcoRI and BamHI sticky ends and linker 9 contained an NcoI restriction site and the ATG initiation codon. This ligation resulted in the loss of the EcoRI and BamHI restriction sites. We transformed E.coli JA221 with this ligation mixture and selected for Ampicillin resistance and Kanamycin resistance to insure the recreation of the HindIII site located within the Kam^R gene. We designated the resultant plasmid pAPN.

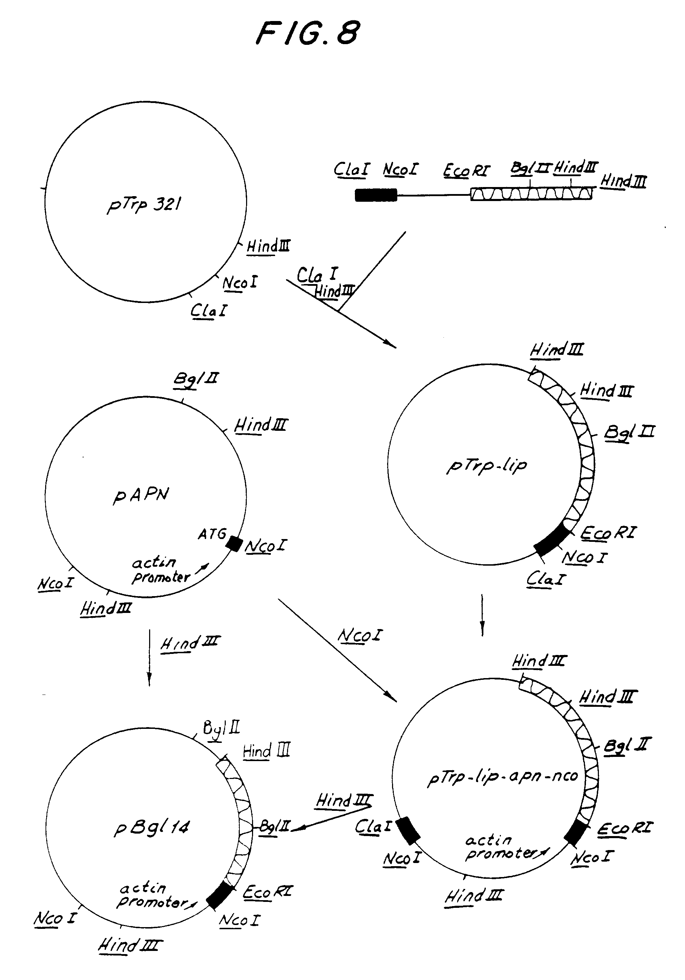

Figure 8 shows the insertion of the DNA sequences coding for human lipocortin into a yeast expression vector. The human lipocortin sequence was cloned in two parts. First, the N-terminal region was inserted behind and operatively linked to the yeast actin expression control sequence. Plasmid pTrp32I (R. Devos et al., "Molecular cloning of human interleukin 2 cDNA and its expression in E.coli," Nucleic Acids Research, II, pp. 4307-23 (1983)) was digested with ClaI and HindIII and the large fragment was ligated to the pL9/20 1090-linker fragment prepared supra, p. 34. We isolated plasmid pTrplip by screening for the expression of human lipocortin as described above.

We next digested pAPN with NcoI and isolated the small fragment containing the yeast actin expression control sequence. We ligated this fragment to pTrp-lip which had been linearized with NcoI. We designated this plasmid pTrp-lip-apn-nco. We digested ptrp-lip-apn-nco with HindIII and isolated the fragment containing the yeast actin expression control sequence and the DNA sequences corresponding to the N-terminal region of the human lipocortin. We ligated this fragment to the large HindIII fragment of pAPN to produce plasmid pBgl14.

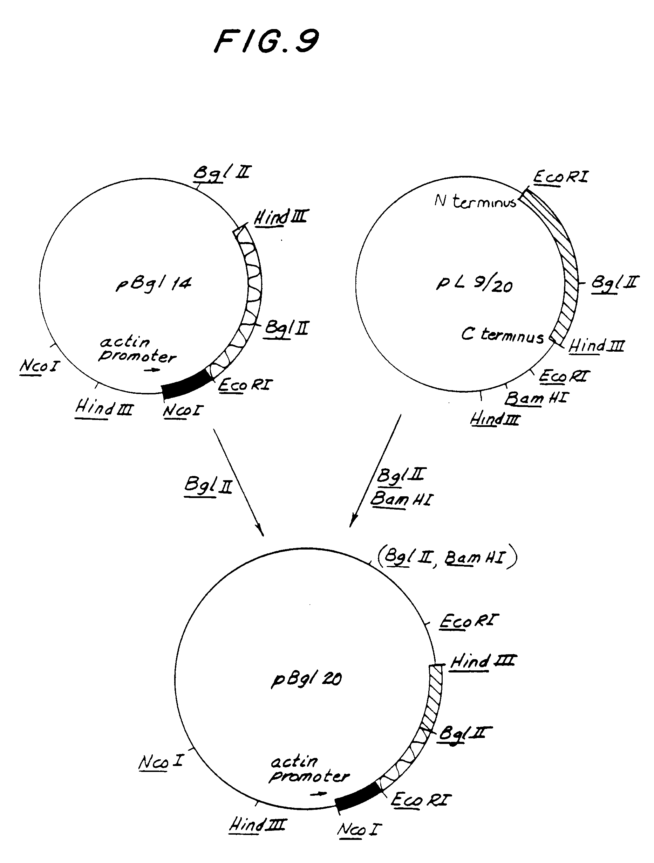

We obtained the DNA sequences corresponding to the remaining C-terminal fragment of the human lipocortin from the 800bp BglII-BamHI fragment of pL9/20, supra (Figure 9). We digested pL9/20 with BglII and BamHI and electroeluted the 800bp fragment. This fragment was ligated to pBgl14 which had been digested with BglII. We isolated plasmid pBgl20, containing DNA sequences coding for human lipocortin operatively linked to the actin expression control sequence.

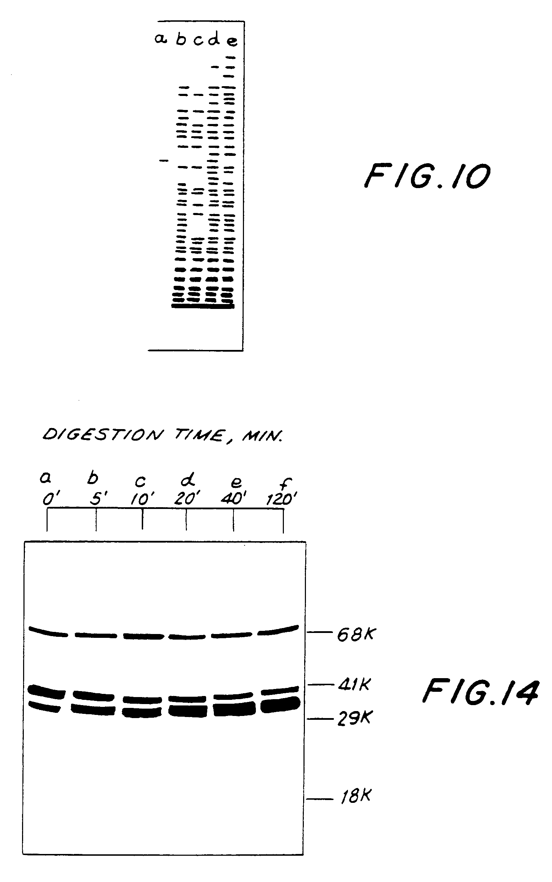

We detected in extracts of yeast transformed with pBg120 a 37 Kd protein not observed in untransformed yeast cells or in yeast cells transformed with plasmids which did not contain the human lipocortin gene. This protein corresponded exactly on SDS-polyacrylamide gel electrophoresis to the human 37 Kd protein produced in E.coli (Figure 10).

In yeast the human 37 Kd protein is produced as a soluble protein. It accounts for about 4% of the total cellular protein. When yeast cultures containing the recombinant protein were lysed with a french pressure cell at 20,000 p.s.i. and particulate matter removed by centrifugation (l0,000 x g, l0 min) approximately 80% of the inhibitory activity was recovered in the soluble fraction. The distribution of inhibitory activity correlated exactly with the distribution of the human protein based on SDS-polyacrylamide gel analysis. The

* We used the α1 antitrypsin DNA fragment solely to insert the BamHI and HindIII restriction sites into the plasmid. The DNA sequence coding for α1 antitrypsin, between the two restriction sites, was later excised and replaced with the lipocortin DNA sequence. The specific sequence used to provide this restriction sites in the plasmid was irrelevant and any DNA sequence could have been used in place of the α1 antitrypsin DNA.

** Linker 9 was a mixture of the two sequences AATTAACAATGGAA and AATTAACCATGGAG. Linker 10 was a mixture of GATCCTCCATGGTT and GATCTTCCATTGTT.

recombinant protein produced in yeast has a blocked amino-terminus.

## F. EXPRESSION OF HUMAN LIPOCORTIN PROTEIN IN MAMMALIAN CELLS

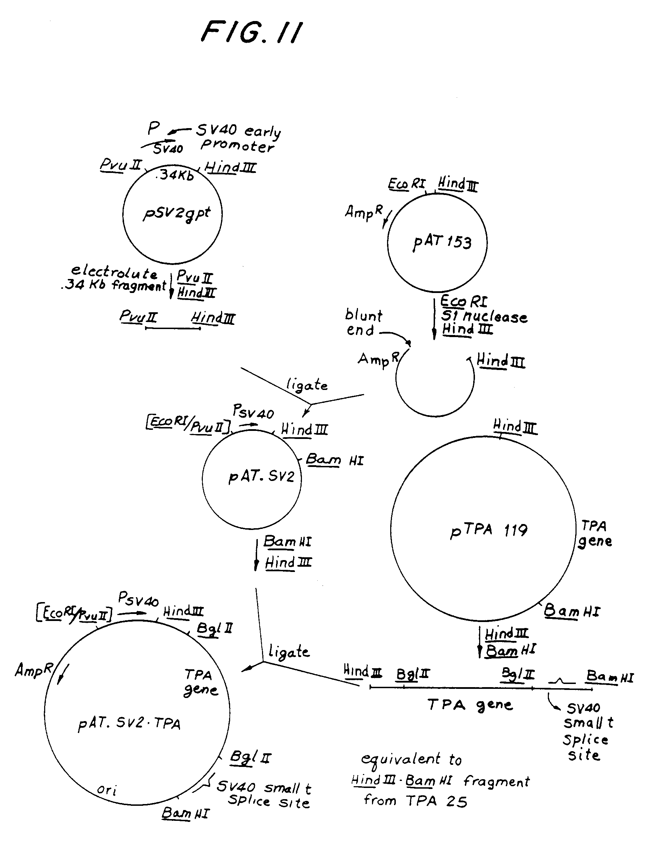

We have also constructed expression vectors for the production of human lipocortin in mammalian hosts. Figures 11-13 show the construction of pSVL9l09 which, when transfected into cos and CHO host cells by the CaPO₄ procedure (F. Graham et al., J. Virology, 52, pp. 455-56 (1973)), expressed human lipocortin as detected by Western blot analysis with the anti-rat lipocortin antibody. We detected a 37 Kd immunoreactive protein not observed in nontransfected cells, indicating that the vector was producing the human inhibitor protein. We constructed pSVL9l09 as follows:

As shown in Figure 11, plasmid pSV2gpt (R. Mulligan et al., Proc. Natl. Acad. Sci. USA, 78, pp. 2072-76 (1981)) was cut with PvuII and HindIII and the 340 bp fragment containing the SV40 early promoter was isolated and inserted into pATl53 which had been previously cut with EcoRI, Sl treated, and then cut with HindIII. The resulting plasmid pAT.SV2 contained the promoter. This plasmid was then cut with HindIII and BamHI. Into this site we cloned a sequence which contained the small t splice site. This sequence was isolated from another plasmid, pTPAll9, by cutting with HindIII and BamHI. The 3 kb insert contained the DNA sequence coding for human tissue plasminogen activator along with the small t splice site. This sequence is equivalent to that found in the pTPA25 HindIII-BamHI segment deposited with the American Type Culture Collection in Rockville, Maryland, on August 21, 1984 under ATCC No. 39808. The HindIII-BamHI 3 kb piece was ligated to pAT.SV2 to yield plasmid pAT.SV2.TPA. This vector contains the SV40 early promoter followed by a HindIII site and the SV40 small t splice signal preceded by a BglII site.

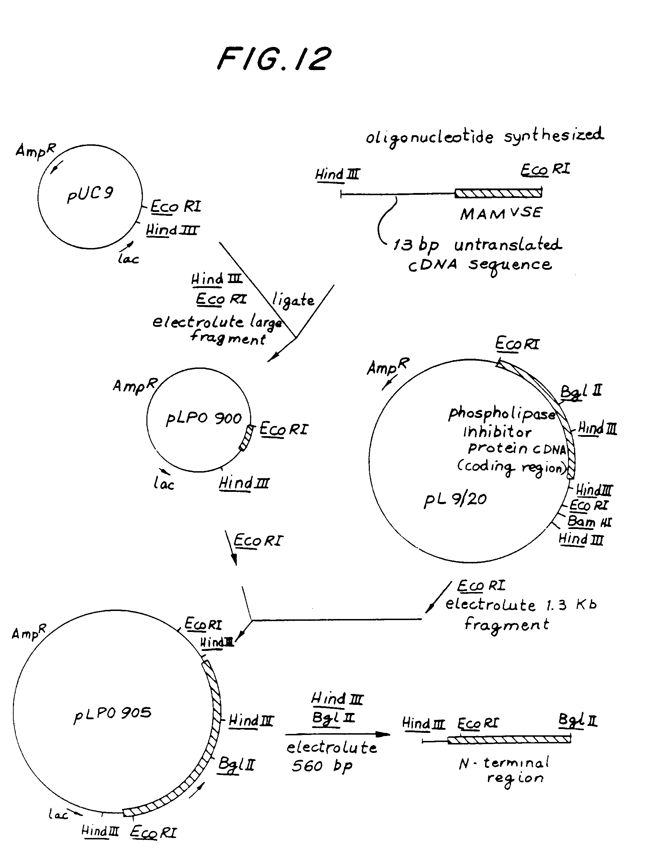

We next inserted the coding sequence for human lipocortin into pAT.SV2.TPA. An oligonucleotide of 29 bp with EcoRI and HindIII ends was synthesized (see Figure 12). This oligonucleotide includes the coding sequence for the first six amino acids of human lipocortin. This sequence was cloned into pUC9 (J. Vieira et al., Gene, 19, pp. 259-68 (1982)) which had been digested with EcoRI and HindIII to yield pLPO900. This plasmid was cut with EcoRI and treated with calf alkaline phosphatase. We then cloned the 1.3 kb EcoRI fragment from pL9/20 which corresponds to the coding region for human lipocortin into pLPO900. The resulting plasmid pLPO905 contains the entire coding region for human lipocortin with a HindIII site upstream (see Figure 12). This makes the gene suitable for cloning behind the SV40 promoter of pAT.SV2.TPA.

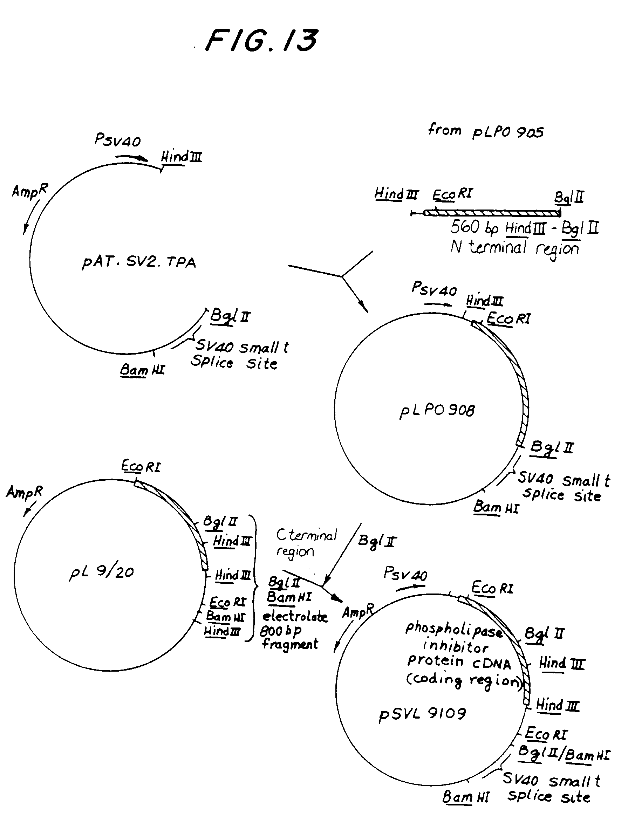

Figure 13 shows the insertion of the gene for human lipocortin into pAT.SV2.TPA to form a mammalian expression vector of this invention. The human lipocortin sequence was cloned into the expression vector in two parts. First, the N-terminal region was inserted behind the SV40 promoter and then the C-terminal region was added. The plasmid pLPO905 was cut with HindIII and BglII and the 560 bp fragment containing the N-terminal region of human lipocortin was isolated. pAT.SV2.TPA was cut with HindIII and BglII and the 5 kb fragment containing the vector was isolated, free of TPA sequences. Into this HindIII-BglII vector, we inserted the 560 bp HindIII-BglII fragment containing the N-terminal region of human lipocortin to yield the plasmid pLPO908.

The C-terminal region of human lipocortin is found within the 800 bp BglII-BamHI fragment of pL9/20. Thus, pL9/20 was cut with BglII and BamHI, followed by electroelution of the 800 bp fragment. This fragment was ligated into the plasmid pLPO908 which had been cut with BglII and treated with calf alkaline phosphatase. Plasmid pSVL9l09 was isolated. This plasmid has the entire human lipocortin coding sequence downstream of the SV40 early promoter followed by the SV40 small t splice signal. Plasmid pSVL9l09 was used to transfect cos and CHO hosts as described above.

Thus, utilizing the DNA sequences of the invention, we have constructed high level expression vectors for the expression of human lipocortin in a biologically active form.

Recombinant DNA sequences prepared by the processes described herein are exemplified by a culture deposited in the culture collection of In Vitro International, Inc., Ann Arbor, Michigan. The culture was deposited on January 9, 1985 and is identified as follows:

λLC: IVI No. 10042 (now transferred to ATCC Deposit No.68812)

Microorganisms prepared by the processes described herein are exemplified by a culture deposited in the above-mentioned depository on March 12, 1985 and on August 14, 1985, respectively, and identified as follows:

E.coli W3110l^Q (pLiPtrc155A: IVI No.10046 (now transferred to ATCC Deposit No.68763).

S.cerevisiae 331-17A (pBg120): IVI No.10088 (now transferred to ATCC Deposit No.74106).

## G. PRODUCTION OF BIOLOGICALLY ACTIVE HUMAN LIPOCORTIN-LIKE POLYPEPTIDE FRAGMENTS

The production of polypeptide fragments smaller than the 37,000 molecular weight human lipocortin which display phospholipase inhibitory activity is highly desirable. A smaller polypeptide is more easily delivered to target cells by, e.g., transdermal infusion rather than intravenous injection. A smaller polypeptide might also have a more stable conformation than the 37K protein. For example, the N-terminal end of the 37K protein contains a cluster of hydrophobic amino acids, which may cause the formation of intermolecular aggregates, while the C-terminal end contains four cysteines capable of forming improper disulfide linkages (see Fig. 4). Thus removal of the N-terminal and C-terminal ends of the large protein may avoid conformational heterogeneity when the protein is produced in high concentrations. Isolation of biologically active fragments will also permit better characterization of the active site of the lipocortin molecule and lead to the design of fragments with optimal therapeutic value.

Although a variety of methods can be used for generation of polypeptide fragments from their parent molecule in accordance with this aspect of our invention, we chose to subject the human lipocortin-like polypeptide to limited protease digestion in the illustrative embodiment described below. We first optimized digestion conditions for each protease so that the fragments generated were few in number, usually less than ten, and therefore easily isolated. The optimum conditions were in general determined by the following factors: (1) the type of protease used; (2) the physical state of the target protein, i.e., denaturing or non-denaturing conditions; (3) the time period for digestion; (4) the temperature; (5) the amount of protease used; and (6) the pH.

We used two different types of proteases to digest our 37K lipocortin-like polypeptide. The first type hydrolyzes peptide bonds non-specifically. This type is represented by elastase and proteinase K proteases. The second type, represented by the proteases plasmin and thrombin, hydrolyzes a specific peptide bond. This second type probably recognizes target proteins both by amino acid sequence and by conformation surrounding the cleavage sites. We also used trypsin, a third type of protease, to localize the C-terminal end of the active peptide fragments (see pp. 48-49, infra). Trypsin hydrolyzes peptide bonds at specific amino acids. Other proteases of this type include chymotrypsin and V8 protease. Because we wished to obtain fragments with biological activity, we performed digestions under non-denaturing conditions.

### 1. Production, Isolation And Characterization Of Plasmin-Digested Fragments From Recombinant Human Lipocortin

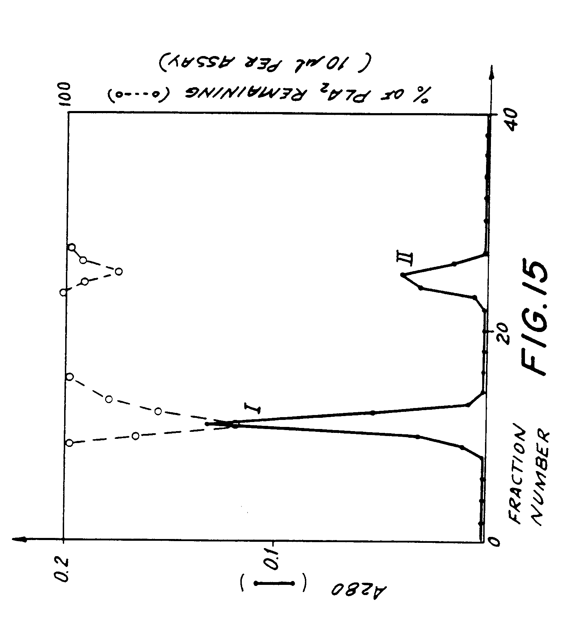

In order to optimize conditions for plasmin digestion, we incubated an aliquot of our E.coli-produced human lipocortin-like polypeptide (100 $\mu$g) in 100 $\mu$l of 0.2M Tris HCl (pH 8.0), 5mM EDTA and 0.1 mg bovine serum albumin/ml solution with 10 $\mu$l (1 $\mu$g/$\mu$l) plasmin (Calbiochem) at room temperature. At different time intervals, 15 $\mu$l were withdrawn and the reaction quenched by adding 5 $\mu$l of buffer containing 8% SDS and then boiling the aliquots for 5 min. The digestion mixture at each time point was analyzed by SDS gel electrophoresis. The results, depicted in Figure 14, show that after an hour of incubation almost all the protein was digested to a fragment with a molecular weight of 33,000. This 33K fragment was resistant to further digestion either with longer incubation times or in the presence of increased amounts of plasmin.

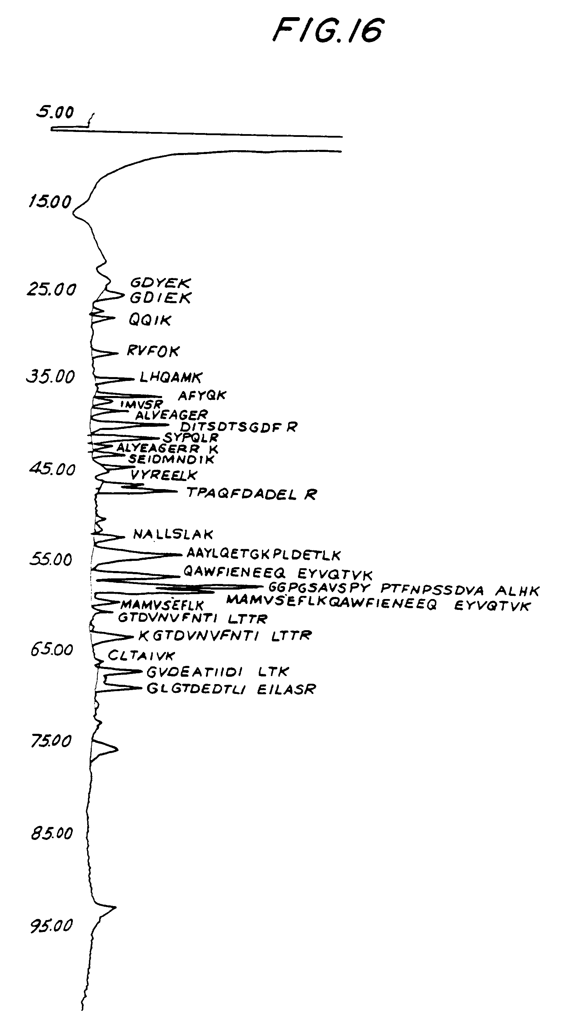

Using the digestion conditions described supra, we incubated 500 $\mu$g of human lipocortin-like polypeptide with 75 $\mu$g of plasmin for 1 hour. The reaction was stopped with 250 KIU of the protease inhibitor aprotinin (Sigma). We loaded the digested samples onto a Biogel P-60 column (1.0 x 50 cm), equilibrated with 0.2M Tris-HCl, pH 7.5, 5mM EDTA, 0.1% bovine serum albumin at 4°C. We collected 1.1 ml fractions. The elution profile of fragments monitored at 280 nm disclosed two protein peaks (Figure 15). We analyzed aliquots of fractions from each peak by high pressure liquid chromatography and by SDS gel electrophoresis. The results showed that peak I contained a fragment with 33,000 molecular weight (designated as Lipo-L), and peak II contained a fragment with 4,000 molecular weight (designated as Lipo-S).

Fractions of the Biogel P-60 column chromatography were assayed for phospholipase A$_2$ inhibitory activity, as described supra. The majority of activity was associated with peak I (Figure 15). Although a small amount of inhibitory activity was detected in peak II, that activity was not dependent on fragment concentration and was probably caused by non-specific binding of the fragment to the membrane.

We also isolated the two cleavage products by reverse phase high pressure liquid chromatography with a gradient of acetonitrile from 0-75% in 0.1% trifluoroacetic acid using C$_4$ column chromatography (Vydac). Lipo-S was eluted with 38% acetonitrile and Lipo-L with 48% acetonitrile.

We determined the N-terminal amino acid sequence of Lipo-L (33K; peak I) by sequential Edman degradation using a gas phase sequencer (Applied Biosystems 470A). PTH-amino acids were analyzed by high pressure liquid chromatography on a 5 $\mu$m cyanocolumn (Hypersil) using a gradient of acetonitrile:methanol (4:1) from 15-55% in 0.02M sodium acetate buffer (pH 5.7). We determined the N-terminal amino acid sequence of Lipo-L to be $H_2$N-Gly-Gly-Pro-Gly-Ser-Ala-Val. The N-terminal glycine of Lipo-L corresponds to Gly-30 of the 37K protein.

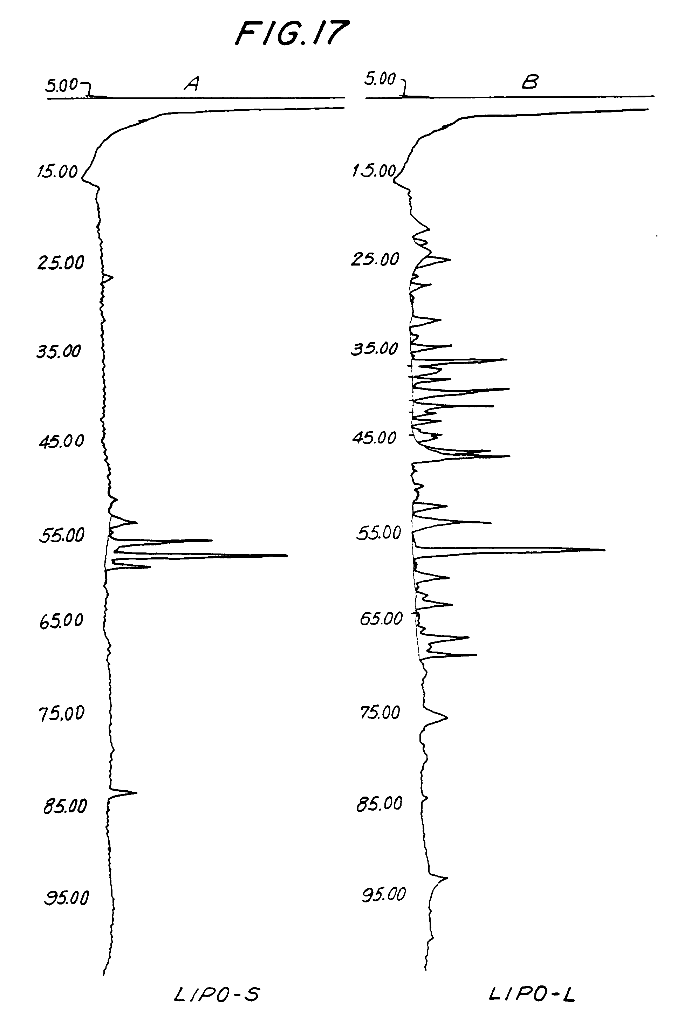

To determine the C-terminal amino acid sequence of our fragments, we compared the tryptic peptide map of the 37K rat lipocortin (Figure 16) with a tryptic peptide map which we generated for each of the fragments. We diluted 20 $\mu$l of the 37K lipocortin-like polypeptide (40 $\mu$g) in 0.2M Tris-HCl, pH 8.0, 5mM EDTA, 0.1 mg bovine serum albumin/ml with 0.2 ml of 0.1M $NH_4HCO_3$ plus 1mM $CaCl_2$. The mixture was then incubated with trypsin for 24 hr. at 37°C. During this incubation, we added trypsin at three time points: 0.5 $\mu$g at time zero, 0.25 $\mu$g after 4 hr. and 0.25 $\mu$g after 19 hr. The digestion was stopped by adding 10 $\mu$l of 90% formic acid to the reaction mixture. We digested the plasmin fragments using identical conditions, except that we first dried down the fragments, which had been purified from high pressure liquid chromatography, with a Speedvac concentrator (Savant), and dissolved the residues in 0.1M $NH_4HCO_3$ and 1mM $CaCl_2$.

We resolved the tryptic fragments by reverse phase high pressure liquid chromatography with a gradient of acetonitrile from 0-75% in 0.1% trifluoroacetic acid on a $C_{18}$ column (Spectraphysics). We then determined the amino acid sequence of each of the peak fractions. First, we established the amino acid composition of each peak by hydrolyzing the peptides in 6N HCl for 24 hr. and determining the composition on a Beckman 6300 amino acid analyzer. We compared the observed compositions with predicted compositions based on sequence data of all possible fragments from the 37K polypeptide and assigned identifications to each peak. We also determined the amino acid sequence of some of the peaks directly by sequence analysis.

By comparing the map of fragment Lipo-L (Figure 17B) with that of 37K polypeptide (Figure 16), we concluded that the biologically active Lipo-L fragment has the same C-terminal amino acid sequence as that of the 37K polypeptide. Therefore, this data, when combined with our N-terminal sequence data, demonstrate that Lipo L contains amino acid residues #30 to #346 of the 37K polypeptide. By comparing the peptide maps of fragment Lipo S (Figure 17A) with that of the 37K polypeptide, we concluded that Lipo S contains amino acid residues #1 to 29 of the 37K protein.

### 2. Production, Isolation And Characterization Of Elastase Digested Fragments From Recombinant Human Lipocortin

We treated 50 $\mu$l of the 37K human lipocortin-like polypeptide (75 $\mu$g), 0.2M Tris-HCl, pH 8.0, 5 mM EDTA, 0.1 mg bovine serum albumin/ml, with 2.5 $\mu$g (1 $\mu$g/$\mu$l in 10mM ammonium acetate, pH 6.0) of elastase (Sigma) at room temperature. We analyzed the digestion products at different time intervals as described supra to optimize conditions for elastase digestion. The results showed that 20 min. of incubation generated fragments with molecular weights ranging from 10K to 33K.

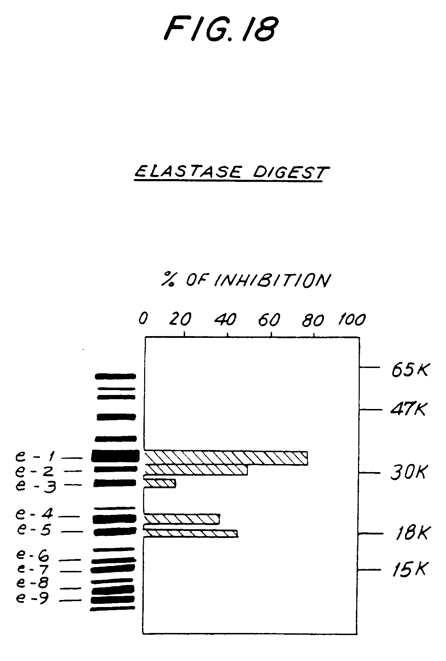

We incubated 150 $\mu$g of the 37K polypeptide with 5 $\mu$g of elastase under optimum conditions as described above and subjected the digested polypeptides to SDS-polyacrylamide gel electrophoresis (D. A. Hager and R. R. Burgess, Anal. Biochem., 109, 76-86 (1980)). The polypeptide bands were visualized by soaking the gel in 0.25M KCl, 2mM DTT at 4°C for 5-10 min. We then cut out the polypeptide bands and removed the KCl from the gel slices by soaking each slice in deionized $H_2O$ containing 2mM DTT for 10 min. at room temperature. The fragments were then eluted from the gel at room temperature for 2 hr. by a solution of 0.1% SDS; 0.2M Tris HCl, pH 7.5; 5mM EDTA; 5mM DTT; 0.01% bovine serum albumin. The fragments-containing solution was then mixed with a solution of ice-cold acetone at -70°C and incubated for 30' at -70°C. We collected the precipitate by centrifugation at 10,000 rpm (SS34 rotor) for 10 min. at 4°C, and rinsed once with ice cold 80% acetone and 20% buffer containing 0.2M Tris HCl, pH 7.5 and 5mM DTT. The precipitate was collected by centrifugation and dried under vacuum for 5-10 min. We then dissolved the dried powder in 6M guanadine hydrochloride in 0.2M Tris HCl (pH 7.9), 2mM DTT, 5mM EDTA, 0.1% bovine serum albumin for 20 min. To the solution we then added a "renaturation" buffer (20% glycerol and 80% 0.2M Tris-HCl (pH 7.9), 2mM DTT, 5mM EDTA, 0.1% bovine serum albumin). The solution was then allowed to stand at 4°C overnight. We next assayed for phospholipase $A_2$ inhibitory activity as described. The activities of the SDS-gel fragments are shown in Figure 18. The fragments e-1, e-2, e-3, e-4 and e-5, with molecular weights of 33K, 30K, 27K, 20K and 18K, respectively, showed phospholipase $A_2$ inhibitory activity.

We then purified the major active fragments (e-1, e-4, and e-5 of Figure 18) by preparative SDS-gel electrophoresis. We visualized the polypeptide bands by soaking the gel in 0.25M KCl and excised the biologically active polypeptide bands. We electroeluted the fragments using standard techniques (M. W.

Hunkapiller, E. Lujan, F. Ostrader and L. E. Hood, Methods Enzymol. 91, pp. 227-236 (1983)).

We determined the N-terminal and C-terminal amino acid sequences of e-1, e-4 and e-5. The purified fragments all showed the identical N-terminal amino acid sequence: H₂N-Gly-Gly-Pro-Gly-Ser-Ala-Val-Ser-Pro-Tyr. This N-terminal glycine corresponds to Gly-30 of the 37K protein.

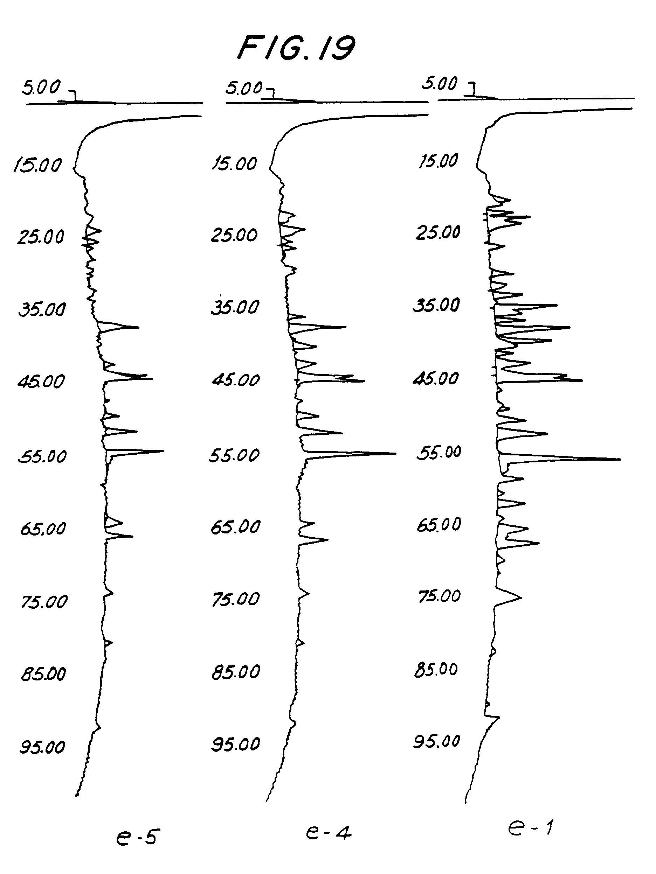

Figure 19 shows the tryptic peptide maps of these purified fragments. We compared these maps with that of the 37K protein (Figure 16) and found that the 18K e-5 fragment lacked the tryptic fragment Ala-Leu-Tyr-Glu-Ala-Gly-Glu-Arg (residues #205-212 of the 37K protein) and all fragments beyond this peptide. This suggests that cleavage is before this peptide. The peptide map contains the fragment Asn-Ala-Leu-Leu-Ser-Leu-Ala-Lys (residues #178-185 of the 37K protein). Thus, the C-terminus of this fragment is between Lys-185 and Arg-212 of the 37K protein. Similarly, the peptide map of the 20K e-4 fragment contains all the fragments found in the 18K fragment plus an additional peptide Ala-Leu-Tyr-Glu-Ala-Gly-Glu-Arg-Arg-Lys (residues #205-214 of the 37K protein). The C-terminal end of this fragment is therefore between Lys-214 and Arg-228 of the 37K protein. The C-terminal end of the 33K e-1 fragment is located between Lys-337 and Asn-346 of the 37K protein.

Thus, we have isolated at least three biologically active peptide fragments of the 37K lipocortin-like polypeptide. All three fragments have identical N-terminal ends, beginning with Gly 30 of the 37K polypeptide, each ending at a different point along the 37K molecule. All three show the phospholipase A2 inhibitory activity of the full-size lipocortin-like polypeptide.

Although we used the above methods for production of polypeptide fragments from human lipocortin-like polypeptide produced in E.coli, it should be understood that such molecules can also be produced by a variety of other methods. For example, such molecules can be produced by proteases other than the ones we used or by chemicals which cleave or synthesize peptide bonds. Furthermore, such molecules can also be produced by expression of truncated DNA sequences coding for these fragments. However, this method may require different purification procedures for each fragment and occasionally results in the production of DNA sequences which are unstable in the expression vector.

## H. THE PRODUCTION OF BIOLOGICALLY ACTIVE HUMAN LIPOCORTIN-LIKE POLYPEPTIDE FRAGMENTS BY RECOMBINANT DNA TECHNIQUES

An alternative method of generating lipocortin-like polypeptide fragments by recombinant DNA techniques is to introduce into the DNA sequence encoding lipocortin codons which, upon expression of the DNA sequence in the form of the polypeptide, result in sites at which the polypeptide can be cleaved (e.g., chemically or proteolytically). These codons can be introduced into the DNA by various techniques known in the art such as mutagenesis or insertion. Thus, an altered lipocortin-like polypeptide is produced by the host cell, purified in the same manner as the unaltered full length polypeptide and cleaved to yield a desired fragment or fragments. Using this strategy, we have altered the lipocortin DNA sequence of the present invention to produce altered polypeptides which when cleaved in vitro, yield lipocortin-like polypeptide fragments displaying phospholipase A₂ inhibitory activity.

According to one embodiment of this technique, we constructed DNA sequences that code for altered lipocortin-like polypeptides by modifying the DNA sequence of lipocortin to introduce methionine codons into the sequence. Upon expression of this sequence in a host cell, altered lipocortin-like polypeptides were produced having either a specific amino acid replaced by a methionine or an inserted methionine at a particular site along the polypeptide sequence. Treatment of these altered polypeptides with cyanogen bromide, which cleaves polypeptides specifically at methionine residues, yielded lipocortin-like polypeptide fragments having phospholipase A₂ inhibitory activity.

We have found that the natural human lipocortin protein has only two methionine residues (at amino acids 56 and 127) which are contained within the biologically active elastase fragments described in Example G(2) above. Since cleavage of a lipocortin-like polypeptide with cyanogen bromide inactivated the polypeptide and any fragments produced thereby, we inferred that one or both of these methionines were necessary for the biological activity of the protein. Thus, a preliminary step in the production of active lipocortin-like polypeptide fragments by cyanogen bromide treatment required the replacement of these two methionines with another amino acid without destroying the biological activity of the lipocortin protein. We therefore replaced methionines 56 and 127 with leucine residues and obtained a biologically active polypeptide fragment upon treatment with cyanogen bromide.

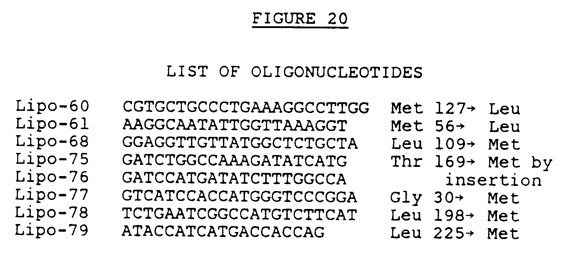

We replaced methionines 56 and 127 by mutagenesis of the DNA sequence encoding lipocortin as follows: We restricted plasmid pLiptrc155A, which contains the DNA sequence encoding lipocortin, with the restriction enzyme PvuI, resulting in a linearized pLiptrc155A molecule. We also restricted plasmid pKK233-2 with the restriction enzymes NcoI and HindIII, which cut the plasmid on either side of the DNA sequence encoding lipocortin (see Example D and Figure 6 for the construction of these plasmids). Mixture of the two restricted plasmids, when denatured and reannealed, yielded a gapped heteroduplex with the DNA sequence encoding lipocortin in an approximately 1300 base pair single-stranded region. Mutagenic oligonucleotides Lipo 60 and 61 were phosphorylated at the 5' end and a 320-fold excess was added to the heteroduplex mixture. Lipo 60 and 61 contain DNA sequences similar to a portion of the lipocortin DNA sequence but having specific nucleotide changes such that expression of the oligonucleotide sequences results in the replacement of met 127 and met 56 of lipocortin with a leucine residue, respectively (see Figure 20). These oligonucleotides therefore hybridize to the single-stranded region of the heteroduplex molecules. The DNA gaps in the heteroduplex molecules were filled in with the Klenow fragment of DNA polymerase I and the DNA ligated with T4 polynucleotide kinase [see, Morinaga, et al., Biotechnology, 2, pp. 636-39 (1984)]. We transformed E.coli strain JA221 with this DNA and selected for transformants by culturing the host cells on LB-ampicillin plates.

We screened the transformants for those colonies containing an altered lipocortin DNA sequence by hybridization with $^{32}$P-labeled oligonucleotides Lipo 60 and 61 as follows: We transferred the colonies to a nitrocellulose filter and placed the filter colony side up on a fresh LB-ampicillin plate. We incubated the plate for 4 h at 37°C. We then transferred the filter to a fresh LB plate containing ampicillin and chloramphenicol (250 $\mu$g/ml) and incubated the plate overnight at 37°C. We lysed the colonies with 0.5 M NaOH-1.5 M NaCl and neutralized the lysate twice with 0.5 M Tris-HCl (pH 7.7)-1.5 M NaCl. We baked the filters in a vacuum oven for 2 h at 80°C and prehybridized them in 200 ml of 6xSSPE (0.9 M NaCl, 90 mM sodium phosphate, 6 mM sodium EDTA, pH 7.0), 0.5% SDS, 100 $\mu$g/ml tRNA and 5x Denhardt's solution at 55°C with shaking for several hours. We then hybridized the filters overnight at 55°C in 200 ml of the prehybridization mixture containing 10 pmoles of $^{32}$P-labeled oligonucleotide. We washed the filters with 6xSSPE and detected hybridization by autoradiography [see T. Maniatis, et al., supra].

We isolated a number of positive clones by this procedure. One of these clones, designated Lipo 8, was grown up and its plasmid DNA, designated pLipo8, was extracted and sequenced according to the technique of Maxam and Gilbert, supra. This sequencing analysis showed that the desired nucleotide replacements, i.e., replacement of the DNA codons encoding met 56 and 127 with codons encoding leucine, had been achieved.

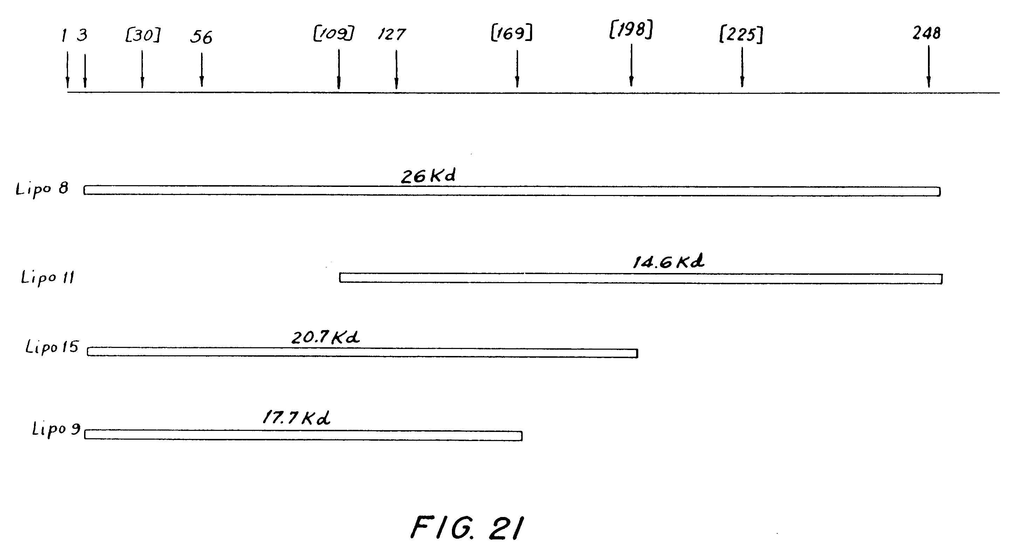

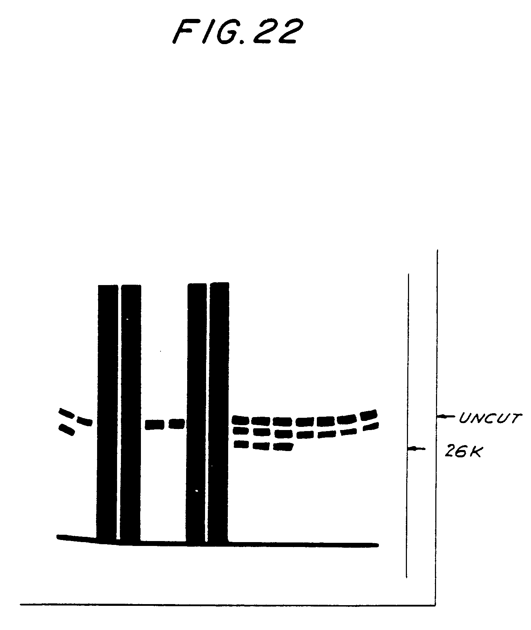

We also extracted the expressed protein from Lipo 8 and treated this preparation with 3 mg/ml of cyanogen bromide in 70% formic acid to produce lipocortin-like polypeptide fragments. We tested this crude fragment mixture for phospholipase A$_2$ inhibitory activity using the in vitro assay described earlier. Whereas wild-type lipocortin was inactivated by cyanogen bromide treatment for 1 h, the fragment mixture showed activity after 4 h of treatment. We then isolated a 26 Kd polypeptide fragment from this mixture by SDS-polyacrylamide gel electrophoresis (see Figures 21 and 22) and renatured the fragment as described in Example G(2) above. We tested this purified fragment for phospholipase A$_2$ activity using the in vitro assay and found that this large fragment of Lipo 8 displayed biological activity.

Once we had demonstrated the ability to replace met 56 and 127 without affecting the biological activity of the resulting lipocortin polypeptide fragment, we could then replace other amino acid residues of the lipocortin protein with methionine or insert methionines into various sites within the amino acid sequence of the lipocortin protein. In this way, new cleavage fragments of lipocortin with biological activity were isolated.

For example, using the procedure outlined above, we replaced the leucine at residue 109 of the amino acid sequence of lipocortin with a methionine. We hybridized mutagenic oligonucleotide Lipo 68 (see Figure 20) to a heteroduplex formed from restriction of plasmids pKK233-2 and pLipo8 (which is identical to the pLiptrc155A of Figure 6 except for the nucleotide changes of Lipo 60 and 61). We thus obtained a transformant, Lipo 11, that contains the desired nucleotide changes to replace Leu 109 with a methionine. pLipoII DNA was extracted from this transformant and sequencing indicated the desired nucleotide changes had been achieved. Clone Lipo 11 was then grown up in culture and expressed protein extracted and treated with cyanogen bromide to produce fragments. We isolated a 14.6 Kd polypeptide fragment (see Figure 21) by SDS-polyacylamide gel filtration of the treated protein extract [See Biorad Catalog/ Price List K, p. 37 (1985)] and assayed this fragment for biological activity using the in vitro assay. This assay indicated that the Lipo 11 fragment possesses phospholipase A$_2$ inhibitory activity.

Similarly, oligonucleotide Lipo 78 (see Figure 20) was used to generate a transformant, Lipo 15, which contains the desired nucleotide changes to replace Leu 198 with a methionine. Cleavage of the extracted expressed protein with cyanogen bromide followed by SDS-polyacrylamide gel filtration resulted in the purification of a 20.7 K polypeptide fragment (see Figure 21) which was then assayed for biological activity. This assay indicated that the Lipo 15 fragment possesses phospholipase A$_2$ inhibitory activity.

Alternatively, we inserted a methionine into the lipocortin amino acid sequence at residue 169 by inserting a synthetic oligonucleotide into a restriction site on the lipocortin DNA sequence, thereby creating a codon for methionine at that site. We restricted pLipo8 with BglII and ligated into that site oligonucleotides Lipo 75 and 76 (see Figure 20) using T4 ligase. These complementary oligonucleotides were constructed with sticky ends complementary to the cohesive ends of the BglII site for insertion into the plasmid. We transformed E.coli JA221 cells with this ligation mixture and selected transformants by growth on LB-ampicillin plate. We screened the transformants for those colonies containing the correct orientation of the inserted oligonucleotides by restriction mapping. One such colony, designated Lipo 9, was grown up in culture and its expressed protein extracted. The crude protein extract was treated with cyanogen bromide and subjected to SDS-polyacrylomide gel electrophoresis. A 17.7 Kd polypeptide fragment was produced and the extra amino acids introduced into the sequence by insertion of the synthetic oligonucleotides cleaved away (see Figure 21).

Thus, we have isolated at least three biologically active lipocortin-like polypeptide fragments by the use of recombinant DNA techniques -- the 26 Kd fragment of Lipo 8, the 14.6 Kd fragment of Lipo 11, and the 20.7 Kd fragment of Lipo 15. It is to be understood, however, that other lipocortin-like polypeptide fragments can be produced by the above-described procedure (e.g., by replacement of other amino acids along the lipocortin amino acid sequence with methionine). Furthermore, lipocortin-like fragments can be produced by altering the DNA sequence encoding lipocortin to introduce or insert amino acids other than methionine to yield an altered polypeptide. This polypeptide can then be cleaved by an appropriate enzyme or chemical to yield biologically active fragments (e.g., cysteines can be inserted and the protein cleaved with NTCB, 2-nitro-5-thiocyanobenzoic acid).

## ISOLATION OF A DNA SEQUENCE ENCODING N-LIPOCORTIN

We have also obtained a nucleotide sequence encoding a portion of N-lipocortin, a human lipocortin-like polypeptide displaying phospholipase $A_2$ inhibitory activity and having amino acid homology to the 37 Kd lipocortin produced according to this invention.

## I. PURIFICATION OF LIPOCORTIN AND N-LIPOCORTIN FROM HUMAN PLACENTA

A fresh placenta was packaged on ice immediately after birth and processed within 6 h as follows: We skinned the placenta, cut it into cubes and washed the tissue with ten 300 ml changes of ice cold PBS (50 mM $Na_2HPO_4$, pH 7.2, 150 mM NaCl) and 5% sucrose until the tissue was pink and no additional hemoglobin was released by the washes. The washed placenta weighed about 350 g.

We washed 30 g of the placenta tissue with 150 ml of extraction buffer (25 mM Tris-HCl, pH 7.7, 5 mM EDTA, 0.l mg/ml aprotinin, 0.l mg/ml soybean trypsin inhibitor, and 40 uM pepstatin A) and then suspended the tissue in 100 ml of the same buffer. We disrupted the preparation with a polytron for 5-7 min on ice and subjected the homogenate to centrifugation at 4°C for 15 min at 10,000 rpm in an SS34 rotor. We decanted the supernatants, disrupted the pellets with a polytron in an additional 100 ml of extraction buffer and subjected the suspension again to centrifugation at 10,000 rpm for 15 min. The supernatants of both extractions were combined and processed.

First, we subjected the extract to DEAE-cellulose chromatography on an 80 ml column (DE52, Whatman Ltd., column dimensions: 2.5 cm dia x 16 cm) that had been equilibrated with 25 mM Tris-HCl (pH 7.7). We concentrated the flow-through preparations by ultrafiltration with an Amicon PM10 membrane to 15 ml. We then subjected the concentrated preparation to centrifugation for 15 min at 18,000 xg in an SS34 rotor. The apparent ionic strength of the concentrate was approximated by measuring the conductivity of the flow-through from the ultrafiltration. Based on this value, we diluted the concentrate with water to an apparent ionic strength equal to that of 25 mM Tris-HCl (pH 7.7) (10 ml of water was added). We next subjected this preparation to a second DEAE-cellulose step on a 40 ml column and further fractionated the DEAE-flow-through by gel filtration chromatography on a 200 ml P150 column (column dimensions: 2.5 cm dia x 40 cm), which also was performed in 25 mM Tris-HCl (pH 7.7). We collected 5 ml fractions of this eluate and assayed an aliquot for phospholipase inhibitory activity using the in vitro assay described in Example A above. According to this assay, the eluate contained a single broad peak of inhibitory activity with an apparent molecular weight of 40 K as determined by gel filtration. We also characterized an aliquot of the eluate by SDS-polyacrylamide gel electrophoresis. This gel analysis indicated that the inhibitory activity in the eluate corresponded to a prominent protein band at 35 Kd. Western blotting analysis demonstrated that the 35 Kd band was immunoreactive with a rabbit antiserum against recombinant lipocortin produced in E.coli.

We next subjected the eluate from the gel filtration column to reverse phase high pressure liquid chromatography (HPLC) on a C4 column (Vydac), using a gradient of acetonitrile from 0-75% in 0.l% trifluoroacetic acid to elute the bound fragments. We found that the eluate actually contained two 35 Kd components: a natural human lipocortin component that eluted from the column with 65.8% acetonitrile and an N-lipocortin component that eluted with 65.l% acetonitrile. Only the natural lipocortin component was immunoreactive with the antibody raised against recombinant lipocortin.

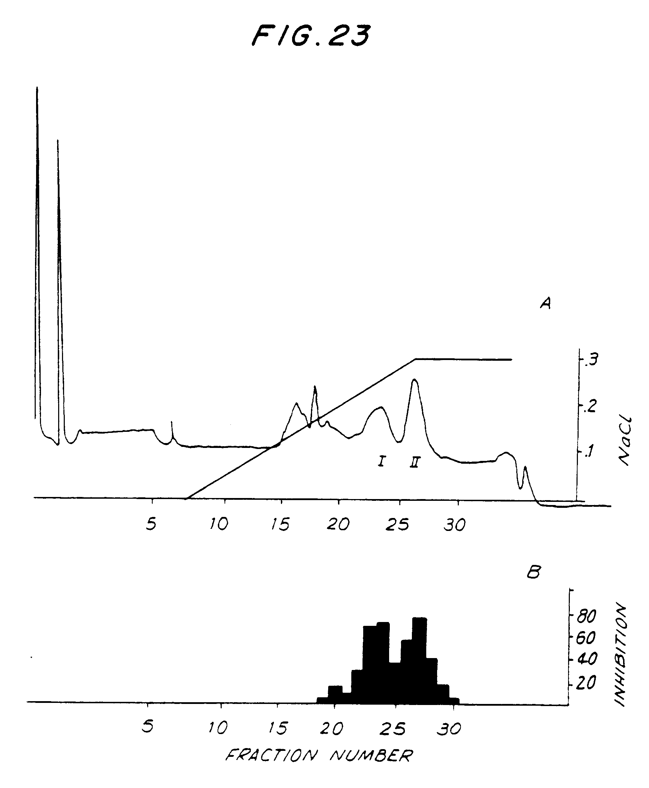

In order to further purify the two components in a manner in which we retained phospholipase A$_2$ inhibitory activity, we subjected the partially purified preparation from the gel filtration column to fast protein liquid chromatography (FPLC, Pharmacia) on a Mono S high resolution cation exchange chromatography resin (HR5/5, Pharmacia). At pH 6.0 in 50 mM MES buffer (2-morpholino-ethanesulfonic acid), both components bound to the Mono S column and were eluted with a gradient of NaCl. Figure 23 shows the elution profile of the proteins (absorbance monitored at 280 nm) (panel A) and the profile of phospholipase A$_2$ inhibitory activity (panel B) from the Mono S column. In panel A, peak I corresponds to lipocortin and peak II to N-lipocortin. Only the material in peak I was immunoreactive with the rabbit antiserum against recombinant lipocortin. Panel B clearly indicates that both lipocortin and N-lipocortin show phospholipase A$_2$ inhibitory activity. Based on their relative amounts in the purified preparations, we estimate that the specific activities of the two proteins as phospholipase inhibitors are roughly identical.

We have also analyzed these two proteins by tryptic mapping. A larger portion of a placenta was processed and the proteins purified using the protocol as detailed above. The sizes and volumes of solutions and columns were adjusted appropriately. After the two 35 Kd proteins were separated by reverse phase HPLC, 100 $\mu$g of each of the purified proteins was subjected to tryptic mapping analysis as follows: We dried the appropriate HPLC fractions under vacuum in a Speed Vac Concentrator (Savant), resuspended the resulting pellets in 400 $\mu$l of 0.1 M ammmonium bicarbonate (pH 8.0, 0.l mM CaCl$_2$), and digested the mixture with trypsin. TPCK-trypsin (Worthington, 5 $\mu$g total) was added to 100 $\mu$g of protein and the digestion performed for 16 h at 37°C. The trypsin was added in three equal aliquots: the first at time zero, the second after 4 h, and the third after 12 h of incubation. The digest was acidified with formic acid to 20% (v/v).

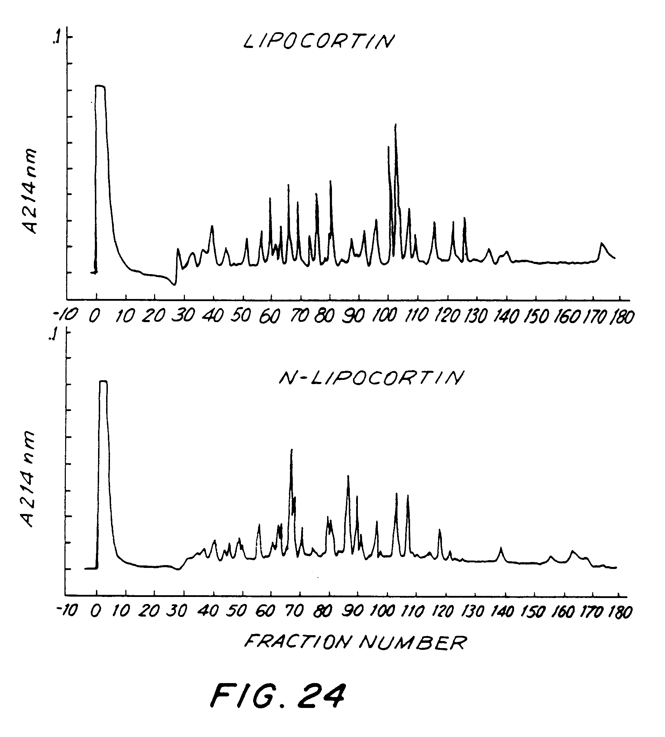

We then resolved the cleavage fragments from the trypsin digestion by reverse phase high pressure liquid chromatography at 40°C on a C18 column as described earlier in Example A. The column eluate was monitored at 214 nm. The tryptic maps for lipocortin (panel A) and N-lipocortin (panel B) are shown in Figure 24. The tryptic map of the natural human lipocortin isolated from placenta is identical to the tryptic map obtained from the 37 Kd lipocortin derived from the expression of human macrophage cDNA. DNA sequence analysis revealed two amino acid differences between the placenta and macrophage-derived lipocortins. The map for N-lipocortin shows that protein to be unique.

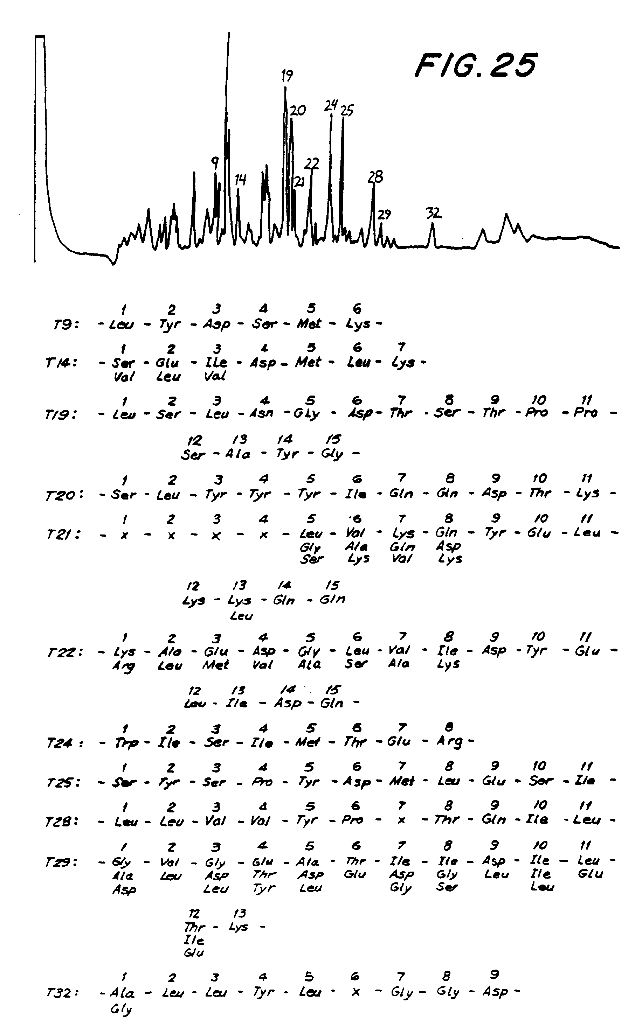

We concentrated the eluate fractions corresponding to eleven of the peaks from the tryptic map of N-lipocortin on a Speed Vac Concentrator and subjected them to amino-terminal protein sequence analysis using a gas phase sequencer (Applied Biosystems 470A). PTH-amino acids from each cycle of the Edman degradation were analyzed by reverse phase high pressure liquid chromatography using the Applied Biosystems 120A PTH analyzer. The sequences derived from these analyses are shown in Figure 25. (The "T" numbers to the left of the sequences in Figure 25 correspond to the peak numbers which are shown on the column profile at the top of the figure.)

Most of the peptide sequences of N-lipocortin are unique, although some show sequence homology to human lipocortin purified in accordance with this invention, suggesting that the two 35 Kd components -- lipocortin and N-lipocortin, respectively -- may be derived from the same family of proteins. As shown in Figure 25, to date, we have determined the primary structure of approximately one third of the N-lipocortin molecule. Since the sequences of those fragments derived from HPLC peaks containing multiple fragments can be determined using techniques known in the art, we have actually provided the data necessary to determine the primary structure of approximately 50% of the molecule.

## J. SYNTHESIS OF OLIGONUCLEOTIDE PROBES FOR N-LIPOCORTIN PROTEIN SEQUENCES

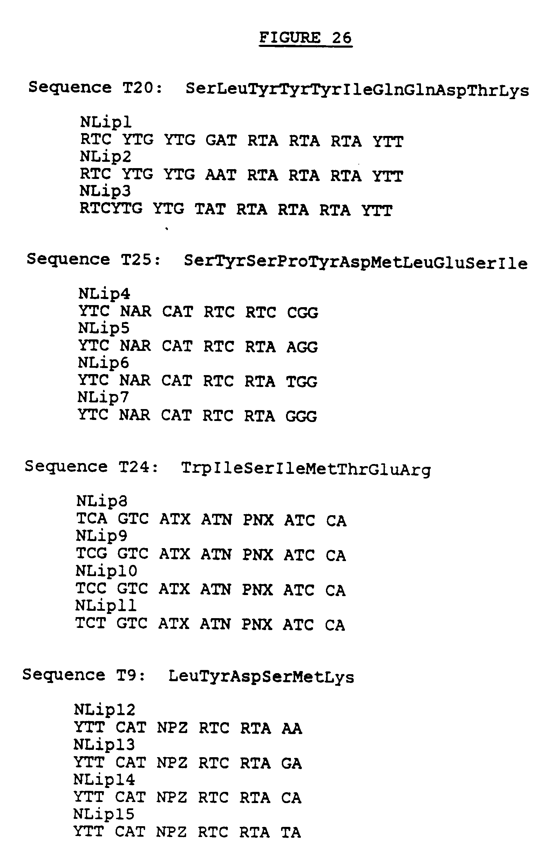

Having determined the amino acid sequences of various regions of N-lipocortin from human placenta (see Figure 25), we chemically synthesized four pools of anti sense oligonucleotide DNA probes that coded for some of those protein sequences. We used the same strategy and procedure as detailed earlier in Example B for recombinant lipocortin. The amino acid sequences of the four selected regions of N-lipocortin and all the possible nucleotide codon combinations that encode them are shown in Figure 26. Coding degeneracies are indicated as follows: N = C, T, A or G; R = A or G; Y = C or T; H = A, C or T, P = G or C; X = A, G or T; and Z = A or T.

As shown in Figure 26, the four pools of DNA probes correspond to the amino acid sequences of tryptic fragments T20, T25, T24 and T9, respectively. To reduce further the degeneracies in the probes, we prepared each pool in subpools. Oligonucleotides NLipl to NLip3, corresponding to the amino acid sequence of fragment T20, are 24-mers with 128 fold degeneracy. Oligonucleotides NLip4 to NLip7 are 18-mers with 32 fold degeneracy and are homologous to the amino acid sequence of fragment T25. Oligonucleotides NLip8 to NLipll are 20-mers with 72 fold degeneracy, corresponding to the amino acid sequence of fragment T24, and NLip12 to NLip15 are 17-mers with 128 fold degeneracy, derived from the amino acid sequence of fragment T9.

To test if our synthetic probes actually recognized human sequences, we hybridized the 3 sub-pools, NLipl, NLip2, and NLip3, which were end-labeled with $^{32}$P using $[\gamma]$-$^{32}$P-ATP and T4 polynucleotide kinase, to GeneScreen filters containing poly (A)$^+$ mRNA from human placenta, utilizing the Northern blotting technique [H. Lehrach et al., supra]. Sub-pool NLipl hybridized to an 1800 nucleotide mRNA which appears to be the same size as lipocortin mRNA.

## K. CONSTRUCTION AND SCREENING OF A HUMAN PLACENTA cDNA LIBRARY

We constructed a human cDNA library from poly (A)$^+$ mRNA isolated from human placenta using essentially the same procedure as described earlier in Example C for construction of the human macrophage cDNA library. In this embodiment, however, we extracted total RNA from the human placenta of a female fetus by the guanidine isothiocyanate method essentially as described by J. M. Chirgwin et al. [Biochemistry, 18, pp. 5294-99 (1979)]. This RNA preparation was enriched for poly (A)$^+$ RNA by two passages over an oligo(dT)-cellulose column (PL Biochem) and used to synthesize double stranded cDNA sequences which were then inserted into λgt10 and amplified in E.coli C600 hfl cells.

We screened the human placenta cDNA library with the labeled oligonucleotide probe, NLipl, using nitrocellulose filters as described below. An overnight culture of C600 hfl cells in L broth and 0.2% maltose was pelleted and resuspended in an equal amount of SM buffer. We pre-adsorbed 0.9 ml of cells with 2 x 10$^6$ phage particles at room temperature for 15 min. We diluted the suspension to 50 ml in LB plus 10 mM MgSO$_4$ and 0.7% agarose at 55°C and plated it on LB plates plus 10 mM MgSO$_4$. We screened 30 such plates. We incubated the plates at 37°C for approximately 5 h and then chilled the plates at 4°C for I h to allow the agarose to harden. We then transferred the λ phage particles from the plaque library plates to S&S nitrocellulose filters by placing the filters onto the plates containing the recombinant plaques for 5 min. We then lifted the filters from the plates, lysed the phages on the filters by placing the filters onto a pool of 0.5 N NaOH-1.5 M NaCl for 5 min and neutralized and submerged the filters in I M Tris-HCl-1.5 M NaCl (pH 7.0). The filters were then air-dried and baked at 80°C for 2 h.

We prehybridized and hybridized the treated filters to the oligonucleotide probe, NLipl, in 0.2% polyvinylpyrrolidone (M.W. 40,000), 0.2% ficoll (M.W. 400,000), 0.2% bovine serum albumin, 0.05 M Tris-HCl (pH 7.5), IM sodium chloride, 0.l% sodium pyrophosphate, l% SDS, 10% dextran sulfate (M.W. 500,000) and denatured salmon sperm DNA (≧100 μg/ml) according to manufacturer's specifications (New England Nuclear) for Colony/Plaque Screen™ membranes. We detected hybridizing cDNA sequences by autoradiography.

By means of this technique, we picked six positive plaques and rescreened at lower density using the same probe. We isolated the DNA of these clones, digested the DNA with EcoRI, and hybridized the sequences with the NLipl probe using the Southern blot technique [E. M. Southern, supra]. The cDNA inserts of all six clones hybridized to the NLipl probe.

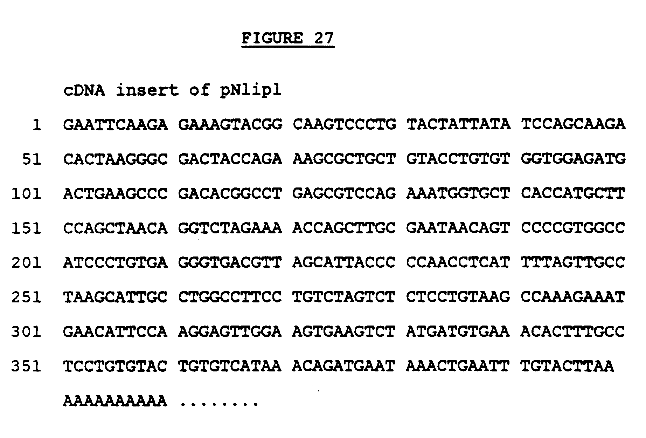

We next restricted the DNA of these phages with EcoRI and isolated the cDNA inserts. Digestion of one of the clones, λ-Nlipo21-2, with EcoRI resulted in an approximately 500 base pair fragment which we subcloned into plasmid pUC9 to produce plasmid pNlipl. We sequenced this plasmid by the method of Maxam and Gilbert [A. M. Maxam and W. Gilbert, supra]. As depicted in Figure 27, the plasmid carries a 394 base pair cDNA insert that contains 99 base pairs of the N-lipocortin coding sequence (corresponding to 33 amino acids), and 295 base pairs of the N-lipocortin 3' non-coding region, including a poly (A) addition site and an approximately 50 base pair poly (A) tail. The DNA coding sequence of N-lipocortin not only contains the sequence corresponding to the oligonucleotide probe derived from tryptic fragment T20, i.e., NLipl, but also the sequence corresponding to tryptic fragment T32 (see Figure 25), thus confirming that we have cloned a portion of the gene encoding the N-lipocortin protein. This cDNA sequence as well as the oligonucleotide probes described in Example J can then be used as probes to screen for and isolate the DNA sequence encoding full length N-lipocortin.

Although clone λ-Nlipo21-2 contains the N-lipocortin DNA sequence encoding only a small region of the protein, the structural homology between lipocortin and N-lipocortin is borne out by this region. As shown in Table 4 below, 11 of the last 17 amino acids at the carboxyl terminus of the two proteins are identical. Of the five differences, most are conservative amino acid substitutions. Based on the similarity in the

phospholipase $A_2$ inhibitory activity of the two proteins and the similarity in the protein and DNA sequences of the proteins, we conclude that N-lipocortin and lipocortin represent a family of related proteins.

## Table IV

### SEQUENCE HOMOLOGY AT C-TERMINUS OF LIPOCORTIN AND N-LIPOCORTIN

```
LIPO:     GlnLysMetTyrGlyIleSerLeuCysGlnAlaIleLeu-
N-LIPO:   LysArgLysTyrGlyLysSerLeuTyrTyrTyrIleGln-

LIPO:     AspGluThrLysGlyAspTyrGluLysIleLeuValAla-
N-LIPO:   GlnAspThrLysGlyAspTyrGlnLysAlaLeuLeuTyr-

LIPO:     LeuCysGlyGlyAsn
N-LIPO:   LeuCysGlyGlyAspAsp
```

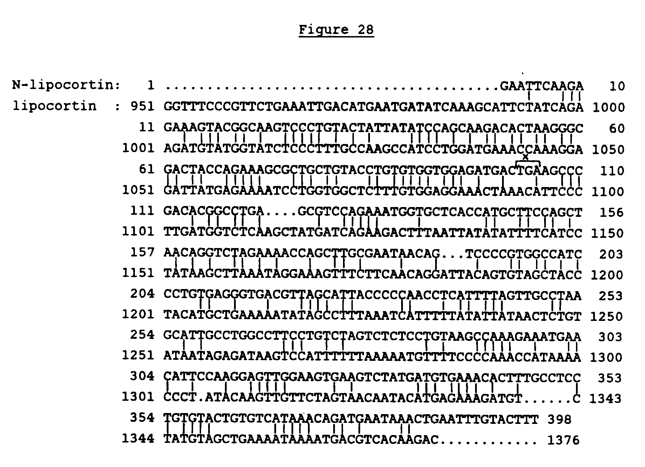

A comparison of the nucleotide sequences of the lipocortin and N-lipocortin of this invention shows approximately 60% homology (see Figure 28).

Microorganisms and recombinant DNA sequences prepared by the processes described herein are exemplified by a culture deposited in the culture collection of In Vitro International, Inc., Ann Arbor, Michigan. The culture was deposited on January 8, 1986 and is identified as follows:

E.coli JA221(pNLipl) : IVI No.10093 (now transferred to ATCC Deposit No.68811).

## IMPROVING THE YIELD AND ACTIVITY OF HUMAN LIPOCORTIN-LIKE POLYPEPTIDES PRODUCED IN ACCORDANCE WITH THIS INVENTION

The level of production of a protein is governed by three major factors: the number of copies of its gene within the cell, the efficiency with which those gene copies are transcribed and the efficiency with which they are translated. Efficiency of transcription and translation (which together comprise expression) is in turn dependent upon nucleotide sequences normally situated ahead of the desired coding sequence. These nucleotide sequences or expression control sequences define, inter alia, the location at which RNA polymerase interacts to initiate transcription (the promoter sequence) and at which ribosomes bind and interact with the mRNA (the product of transcription) to initiate translation. Not all such expression control sequences function with equal efficiency. It is thus of advantage to separate the specific lipocortin coding sequences of this invention from their adjacent nucleotide sequences and to fuse them instead to other known expression control sequences so as to favor higher levels of expression and production of lipocortin-like polypeptides. This having been achieved, the newly engineered DNA fragments may be inserted into higher copy number plasmids or bacteriophage derivatives in order to increase the number of gene copies within the cell and thereby further to improve the yield of expressed lipocortin-like polypeptides.

Several expression control sequences may be employed as described above. These include the operator, promoter and ribosome binding and interaction sequences (including sequences such as the Shine-Dalgarno sequences) of the lactose operon of E.coli ("the lac system"), the corresponding sequences of the tryptophan synthetase system of E.coli ("the trp system"), the major operator and promoter regions of phage λ ($O_LP_L$ as described above and $O_RP_R$), a control region of filamentous single-stranded DNA phages, the tac or trc system, the promoter for 3-phosphoglycerate kinase or other glycolytic enzymes, the promoters of acid phosphatase, e.g., Pho5, the promoters of the yeast α-mating factors, promoters for mammalian cells such as the SV40 early and late promoters, adenovirus late promoter and metallothionine promoter, and other sequences which control the expression of genes of prokaryotic or eukaryotic cells and their viruses or combinations thereof.

Therefore, to improve the production of the lipocortin-like polypeptides of this invention, the DNA sequences for those polypeptides may be prepared as before and inserted into a recombinant DNA molecule closer to its former expression control sequence or under the control of one of the above improved expression control sequences. Such methods are known in the art.

Other methods useful to improve the efficiency of translation involve the insertion of chemically or enzymatically prepared oligonucleotides in front of the initiating codon of the lipocortin-related DNA sequences of this invention or the replacement of codons at the N-terminal end of the DNA sequence with those chemically or enzymatically prepared oligonucleotides. By this procedure, a more optimal primary and higher order structure of the messenger RNA can be obtained. More specifically, a sequence can be so designed that the initiating AUG codon occurs in a readily accessible position (i.e., not masked by secondary structure) either at the top of a hairpin or in other single-stranded regions. The position and sequence of the aforementioned Shine-Dalgarno segment can similarly be optimized. The importance of the general structure (folding) of the messenger RNA has been documented (D. Iserentant and W. Fiers, "Secondary Structure Of mRNA And Efficiency Of Translation Initiation", Gene, 9, pp. 1-12 (1980)).

Further increases in the cellular yield of the lipocortin-like polypeptides of this invention may be achieved by increasing the number of genes that can be utilized in the cell. This may be achieved by insertion of the lipocortin gene (with or without its transcription and translation control elements) into a higher copy number plasmid or into a temperature-controlled copy number plasmid (i.e., a plasmid which carries a mutation such that the copy number of the plasmid increases after shifting the temperature (B. Uhlin et al., "Plasmids With Temperature-Dependent Copy Number For Amplification Of Cloned Genes And Their Products", Gene, 6, pp. 91-106 (1979)).

Alternatively, an increase in gene dosage can be achieved, for example, by insertion of recombinant DNA molecules, engineered in the manner described above, into the temperate bacteriophage λ, most simply by digestion of the plasmid with a restriction enzyme, to give a linear molecule which is then mixed with a restricted phage λ cloning vehicle (e.g., of the type described by N. E. Murray et al., "Lambdoid Phages That Simplify The Recovery Of In Vitro Recombinants", Mol. Gen. Genet., 150, pp. 53-61 (1977), and N. E. Murray et al., "Molecular Cloning Of The DNA Ligase Gene From Bacteriophage T4", J. Mol. Biol., 132, pp. 493-505 (1979)), and the recombinant DNA molecule produced by incubation with DNA ligase. The desired recombinant phage is then selected and used to lysogenize a host strain of E.coli.

Therefore, it should be understood that the lipocortin-like polypeptide coding sequences of this invention may be removed from the disclosed vectors and inserted into other expression vectors, as previously described (supra) and these vectors employed in various hosts, as previously described (supra) to improve the production of the human lipocortin-like polypeptides of this invention.

While we have hereinbefore presented a number of embodiments of this invention, it is apparent that our basic construction can be altered to provide other embodiments which utilize the processes and compositions of this invention. Therefore, it will be appreciated that the scope of this invention is to be defined by the claims appended hereto rather than by the specific embodiments which have been presented hereinbefore by way of example.

## Claims

**Claims for the following Contracting States : BE, CH, DE, FR, GB, IT, LI, LU, NL, SE**

1. A recombinant DNA molecule characterized by a DNA sequence coding for a human polypeptide having phospholipase $A_2$ inhibitory activity and the immunological activity of a human lipocortin, and being selected from the group consisting of:

   (a) the cDNA insert of λLC (ATCC Deposit No. 68812) bounded at both ends by EcoRI restriction sites,

   (b) DNA sequences that hybridize to the DNA insert of claim 1(a) and which encode a human polypeptide having phospholipase $A_2$ inhibitory activity and an immunological activity of the human lipocortin encoded by the cDNA insert of part (a) above, and

   (c) DNA sequences that are degenerate as a result of the genetic code as to the DNA insert and sequences of (a) and (b) above.

EP 0 209 568 B1

2. The recombinant DNA molecule according to claim 1, wherein the DNA sequence consists of:

```
ATGGCAATGGTATCAGAATTCCTCAAGCAGGCCTGGTTTATTGAAAATGAAGAGC
AGGAATATGTTCAAACTGTGAAGTCATCCAAAGGTGGTCCCGGATCAGCGGTGAG
CCCCTATCCTACCTTCAATCCATCCTCGGATGTCGCTGCCTTGCATAAGGCCATA
ATGGTTAAAGGTGTGGATGAAGCAACCATCATTGACATTCTAACTAAGCGAAACA
ATGCACAGCGTCAACAGATCAAAGCAGCATATCTCCAGGAAACAGGAAAGCCCCT
GGATGAAACACTTAAGAAAGCCCTTACAGGTCACCTTGAGGAGGTTGTTTTAGCT
CTGCTAAAAACTCCAGCGCAATTTGATGCTGATGAACTTCGTGCTGCCATGAAGG
GCCTTGGAACTGATGAAGATACTCTAATTGAGATTTTGGCATCAAGAACTAACAA
AGAAATCAGAGACATTAACAGGGTCTACAGAGAGGAACTGAAGAGAGATCTGGCC
AAAGACATAACCTCAGACACATCTGGAGATTTTCGGAACGCTTTGCTTTCTCTTG
CTAAGGGTGACCGATCTGAGGACTTTGGTGTGAATGAAGACTTGGCTGATTCAGA
TGCCAGGGCCTTGTATGAAGCAGGAGAAAGGAGAAAGGGGACAGACGTAAACGTG
TTCAATACCATCCTTACCACCAGAAGCTATCCACAACTTCGCAGAGTGTTTCAGA
AATACACCAAGTACAGTAAGCATGACATGAACAAAGTTCTGGACCTGGAGTTGAA
AGGTGACATTGAGAAATGCCTCACAGCTATCGTGAAGTGCGCCACAAGCAAACCA
GCTTTCTTTGCAGAGAAGCTTCATCAAGCCATGAAAGGTGTTGGAACTCGCCATA


AGGCATTGATCAGGATTATGGTTTCCCGTTCTGAAATTGACATGAATGATATCAA
AGCATTCTATCAGAAGATGTATGGTATCTCCCTTTGCCAAGCCATCCTGGATGAA
ACCAAAGGAGATTATGAGAAAATCCTGGTGGCTCTTTGTGGAGGAAACTAA.
```

3. A recombinant DNA molecule selected from the group consisting of:
   (a) the cDNA insert of pNLip1 (ATCC Deposit No.68811) bounded at both ends by EcoRI restriction sites,
   (b) DNA sequences that hybridize to the DNA insert of claim 3(a) and which encode a human polypeptide having phospholipase A$_2$ inhibitory activity and an immunological activity of the human lipocortin encoded by the cDNA insert of part (a) above, and
   (c) DNA sequences that are degenerate as a result of the genetic code as to the DNA insert and sequences of (a) and (b) above.

4. A recombinant DNA molecule characterized by the sequence:

```
GAATTCAAGAGAAAGTACGGCAAGTCCCTGTACTATTATATCCAGCAAGACACTA
AGGGCGACTACCAGAAAGCGCTGCTGTACCTGTGTGGTGGAGATGACTGA.
```

5. The recombinant DNA molecule according to claim 1 or 2, wherein said DNA sequence is operatively linked to an expression control sequence in the molecule.

30

6. The recombinant DNA molecule according to claim 5, wherein said expression control sequence is selected from the group consisting of the lac system, the β-lactamase system, the trp system, the tac system, the trc system, major operator and promoter regions of phage λ, the control region of fd coat protein, the promoter for 3-phosphoglycerate kinase or other glycolytic enzymes, the promoters of acid phosphatases the promoters of the yeast α-mating factors, promoters for mammalian cells, the SV40 early and late promoters, adenovirus late promoter and metallothionine promoter, and other sequences which control the expression of genes of prokaryotic or eukaryotic cells and their viruses and combinations thereof.

7. The recombinant DNA molecule according to claim 5, which is selected from the group consisting of pLipPLT4A, pLipPLT4T and pSVL9109.

8. The recombinant DNA molecule pBgl20 (ATCC Deposit No.74106).

9. The recombinant DNA molecule pNLip1 (ATCC Deposit No.68811).

10. A unicellular host transformed with at least one recombinant DNA molecule according to claim 5.

11. The transformed host according to claim 10, selected from the group consisting of strains of E.coli, Pseudomonas, Bacillus, Streptomyces, yeast cells, other fungi, mouse cells or other animal cell hosts, plant cell hosts, and human tissue cells.

12. A human polypeptide having phospholipase A$_2$ inhibitory activity and the immunological activity of a human lipocortin, the polypeptide being selected from the group consisting of:
    (a)

```
MetAlaMetValSerGluPheLeuLysGlnAlaTrpPheIleGluAsnGluGlu
GlnGluTyrValGlnThrValLysSerSerLysGlyGlyProGlySerAlaVal
```

SerProTyrProThrPheAsnProSerSerAspValAlaAlaLeuHisLysAla
IleMetValLysGlyValAspGluAlaThrIleIleAspIleLeuThrLysArg
AsnAsnAlaGlnArgGlnGlnIleLysAlaAlaTyrLeuGlnGluThrGlyLys
ProLeuAspGluThrLeuLysLysAlaLeuThrGlyHisLeuGluGluValVal
LeuAlaLeuLeuLysThrProAlaGlnPheAspAlaAspGluLeuArgAlaAla
MetLysGlyLeuGlyThrAspGluAspThrLeuIleGluIleLeuAlaSerArg
ThrAsnLysGluIleArgAspIleAsnArgValTyrArgGluGluLeuLysArg
AspLeuAlaLysAspIleThrSerAspThrSerGlyAspPheArgAsnAlaLeu
LeuSerLeuAlaLysGlyAspArgSerGluAspPheGlyValAsnGluAspLeu
AlaAspSerAspAlaArgAlaLeuTyrGluAlaGlyGluArgArgLysGlyThr
AspValAsnValPheAsnThrIleLeuThrThrArgSerTyrProGlnLeuArg
ArgValPheGlnLysTyrThrLysTyrSerLysHisAspMetAsnLysValLeu
AspLeuGluLeuLysGlyAspIleGluLysCysLeuThrAlaIleValLysCys
AlaThrSerLysProAlaPhePheAlaGluLysLeuHisGlnAlaMetLysGly
ValGlyThrArgHisLysAlaLeuIleArgIleMetValSerArgSerGluIle
AspMetAsnAspIleLysAlaPheTyrGlnLysMetTyrGlyIleSerLeuCys
GlnAlaIleLeuAspGluThrLysGlyAspTyrGluLysIleLeuValAlaLeu
CysGlyGlyAsn;

(b)

AlaMetValSerGluPheLeuLysGlnAlaTrpPheIleGluAsnGluGluGln
GluTyrValGlnThrValLysSerSerLysGlyGlyProGlySerAlaValSer
ProTyrProThrPheAsnProSerSerAspValAlaAlaLeuHisLysAlaIle
MetValLysGlyValAspGluAlaThrIleIleAspIleLeuThrLysArgAsn
AsnAlaGlnArgGlnGlnIleLysAlaAlaTyrLeuGlnGluThrGlyLysPro
LeuAspGluThrLeuLysLysAlaLeuThrGlyHisLeuGluGluValValLeu
AlaLeuLeuLysThrProAlaGlnPheAspAlaAspGluLeuArgAlaAlaMet
LysGlyLeuGlyThrAspGluAspThrLeuIleGluIleLeuAlaSerArgThr
AsnLysGluIleArgAspIleAsnArgValTyrArgGluGluLeuLysArgAsp
LeuAlaLysAspIleThrSerAspThrSerGlyAspPheArgAsnAlaLeuLeu
SerLeuAlaLysGlyAspArgSerGluAspPheGlyValAsnGluAspLeuAla
AspSerAspAlaArgAlaLeuTyrGluAlaGlyGluArgArgLysGlyThrAsp
ValAsnValPheAsnThrIleLeuThrThrArgSerTyrProGlnLeuArgArg
ValPheGlnLysTyrThrLysTyrSerLysHisAspMetAsnLysValLeuAsp
LeuGluLeuLysGlyAspIleGluLysCysLeuThrAlaIleValLysCysAla
ThrSerLysProAlaPhePheAlaGluLysLeuHisGlnAlaMetLysGlyVal

```
GlyThrArgHisLysAlaLeuIleArgIleMetValSerArgSerGluIleAsp
MetAsnAspIleLysAlaPheTyrGlnLysMetTyrGlyIleSerLeuCysGln
AlaIleLeuAspGluThrLysGlyAspTyrGluLysIleLeuValAlaLeuCys
GlyGlyAsn;
```

and

(c) polypeptide fragments of the polypeptide of part (a) or (b) above displaying a biological or immunological activity of the lipocortin defined in part (a) or (b) above.

13. An N-lipocortin polypeptide characterized by the following properties:
    (a) having the ability to inhibit the enzyme phospholipase $A_2$,
    (b) comprising the tryptic fragments:
        (1) LeuTyrAspSerMetLys;
        (2) LeuSerLeuAsnGlyAspThrSer ThrProProSerAlaTyrGly;
        (3) SerLeuTyrTyrTyrIleGln GlnAspThrLys;
        (4) TrpIleSerIleMetThrGluArg;
        (5) SerTyrSerProTyrAspMet LeuGluSerIle;
        (6) LeuLeuValValTyrProX ThrGlnIleLeu;
    wherein x is any amino acid, and
    (c) having a carboxy-terminus consisting of the amino acid sequence,

```
LysArgLysTyrGlyLysSerLeuTyrTyrTyrIleGlnGlnAspThrLysGly
AspTyrGlnLysAlaLeuLeuTyrLeuCysGlyGlyAspAsp.
```

14. A human lipocortin-like polypeptide encoded by the recombinant DNA molecules of claim 1 or 2.

15. The polypeptide according to claim 12, wherein the polypeptide is selected from the group consisting of:

(a)

GlyGlyProGlySerAlaValSerProTyrProThrPheAsnProSerSerAsp
ValAlaAlaLeuHisLysAlaIleMetValLysGlyValAspGluAlaThrIle
IleAspIleLeuThrLysArgAsnAsnAlaGlnArgGlnGlnIleLysAlaAla
TyrLeuGlnGluThrGlyLysProLeuAspGluThrLeuLysLysAlaLeuThr
GlyHisLeuGluGluValValLeuAlaLeuLeuLysThrProAlaGlnPheAsp
AlaAspGluLeuArgAlaAlaMetLysGlyLeuGlyThrAspGluAspThrLeu
IleGluIleLeuAlaSerArgThrAsnLysGluIleArgAspIleAsnArgVal
TyrArgGluGluLeuLysArgAspLeuAlaLysAspIleThrSerAspThrSer
GlyAspPheArgAsnAlaLeuLeuSerLeuAlaLysGlyAspArgSerGluAsp
PheGlyValAsnGluAspLeuAlaAspSerAspAlaArgAlaLeuTyrGluAla
GlyGluArgArgLysGlyThrAspValAsnValPheAsnThrIleLeuThrThr
ArgSerTyrProGlnLeuArgArgValPheGlnLysTyrThrLysTyrSerLys
HisAspMetAsnLysValLeuAspLeuGluLeuLysGlyAspIleGluLysCys
LeuThrAlaIleValLysCysAlaThrSerLysProAlaPhePheAlaGluLys
LeuHisGlnAlaMetLysGlyValGlyThrArgHisLysAlaLeuIleArgIle
MetValSerArgSerGluIleAspMetAsnAspIleLysAlaPheTyrGlnLys
MetTyrGlyIleSerLeuCysGlnAlaIleLeuAspGluThrLysGlyAspTyr
GluLysIleLeuValAlaLeuCysGlyGly;

(b)

GlyGlyProGlySerAlaValSerProTyrProThrPheAsnProSerSerAsp
ValAlaAlaLeuHisLysAlaIleMetValLysGlyValAspGluAlaThrIle
IleAspIleLeuThrLysArgAsnAsnAlaGlnArgGlnGlnIleLysAlaAla
TyrLeuGlnGluThrGlyLysProLeuAspGluThrLeuLysLysAlaLeuThr
GlyHisLeuGluGluValValLeuAlaLeuLeuLysThrProAlaGlnPheAsp
AlaAspGluLeuArgAlaAlaMetLysGlyLeuGlyThrAspGluAspThrLeu
IleGluIleLeuAlaSerArgThrAsnLysGluIleArgAspIleAsnArgVal
TyrArgGluGluLeuLysArgAspLeuAlaLysAspIleThrSerAspThrSer
GlyAspPheArgAsnAlaLeuLeuSerLeuAlaLysGlyAspArgSerGluAsp
PheGlyValAsnGluAspLeuAlaAspSerAspAlaArgAlaLeuTyrGluAla
GlyGluArgArgLysGlyThrAspValAsnValPheAsnThrIleLeuThrThr;

(c)

```
GlyGlyProGlySerAlaValSerProTyrProThrPheAsnProSerSerAsp
ValAlaAlaLeuHisLysAlaIleMetValLysGlyValAspGluAlaThrIle
IleAspIleLeuThrLysArgAsnAsnAlaGlnArgGlnGlnIleLysAlaAla
TyrLeuGlnGluThrGlyLysProLeuAspGluThrLeuLysLysAlaLeuThr
GlyHisLeuGluGluValValLeuAlaLeuLeuLysThrProAlaGlnPheAsp
AlaAspGluLeuArgAlaAlaMetLysGlyLeuGlyThrAspGluAspThrLeu
IleGluIleLeuAlaSerArgThrAsnLysGluIleArgAspIleAsnArgVal
TyrArgGluGluLeuLysArgAspLeuAlaLysAspIleThrSerAspThrSer
GlyAspPheArgAsnAlaLeuLeuSerLeuAlaLysGlyAspArgSerGluAsp
PheGlyValAsnGluAspLeuAlaAspSerAspAlaArgAlaLeuTyrGluAla
GlyGlu;
```

(d)

```
ValSerGluPheLeuLysGlnAlaTrpPheIleGluAsnGluGluGlnGluTyr
ValGlnThrValLysSerSerLysGlyGlyProGlySerAlaValSerProTyr
ProThrPheAsnProSerSerAspValAlaAlaLeuHisLysAlaIleLeuVal
LysGlyValAspGluAlaThrIleIleAspIleLeuThrLysArgAsnAsnAla
GlnArgGlnGlnIleLysAlaAlaTyrLeuGlnGluThrGlyLysProLeuAsp
GluThrLeuLysLysAlaLeuThrGlyHisLeuGluGluValValLeuAlaLeu
LeuLysThrProAlaGlnPheAspAlaAspGluLeuArgAlaAlaLeuLysGly
LeuGlyThrAspGluAspThrLeuIleGluIleLeuAlaSerArgThrAsnLys
GluIleArgAspIleAsnArgValTyrArgGluGluLeuLysArgAspLeuAla
LysAspIleThrSerAspThrSerGlyAspPheArgAsnAlaLeuLeuSerLeu
AlaLysGlyAspArgSerGluAspPheGlyValAsnGluAspLeuAlaAspSer
AspAlaArgAlaLeuTyrGluAlaGlyGluArgArgLysGlyThrAspValAsn
ValPheAsnThrIleLeuThrThrArgSerTyrProGlnLeuArgArgValPhe
GlnLysTyrThrLysTyrSerLysHisAsp;
```

(e)

```
ValSerGluPheLeuLysGlnAlaTrpPheIleGluAsnGluGluGlnGluTyr
ValGlnThrValLysSerSerLysGlyGlyProGlySerAlaValSerProTyr
ProThrPheAsnProSerSerAspValAlaAlaLeuHisLysAlaIleLeuVal
LysGlyValAspGluAlaThrIleIleAspIleLeuThrLysArgAsnAsnAla
GlnArgGlnGlnIleLysAlaAlaTyrLeuGlnGluThrGlyLysProLeuAsp
GluThrLeuLysLysAlaLeuThrGlyHisLeuGluGluValVal;
```

and

(f)

ValSerGluPheLeuLysGlnAlaTrpPheIleGluAsnGluGluGlnGluTyr
ValGlnThrValLysSerSerLysGlyGlyProGlySerAlaValSerProTyr
ProThrPheAsnProSerSerAspValAlaAlaLeuHisLysAlaIleLeuVal
LysGlyValAspGluAlaThrIleIleAspIleLeuThrLysArgAsnAsnAla
GlnArgGlnGlnIleLysAlaAlaTyrLeuGlnGluThrGlyLysProLeuAsp
GluThrLeuLysLysAlaLeuThrGlyHisLeuGluGluValValLeuAlaLeu
LeuLysThrProAlaGlnPheAspAlaAspGluLeuArgAlaAlaLeuLysGly
LeuGlyThrAspGluAspThrLeuIleGluIleLeuAlaSerArgThrAsnLys
GluIleArgAspIleAsnArgValTyrArgGluGluLeuLysArgAspLeuAla
LysAspIleThrSerAspThrSerGlyAspPheArgAsnAlaLeuLeuSerLeu
AlaLysGlyAspArgSerGluAspPheGlyValAsnGluAsp.

**16.** The polypeptide according to claim 12, wherein the polypeptide has the amino acid sequence:

GlyGlyProGlySerAlaValSerProTyrProThrPheAsnProSerSerAsp
ValAlaAlaLeuHisLysAlaIleMetValLysGlyValAspGluAlaThrIle
IleAspIleLeuThrLysArgAsnAsnAlaGlnArgGlnGlnIleLysAlaAla
TyrLeuGlnGluThrGlyLysProLeuAspGluThrLeuLysLysAlaLeuThr
GlyHisLeuGluGluValValLeuAlaLeuLeuLysThrProAlaGlnPheAsp
AlaAspGluLeuArgAlaAlaMetLysGlyLeuGlyThrAspGluAspThrLeu
IleGluIleLeuAlaSerArgThrAsnLysGluIleArgAspIleAsnArgVal
TyrArgGluGluLeuLysArgAspLeuAlaLysAspIleThrSerAspThrSer
GlyAspPheArgAsnAlaLeuLeuSerLeuAlaLysGlyAspArgSerGluAsp
PheGlyValAsnGluAspLeuAlaAspSerAspAlaArgAlaLeuTyrGluAla
GlyGluArgArgLysGlyThrAspValAsnValPheAsnThrIleLeuThrThr
ArgSerTyrProGlnLeuArgArgValPheGlnLysTyrThrLysTyrSerLys
HisAspMetAsnLysValLeuAspLeuGluLeuLysGlyAspIleGluLysCys
LeuThrAlaIleValLysCysAlaThrSerLysProAlaPhePheAlaGluLys
LeuHisGlnAlaMetLysGlyValGlyThrArgHisLysAlaLeuIleArgIle
MetValSerArgSerGluIleAspMetAsnAspIleLysAlaPheTyrGlnLys
MetTyrGlyIleSerLeuCysGlnAlaIleLeuAspGluThrLysGlyAspTyr
GluLysIleLeuValAlaLeuCysGlyGlyAsn.

36

**17.** A DNA sequence consisting of:

```
ATGGCAATGGTATCAGAATTCCTCAAGCAGGCCTGGTTTATTGAAAATGAAGAGC
AGGAATATGTTCAAACTGTGAAGTCATCCAAAGGTGGTCCCGGATCAGCGGTGAG
CCCCTATCCTACCTTCAATCCATCCTCGGATGTCGCTGCCTTGCATAAGGCCATA
ATGGTTAAAGGTGTGGATGAAGCAACCATCATTGACATTCTAACTAAGCGAAACA
ATGCACAGCGTCAACAGATCAAAGCAGCATATCTCCAGGAAACAGGAAAGCCCCT
GGATGAAACACTTAAGAAAGCCCTTACAGGTCACCTTGAGGAGGTTGTTTTAGCT
CTGCTAAAAACTCCAGCGCAATTTGATGCTGATGAACTTCGTGCTGCCATGAAGG
GCCTTGGAACTGATGAAGATACTCTAATTGAGATTTTGGCATCAAGAACTAACAA
AGAAATCAGAGACATTAACAGGGTCTACAGAGAGGAACTGAAGAGAGATCTGGCC
AAAGACATAACCTCAGACACATCTGGAGATTTTCGGAACGCTTTGCTTTCTCTTG
CTAAGGGTGACCGATCTGAGGACTTTGGTGTGAATGAAGACTTGGCTGATTCAGA
TGCCAGGGCCTTGTATGAAGCAGGAGAAAGGAGAAAGGGGACAGACGTAAACGTG
TTCAATACCATCCTTACCACCAGAAGCTATCCACAACTTCGCAGAGTGTTTCAGA
AATACACCAAGTACAGTAAGCATGACATGAACAAAGTTCTGGACCTGGAGTTGAA
AGGTGACATTGAGAAATGCCTCACAGCTATCGTGAAGTGCGCCACAAGCAAACCA
GCTTTCTTTGCAGAGAAGCTTCATCAAGCCATGAAAGGTGTTGGAACTCGCCATA
AGGCATTGATCAGGATTATGGTTTCCCGTTCTGAAATTGACATGAATGATATCAA
AGCATTCTATCAGAAGATGTATGGTATCTCCCTTTGCCAAGCCATCCTGGATGAA
ACCAAAGGAGATTATGAGAAATCCTGGTGGCTCTTTGTGGAGGAAACTAA.
```

**18.** A DNA sequence consisting of:

```
GAATTCAAGAGAAAGTACGGCAAGTCCCTGTACTATTATATCCAGCAAGACACTA
AGGGCGACTACCAGAAAGCGCTGCTGTACCTGTGTGGTGGAGATGACTGA.
```

**19.** A method for producing a human polypeptide having phospholipase A$_2$ inhibitory activity and the immunological activity of a human lipocortin comprising the steps of culturing a host transformed by a recombinant DNA molecule according to claim 5 and collecting said polypeptide.

**20.** A pharmaceutically acceptable composition useful in the treatment of arthritic, allergic, dermatologic, ophthalmic, and collagen diseases and other disorders involving inflammatory processes which comprises a pharmaceutically effective amount of at least one polypeptide selected from the group consisting of polypeptides according to claim 12, 14, 15 or 16.

**21.** A pharmaceutically acceptable composition useful in the treatment of arthritic, allergic, dermatologic, ophthalmic, and collagen diseases and other disorders involving inflammatory processes which comprises a pharmaceutically effective amount of at least one polypeptide according to claim 13.

**Claims for the following Contracting State : AT**

1. A unicellular host transformed with at least one recombinant DNA molecule characterized by a DNA sequence coding for a human polypeptide having phospholipase $A_2$ inhibitory activity and the immunological activity of a human lipocortin, and being selected from the group consisting of:

   (a) the cDNA insert of λLC (ATCC Deposit No. 68812) bounded at both ends by EcoRI restriction sites,

   (b) DNA sequences that hybridize to the DNA insert of claim 1(a) and which encode a human polypeptide having phospholipase $A_2$ inhibitory activity and an immunological activity of the human lipocortin encoded by the cDNA insert of part (a) above, and

   (c) DNA sequences that are degenerate as a result of the genetic code as to the DNA insert and sequences of (a) and (b) above,

   wherein said DNA sequence is operatively linked to an expression control sequence in the molecule.

2. The transformed host according to claim 1, wherein the DNA sequence consists of:

```
ATGGCAATGGTATCAGAATTCCTCAAGCAGGCCTGGTTTATTGAAAATGAAGAGC
AGGAATATGTTCAAACTGTGAAGTCATCCAAAGGTGGTCCCGGATCAGCGGTGAG
CCCCTATCCTACCTTCAATCCATCCTCGGATGTCGCTGCCTTGCATAAGGCCATA
ATGGTTAAAGGTGTGGATGAAGCAACCATCATTGACATTCTAACTAAGCGAAACA
ATGCACAGCGTCAACAGATCAAAGCAGCATATCTCCAGGAAACAGGAAAGCCCCT
GGATGAAACACTTAAGAAAGCCCTTACAGGTCACCTTGAGGAGGTTGTTTTAGCT
CTGCTAAAAACTCCAGCGCAATTTGATGCTGATGAACTTCGTGCTGCCATGAAGG
GCCTTGGAACTGATGAAGATACTCTAATTGAGATTTTGGCATCAAGAACTAACAA
AGAAATCAGAGACATTAACAGGGTCTACAGAGAGGAACTGAAGAGAGATCTGGCC
AAAGACATAACCTCAGACACATCTGGAGATTTTCGGAACGCTTTGCTTTCTCTTG
CTAAGGGTGACCGATCTGAGGACTTTGGTGTGAATGAAGACTTGGCTGATTCAGA
TGCCAGGGCCTTGTATGAAGCAGGAGAAAGGAGAAAGGGGACAGACGTAAACGTG
TTCAATACCATCCTTACCACCAGAAGCTATCCACAACTTCGCAGAGTGTTTCAGA

AATACACCAAGTACAGTAAGCATGACATGAACAAAGTTCTGGACCTGGAGTTGAA
AGGTGACATTGAGAAATGCCTCACAGCTATCGTGAAGTGCGCCACAAGCAAACCA
GCTTTCTTTGCAGAGAAGCTTCATCAAGCCATGAAAGGTGTTGGAACTCGCCATA
AGGCATTGATCAGGATTATGGTTTCCCGTTCTGAAATTGACATGAATGATATCAA
AGCATTCTATCAGAAGATGTATGGTATCTCCCTTTGCCAAGCCATCCTGGATGAA
ACCAAAGGAGATTATGAGAAAATCCTGGTGGCTCTTTGTGGAGGAAACTAA.
```

3. A unicellular host transformed with at least one recombinant DNA molecule selected from the group consisting of:

   (a) the cDNA insert of pNLipl (ATCC Deposit No. 68811) bounded at both ends by EcoRI restriction sites,

   (b) DNA sequences that hybridize to the DNA Insert of claim 3(a) and which encode a human polypeptide having phospholipase $A_2$ inhibitory activity and an immunological activity of the human lipocortin encoded by the cDNA insert of part (a) above, and

(c) DNA sequences that are degenerate as a result of the genetic code as to the DNA insert and sequences of (a) and (b) above,

wherein said DNA sequence is operatively linked to an expression control sequence in the molecule.

4. A unicellular host transformed with at least one recombinant DNA molecule characterized by the sequence:

GAATTCAAGAGAAAGTACGGCAAGTCCCTGTACTATTATATCCAGCAAGACACTA
AGGGCGACTACCAGAAAGCGCTGCTGTACCTGTGTGGTGGAGATGACTGA,

wherein said DNA sequence is operatively linked to an expression control sequence in the molecule.

5. The transformed host according to any of claims 1 to 5, wherein the recombinant DNA molecule is selected from the group consisting of pLipPLT4A, pLipPLT4T and pSVL9109.

6. A unicellular host transformed with at least one recombinant DNA molecule pBg120 (ATCC Deposit No. 74106),

wherein said DNA sequence is operatively linked to an expression control sequence in the molecule.

7. A unicellular host transformed with at least one recombinant DNA molecule pNLip1 (ATCC Deposit No. 68811),

wherein said DNA sequence is operatively linked to an expression control sequence in the molecule.

8. A unicellular host transformed with at least one recombinant DNA molecule consisting of:

```
ATGGCAATGGTATCAGAATTCCTCAAGCAGGCCTGGTTTATTGAAAATGAAGAGC
AGGAATATGTTCAAACTGTGAAGTCATCCAAAGGTGGTCCCGGATCAGCGGTGAG
CCCCTATCCTACCTTCAATCCATCCTCGGATGTCGCTGCCTTGCATAAGGCCATA
ATGGTTAAAGGTGTGGATGAAGCAACCATCATTGACATTCTAACTAAGCGAAACA
ATGCACAGCGTCAACAGATCAAAGCAGCATATCTCCAGGAAACAGGAAAGCCCCT
GGATGAAACACTTAAGAAAGCCCTTACAGGTCACCTTGAGGAGGTTGTTTTAGCT
CTGCTAAAAACTCCAGCGCAATTTGATGCTGATGAACTTCGTGCTGCCATGAAGG
GCCTTGGAACTGATGAAGATACTCTAATTGAGATTTTGGCATCAAGAACTAACAA
AGAAATCAGAGACATTAACAGGGTCTACAGAGAGGAACTGAAGAGAGATCTGGCC
AAAGACATAACCTCAGACACATCTGGAGATTTTCGGAACGCTTTGCTTTCTCTTG
CTAAGGGTGACCGATCTGAGGACTTTGGTGTGAATGAAGACTTGGCTGATTCAGA
TGCCAGGGCCTTGTATGAAGCAGGAGAAAGGAGAAAGGGGACAGACGTAAACGTG
TTCAATACCATCCTTACCACCAGAAGCTATCCACAACTTCGCAGAGTGTTTCAGA
AATACACCAAGTACAGTAAGCATGACATGAACAAAGTTCTGGACCTGGAGTTGAA
AGGTGACATTGAGAAATGCCTCACAGCTATCGTGAAGTGCGCCACAAGCAAACCA
GCTTTCTTTGCAGAGAAGCTTCATCAAGCCATGAAAGGTGTTGGAACTCGCCATA
AGGCATTGATCAGGATTATGGTTTCCCGTTCTGAAATTGACATGAATGATATCAA
AGCATTCTATCAGAAGATGTATGGTATCTCCCTTTGCCAAGCCATCCTGGATGAA
ACCAAAGGAGATTATGAGAAAATCCTGGTGGCTCTTTGTGGAGGAAACTAA,
```

wherein said DNA sequence is operatively linked to an expression control sequence in the molecule.

9. A unicellular host transformed with at least one recombinant DNA molecule consisting of:

```
GAATTCAAGAGAAAGTACGGCAAGTCCCTGTACTATTATATCCAGCAAGACACTA
AGGGCGACTACCAGAAAGCGCTGCTGTACCTGTGTGGTGGAGATGACTGA,
```

wherein said DNA sequence is operatively Linked to an expression control sequence in the molecule.

10. The transformed host according to any of claims 1-9, wherein said expression control sequence is selected from the group consisting of the lac system, the $\beta$-lactamase system, the trp system, the tac system, the trc system, major operator and promoter regions of Phage $\lambda$ , the control region of fd coat protein, the promoter for 3-phosphoglycerate kinase or other glycolytic enzymes, the promoters of acid phosphates, the promoters of the yeast $\alpha$-mating factors, promoters for mammalian cells, the SV40 early and late promoters, adenovirus late promoter and metallothionine promoter, and other sequences which control the expression of genes of prokaryotic or eukaryotic sells and their viruses and combinations thereof.

11. A method for producing a human polypeptide having phospholipase $A_2$ Inhibitory activity and the immunological activity of a human lipocortin comprising the steps of culturing a transformed host according to claims 1 to 10 and collecting said polypeptide.

12. The method according to claim 11, for producing human polypeptide having phospholipase A₂ inhibitory activity and the immunological activity of a human lipocortin, the polypeptide being selected from the group consisting of:

(a)

```
MetAlaMetValSerGluPheLeuLysGlnAlaTrpPheIleGluAsnGluGlu
GlnGluTyrValGlnThrValLysSerSerLysGlyGlyProGlySerAlaVal
SerProTyrProThrPheAsnProSerSerAspValAlaAlaLeuHisLysAla
IleMetValLysGlyValAspGluAlaThrIleIleAspIleLeuThrLysArg
AsnAsnAlaGlnArgGlnGlnIleLysAlaAlaTyrLeuGlnGluThrGlyLys
ProLeuAspGluThrLeuLysLysAlaLeuThrGlyHisLeuGluGluValVal
LeuAlaLeuLeuLysThrProAlaGlnPheAspAlaAspGluLeuArgAlaAla
MetLysGlyLeuGlyThrAspGluAspThrLeuIleGluIleLeuAlaSerArg
ThrAsnLysGluIleArgAspIleAsnArgValTyrArgGluGluLeuLysArg
AspLeuAlaLysAspIleThrSerAspThrSerGlyAspPheArgAsnAlaLeu
LeuSerLeuAlaLysGlyAspArgSerGluAspPheGlyValAsnGluAspLeu
```

```
AlaAspSerAspAlaArgAlaLeuTyrGluAlaGlyGluArgArgLysGlyThr
AspValAsnValPheAsnThrIleLeuThrThrArgSerTyrProGlnLeuArg
ArgValPheGlnLysTyrThrLysTyrSerLysHisAspMetAsnLysValLeu
AspLeuGluLeuLysGlyAspIleGluLysCysLeuThrAlaIleValLysCys
AlaThrSerLysProAlaPhePheAlaGluLysLeuHisGlnAlaMetLysGly
ValGlyThrArgHisLysAlaLeuIleArgIleMetValSerArgSerGluIle
AspMetAsnAspIleLysAlaPheTyrGlnLysMetTyrGlyIleSerLeuCys
GlnAlaIleLeuAspGluThrLysGlyAspTyrGluLysIleLeuValAlaLeu
CysGlyGlyAsn;
```

(b)

```
AlaMetValSerGluPheLeuLysGlnAlaTrpPheIleGluAsnGluGluGln
GluTyrValGlnThrValLysSerSerLysGlyGlyProGlySerAlaValSer
ProTyrProThrPheAsnProSerSerAspValAlaAlaLeuHisLysAlaIle
MetValLysGlyValAspGluAlaThrIleIleAspIleLeuThrLysArgAsn
AsnAlaGlnArgGlnGlnIleLysAlaAlaTyrLeuGlnGluThrGlyLysPro
LeuAspGluThrLeuLysLysAlaLeuThrGlyHisLeuGluGluValValLeu
AlaLeuLeuLysThrProAlaGlnPheAspAlaAspGluLeuArgAlaAlaMet
LysGlyLeuGlyThrAspGluAspThrLeuIleGluIleLeuAlaSerArgThr
AsnLysGluIleArgAspIleAsnArgValTyrArgGluGluLeuLysArgAsp
LeuAlaLysAspIleThrSerAspThrSerGlyAspPheArgAsnAlaLeuLeu
SerLeuAlaLysGlyAspArgSerGluAspPheGlyValAsnGluAspLeuAla
AspSerAspAlaArgAlaLeuTyrGluAlaGlyGluArgArgLysGlyThrAsp
ValAsnValPheAsnThrIleLeuThrThrArgSerTyrProGlnLeuArgArg
ValPheGlnLysTyrThrLysTyrSerLysHisAspMetAsnLysValLeuAsp
LeuGluLeuLysGlyAspIleGluLysCysLeuThrAlaIleValLysCysAla
ThrSerLysProAlaPhePheAlaGluLysLeuHisGlnAlaMetLysGlyVal
GlyThrArgHisLysAlaLeuIleArgIleMetValSerArgSerGluIleAsp
MetAsnAspIleLysAlaPheTyrGlnLysMetTyrGlyIleSerLeuCysGln
AlaIleLeuAspGluThrLysGlyAspTyrGluLysIleLeuValAlaLeuCys
GlyGlyAsn;
```

and
(c) polypeptide fragments of the polypeptide of part (a) or (b) above displaying a biological or immunological activity of the lipocortin defined in part (a) or (b) above.

13. The method according to claim 11, for producing a N-lipocortin polypeptide characterized by the following properties:
    (a) having the ability to inhibit the enzyme phospholipase $A_2$,
    (b) comprising the tryptic fragments:
        (1) LeuTyrAspSerMetLys;
        (2) LeuSerLeuAsnGlyAspThrSer ThrProProSerAlaTyrGly;
        (3) SerLeuTyrTyrTyrIleGln GlnAspThrLys;
        (4) TrpIleSerIleMetThrGluArg;
        (5) SerTyrSerProTyrAspMet LeuGluSerIle;
        (6) LeuLeuValValTyrProX ThrGlnIleLeu;
    wherein x is any amino acid, and
    (c) having a carboxy-terminus consisting of the amino acid sequence,

```
LysArgLysTyrGlyLysSerLeuTyrTyrTyr-
IleGlnGlnAspThrLysGly AspTyrGlnLysAlaLeuLeuTyrLeuCysGly-
GlyAspAsp.
```

**14.** The method according to claim 11, for producing a human lipocortin-like polypeptide encoded by the recombinant DNA molecules of claim 1 or 2.

**15.** The method according to claim 11, for producing a polypeptide wherein the polypeptide is selected from the group consisting of:

(a)

```
GlyGlyProGlySerAlaValSerProTyrProThrPheAsnProSerSerAsp
ValAlaAlaLeuHisLysAlaIleMetValLysGlyValAspGluAlaThrIle
IleAspIleLeuThrLysArgAsnAsnAlaGlnArgGlnGlnIleLysAlaAla
TyrLeuGlnGluThrGlyLysProLeuAspGluThrLeuLysLysAlaLeuThr
GlyHisLeuGluGluValValLeuAlaLeuLeuLysThrProAlaGlnPheAsp
AlaAspGluLeuArgAlaAlaMetLysGlyLeuGlyThrAspGluAspThrLeu
IleGluIleLeuAlaSerArgThrAsnLysGluIleArgAspIleAsnArgVal
TyrArgGluGluLeuLysArgAspLeuAlaLysAspIleThrSerAspThrSer
GlyAspPheArgAsnAlaLeuLeuSerLeuAlaLysGlyAspArgSerGluAsp
PheGlyValAsnGluAspLeuAlaAspSerAspAlaArgAlaLeuTyrGluAla
GlyGluArgArgLysGlyThrAspValAsnValPheAsnThrIleLeuThrThr
```

```
ArgSerTyrProGlnLeuArgArgValPheGlnLysTyrThrLysTyrSerLys
HisAspMetAsnLysValLeuAspLeuGluLeuLysGlyAspIleGluLysCys
LeuThrAlaIleValLysCysAlaThrSerLysProAlaPhePheAlaGluLys
LeuHisGlnAlaMetLysGlyValGlyThrArgHisLysAlaLeuIleArgIle
MetValSerArgSerGluIleAspMetAsnAspIleLysAlaPheTyrGlnLys
MetTyrGlyIleSerLeuCysGlnAlaIleLeuAspGluThrLysGlyAspTyr
GluLysIleLeuValAlaLeuCysGlyGly;
```

(b)

```
GlyGlyProGlySerAlaValSerProTyrProThrPheAsnProSerSerAsp
ValAlaAlaLeuHisLysAlaIleMetValLysGlyValAspGluAlaThrIle
IleAspIleLeuThrLysArgAsnAsnAlaGlnArgGlnGlnIleLysAlaAla
TyrLeuGlnGluThrGlyLysProLeuAspGluThrLeuLysLysAlaLeuThr
GlyHisLeuGluGluValValLeuAlaLeuLeuLysThrProAlaGlnPheAsp
AlaAspGluLeuArgAlaAlaMetLysGlyLeuGlyThrAspGluAspThrLeu
IleGluIleLeuAlaSerArgThrAsnLysGluIleArgAspIleAsnArgVal
TyrArgGluGluLeuLysArgAspLeuAlaLysAspIleThrSerAspThrSer
GlyAspPheArgAsnAlaLeuLeuSerLeuAlaLysGlyAspArgSerGluAsp
PheGlyValAsnGluAspLeuAlaAspSerAspAlaArgAlaLeuTyrGluAla
GlyGluArgArgLysGlyThrAspValAsnValPheAsnThrIleLeuThrThr;
```

(c)

GlyGlyProGlySerAlaValSerProTyrProThrPheAsnProSerSerAsp
ValAlaAlaLeuHisLysAlaIleMetValLysGlyValAspGluAlaThrIle
IleAspIleLeuThrLysArgAsnAsnAlaGlnArgGlnGlnIleLysAlaAla
TyrLeuGlnGluThrGlyLysProLeuAspGluThrLeuLysLysAlaLeuThr
GlyHisLeuGluGluValValLeuAlaLeuLeuLysThrProAlaGlnPheAsp
AlaAspGluLeuArgAlaAlaMetLysGlyLeuGlyThrAspGluAspThrLeu
IleGluIleLeuAlaSerArgThrAsnLysGluIleArgAspIleAsnArgVal
TyrArgGluGluLeuLysArgAspLeuAlaLysAspIleThrSerAspThrSer
GlyAspPheArgAsnAlaLeuLeuSerLeuAlaLysGlyAspArgSerGluAsp
PheGlyValAsnGluAspLeuAlaAspSerAspAlaArgAlaLeuTyrGluAla
GlyGlu;

(d)

ValSerGluPheLeuLysGlnAlaTrpPheIleGluAsnGluGluGlnGluTyr
ValGlnThrValLysSerSerLysGlyGlyProGlySerAlaValSerProTyr
ProThrPheAsnProSerSerAspValAlaAlaLeuHisLysAlaIleLeuVal

LysGlyValAspGluAlaThrIleIleAspIleLeuThrLysArgAsnAsnAla
GlnArgGlnGlnIleLysAlaAlaTyrLeuGlnGluThrGlyLysProLeuAsp
GluThrLeuLysLysAlaLeuThrGlyHisLeuGluGluValValLeuAlaLeu
LeuLysThrProAlaGlnPheAspAlaAspGluLeuArgAlaAlaLeuLysGly
LeuGlyThrAspGluAspThrLeuIleGluIleLeuAlaSerArgThrAsnLys
GluIleArgAspIleAsnArgValTyrArgGluGluLeuLysArgAspLeuAla
LysAspIleThrSerAspThrSerGlyAspPheArgAsnAlaLeuLeuSerLeu
AlaLysGlyAspArgSerGluAspPheGlyValAsnGluAspLeuAlaAspSer
AspAlaArgAlaLeuTyrGluAlaGlyGluArgArgLysGlyThrAspValAsn
ValPheAsnThrIleLeuThrThrArgSerTyrProGlnLeuArgArgValPhe
GlnLysTyrThrLysTyrSerLysHisAsp;

(e)

ValSerGluPheLeuLysGlnAlaTrpPheIleGluAsnGluGluGlnGluTyr
ValGlnThrValLysSerSerLysGlyGlyProGlySerAlaValSerProTyr
ProThrPheAsnProSerSerAspValAlaAlaLeuHisLysAlaIleLeuVal
LysGlyValAspGluAlaThrIleIleAspIleLeuThrLysArgAsnAsnAla
GlnArgGlnGlnIleLysAlaAlaTyrLeuGlnGluThrGlyLysProLeuAsp
GluThrLeuLysLysAlaLeuThrGlyHisLeuGluGluValVal;

44

and

(f)

```
ValSerGluPheLeuLysGlnAlaTrpPheIleGluAsnGluGluGlnGluTyr
ValGlnThrValLysSerSerLysGlyGlyProGlySerAlaValSerProTyr
ProThrPheAsnProSerSerAspValAlaAlaLeuHisLysAlaIleLeuVal
LysGlyValAspGluAlaThrIleIleAspIleLeuThrLysArgAsnAsnAla
GlnArgGlnGlnIleLysAlaAlaTyrLeuGlnGluThrGlyLysProLeuAsp
GluThrLeuLysLysAlaLeuThrGlyHisLeuGluGluValValLeuAlaLeu
LeuLysThrProAlaGlnPheAspAlaAspGluLeuArgAlaAlaLeuLysGly
LeuGlyThrAspGluAspThrLeuIleGluIleLeuAlaSerArgThrAsnLys
GluIleArgAspIleAsnArgValTyrArgGluGluLeuLysArgAspLeuAla
LysAspIleThrSerAspThrSerGlyAspPheArgAsnAlaLeuLeuSerLeu
AlaLysGlyAspArgSerGluAspPheGlyValAsnGluAsp.
```

16. The method according to claim 11, for producing a polypeptide wherein the polypeptide has the amino acid sequence:

```
GlyGlyProGlySerAlaValSerProTyrProThrPheAsnProSerSerAsp
ValAlaAlaLeuHisLysAlaIleMetValLysGlyValAspGluAlaThrIle
```

```
IleAspIleLeuThrLysArgAsnAsnAlaGlnArgGlnGlnIleLysAlaAla
TyrLeuGlnGluThrGlyLysProLeuAspGluThrLeuLysLysAlaLeuThr
GlyHisLeuGluGluValValLeuAlaLeuLeuLysThrProAlaGlnPheAsp
AlaAspGluLeuArgAlaAlaMetLysGlyLeuGlyThrAspGluAspThrLeu
IleGluIleLeuAlaSerArgThrAsnLysGluIleArgAspIleAsnArgVal
TyrArgGluGluLeuLysArgAspLeuAlaLysAspIleThrSerAspThrSer
GlyAspPheArgAsnAlaLeuLeuSerLeuAlaLysGlyAspArgSerGluAsp
PheGlyValAsnGluAspLeuAlaAspSerAspAlaArgAlaLeuTyrGluAla
GlyGluArgArgLysGlyThrAspValAsnValPheAsnThrIleLeuThrThr
ArgSerTyrProGlnLeuArgArgValPheGlnLysTyrThrLysTyrSerLys
HisAspMetAsnLysValLeuAspLeuGluLeuLysGlyAspIleGluLysCys
LeuThrAlaIleValLysCysAlaThrSerLysProAlaPhePheAlaGluLys
LeuHisGlnAlaMetLysGlyValGlyThrArgHisLysAlaLeuIleArgIle
MetValSerArgSerGluIleAspMetAsnAspIleLysAlaPheTyrGlnLys
MetTyrGlyIleSerLeuCysGlnAlaIleLeuAspGluThrLysGlyAspTyr
GluLysIleLeuValAlaLeuCysGlyGlyAsn.
```

**17.** The transformed host according to any of claims 1 to 10, selected from the group consisting of strains of E. coli. Pseudomonas, Bacillus, Streptomyces, yeast cells, other fungus, mouse calls or other animal cell hosts, plant cell hosts, and human tissue cells.

**Patentansprüche**

**Patentansprüche für folgende Vertragsstaaten : BE, CH, DE, FR, GB, IT, LI, LU, NL, SE**

**1.** Rekombinantes DNA-Molekül, gekennzeichnet durch eine DNA-Sequenz, die ein menschliches Polypeptid mit Hemmwirkung auf Phospholipase $A_2$ und der immunologischen Aktivität eines menschlichen Lipocortins codiert und ausgewählt ist aus der Gruppe, bestehend aus

(a) der cDNA-Insertion von λ LC,

(b) DNA-Sequenzen, die mit der DNA-Insertion von Anspruch 1 (a) hybridisieren und ein menschliches Polypeptid mit Hemmwirkung auf Phospholipase $A_2$ und einer immunologischen Aktivität des von der cDNA-Insertion von Teil (a), oben, codierten menschlichen Lipocortins codieren, und

(c) DNA-Sequenzen, die als Ergebnis des genetischen Codes gegenüber der DNA-Insertion und den Sequenzen von (a) und (b), oben, degeneriert sind.

**2.** Rekombinantes DNA-Molekül nach Anspruch 1, wobei die DNA-Sequenz besteht aus:

```
ATGGCAATGGTATCAGAATTCCTCAAGCAGGCCTGGTTTATTGAAAATGAAGAGC
AGGAATATGTTCAAACTGTGAAGTCATCCAAAGGTGGTCCCGGATCAGCGGTGAG
CCCCTATCCTACCTTCAATCCATCCTCGGATGTCGCTGCCTTGCATAAGGCCATA
ATGGTTAAAGGTGTGGATGAAGCAACCATCATTGACATTCTAACTAAGCGAAACA
ATGCACAGCGTCAACAGATCAAAGCAGCATATCTCCAGGAAACAGGAAAGCCCCT
GGATGAAACACTTAAGAAAGCCCTTACAGGTCACCTTGAGGAGGTTGTTTTAGCT
CTGCTAAAAACTCCAGCGCAATTTGATGCTGATGAACTTCGTGCTGCCATGAAGG
GCCTTGGAACTGATGAAGATACTCTAATTGAGATTTTGGCATCAAGAACTAACAA
AGAAATCAGAGACATTAACAGGGTCTACAGAGAGGAACTGAAGAGAGATCTGGCC
AAAGACATAACCTCAGACACATCTGGAGATTTTCGGAACGCTTTGCTTTCTCTTG
CTAAGGGTGACCGATCTGAGGACTTTGGTGTGAATGAAGACTTGGCTGATTCAGA
TGCCAGGGCCTTGTATGAAGCAGGAGAAAGGAGAAAGGGGACAGACGTAAACGTG
TTCAATACCATCCTTACCACCAGAAGCTATCCACAACTTCGCAGAGTGTTTCAGA
AATACACCAAGTACAGTAAGCATGACATGAACAAAGTTCTGGACCTGGAGTTGAA
```

```
AGGTGACATTGAGAAATGCCTCACAGCTATCGTGAAGTGCGCCACAAGCAAACCA
GCTTTCTTTGCAGAGAAGCTTCATCAAGCCATGAAAGGTGTTGGAACTCGCCATA
AGGCATTGATCAGGATTATGGTTTCCCGTTCTGAAATTGACATGAATGATATCAA
AGCATTCTATCAGAAGATGTATGGTATCTCCCTTTGCCAAGCCATCCTGGATGAA
ACCAAAGGAGATTATGAGAAATCCTGGTGGCTCTTTGTGGAGGAAACTAA.
```

**3.** Rekombinantes DNA-Molekül, das ausgewählt ist aus der Gruppe bestehend aus:

(a) der cDNA-Insertion von pNLip1 (ATCC-Hinterlegung Nr. 68811), die an beiden Enden von EcoRI-Restriktionsstellen begrenzt ist,

(b) DNA-Sequenzen, die mit der DNA-Insertion nach Anspruch 3 (a) hybridisieren und ein menschliches Polypeptid codieren, das eine Hemmwirkung auf die Phospholipase $A_2$ und eine immunologische Aktivität des menschlichen Lipocortins aufweist, das von der vorstehenden cDNA-Insertion von Teil (a) codiert wird, und

(c) DNA-Sequenzen, die als Ergebnis des genetischen Codes gegenüber der vorstehenden DNA-Insertion und den Sequenzen von (a) und (b), oben, degeneriert sind.

4. Rekombinantes DNA-Molekül, gekennzeichnet durch die Sequenz:

**GAATTCAAGAGAAAGTACGGCAAGTCCTGTACTATTATATCCAGCAAGACACTA AGGGCGACTACCAGAAAGCGCTGCTGTACCTGTGTGGTGGAGATGACTGA.**

5. Rekombinantes DNA-Molekül nach Anspruch 1 oder 2, wobei die DNA-Sequenz funktionell an eine Expressionskontrollsequenz im Molekül gebunden ist.

6. Rekombinantes DNA-Molekül nach Anspruch 5, wobei die Expressionskontrollsequenz ausgewählt ist aus der Gruppe, bestehend aus dem lac-System, $\beta$-Lactamase-System, trp-System, tac-System, trc-System, den wichtigsten Operator- und Promotorregionen des Phagen λ, der Kontrollregion des fd-Hüllproteins, dem Promotor der 3-Phosphoglycerat-Kinase oder anderen glycolytischen Enzymen, den Promotoren der sauren Phosphatase, den Promotoren der $\alpha$-Paarungssfaktoren der Hefe, Promotoren von Säugerzellen, den frühen und späten Promotoren von SV40, dem späten Promotor von Adenovirus und dem Metallothionin-Promotor und weiteren Sequenzen, die die Expression von Genen von prokaryotischen oder eukaryotischen Zellen und ihrer Viren und deren Kombinationen kontrollieren.

7. Rekombinantes DNA-Molekül nach Anspruch 5, das ausgewählt ist aus der Gruppe, die aus pLipPLT4A, pLipPLT4T und pSVL9109 besteht.

8. Rekombinantes DNA-Molekül pBg120 (ATCC-Hinterlegung Nr. 74106).

9. Rekombinantes DNA-Molekül pNLip1 (ATCC-Hinterlegung Nr. 68811).

10. Einzelliger Wirt, der mit mindestens einem rekombinanten DNA-Molekül nach Anspruch 5 transformiert ist.

11. Transformierter Wirt nach Anspruch 10, der ausgewählt ist aus der Gruppe bestehend aus den Stämmen E. coli, Pseudomonas, Bacillus und Streptomyces, und aus Hefezellen oder anderen Pilzen, Mauszellen oder anderen Zellen von tierischen oder pflanzlichen Wirten und Zellen von menschlichem Gewebe.

12. Menschliches Polypeptid, das eine Hemmwirkung auf die Phospholipase $A_2$ und die immunologische Aktivität eines menschlichen Lipocortins aufweist, wobei das Polypeptid ausgewählt ist aus der Gruppe bestehend aus

(a)

MetAlaMetValSerGluPheLeuLysGlnAlaTrpPheIleGluAsnGluGlu
GlnGluTyrValGlnThrValLysSerSerLysGlyGlyProGlySerAlaVal
SerProTyrProThrPheAsnProSerSerAspValAlaAlaLeuHisLysAla
IleMetValLysGlyValAspGluAlaThrIleIleAspIleLeuThrLysArg
AsnAsnAlaGlnArgGlnGlnIleLysAlaAlaTyrLeuGlnGluThrGlyLys
ProLeuAspGluThrLeuLysLysAlaLeuThrGlyHisLeuGluGluValVal
LeuAlaLeuLeuLysThrProAlaGlnPheAspAlaAspGluLeuArgAlaAla
MetLysGlyLeuGlyThrAspGluAspThrLeuIleGluIleLeuAlaSerArg
ThrAsnLysGluIleArgAspIleAsnArgValTyrArgGluGluLeuLysArg
AspLeuAlaLysAspIleThrSerAspThrSerGlyAspPheArgAsnAlaLeu
LeuSerLeuAlaLysGlyAspArgSerGluAspPheGlyValAsnGluAspLeu
AlaAspSerAspAlaArgAlaLeuTyrGluAlaGlyGluArgArgLysGlyThr
AspValAsnValPheAsnThrIleLeuThrThrArgSerTyrProGlnLeuArg


ArgValPheGlnLysTyrThrLysTyrSerLysHisAspMetAsnLysValLeu
AspLeuGluLeuLysGlyAspIleGluLysCysLeuThrAlaIleValLysCys
AlaThrSerLysProAlaPhePheAlaGluLysLeuHisGlnAlaMetLysGly
ValGlyThrArgHisLysAlaLeuIleArgIleMetValSerArgSerGluIle
AspMetAsnAspIleLysAlaPheTyrGlnLysMetTyrGlyIleSerLeuCys
GlnAlaIleLeuAspGluThrLysGlyAspTyrGluLysIleLeuValAlaLeu
CysGlyGlyAsn

(b)

```
AlaMetValSerGluPheLeuLysGlnAlaTrpPheIleGluAsnGluGluGln
GluTyrValGlnThrValLysSerSerLysGlyGlyProGlySerAlaValSer
ProTyrProThrPheAsnProSerSerAspValAlaAlaLeuHisLysAlaIle
MetValLysGlyValAspGluAlaThrIleIleAspIleLeuThrLysArgAsn
AsnAlaGlnArgGlnGlnIleLysAlaAlaTyrLeuGlnGluThrGlyLysPro
LeuAspGluThrLeuLysLysAlaLeuThrGlyHisLeuGluGluValValLeu
AlaLeuLeuLysThrProAlaGlnPheAspAlaAspGluLeuArgAlaAlaMet
LysGlyLeuGlyThrAspGluAspThrLeuIleGluIleLeuAlaSerArgThr
AsnLysGluIleArgAspIleAsnArgValTyrArgGluGluLeuLysArgAsp
LeuAlaLysAspIleThrSerAspThrSerGlyAspPheArgAsnAlaLeuLeu
SerLeuAlaLysGlyAspArgSerGluAspPheGlyValAsnGluAspLeuAla
AspSerAspAlaArgAlaLeuTyrGluAlaGlyGluArgArgLysGlyThrAsp
ValAsnValPheAsnThrIleLeuThrThrArgSerTyrProGlnLeuArgArg
ValPheGlnLysTyrThrLysTyrSerLysHisAspMetAsnLysValLeuAsp
LeuGluLeuLysGlyAspIleGluLysCysLeuThrAlaIleValLysCysAla
ThrSerLysProAlaPhePheAlaGluLysLeuHisGlnAlaMetLysGlyVal
GlyThrArgHisLysAlaLeuIleArgIleMetValSerArgSerGluIleAsp
MetAsnAspIleLysAlaPheTyrGlnLysMetTyrGlyIleSerLeuCysGln
AlaIleLeuAspGluThrLysGlyAspTyrGluLysIleLeuValAlaLeuCys
GlyGlyAsn
```

und

(c) Polypeptidfragmenten des Polypeptids des vorstehenden Teils (a) oder (b), das eine biologische oder immunologische Aktivität des im vorstehenden Teil (a) oder (b) definierten Lipocortins zeigt.

**13.** N-Lipocortin-Polypeptid, das durch die nachstehenden Eigenschaften gekennzeichnet ist:
   (a) es hat die Fähigkeit zur Hemmung des Enzyms Phospholipase $A_2$,
   (b) es enthält die Trypsin-Fragmente:
      (1) LeuTyrAspSerMetLys;
      (2) LeuSerLeuAsnGlyAspThrSer ThrProProSerAlaTyrGly;
      (3) SerLeuTyrTyrTyrIleGln GlnAspThrLys;
      (4) TrpIleSerIleMetThrGluArg;
      (5) SerTyrSerProTyrAspMet LeuGluSerIle;
      (6) LeuLeuValValTyrProX ThrGlnIleLeu;
         wobei X eine beliebige Aminosäure ist, und
   (c) es weist einen Carboxy-Terminus auf, der aus der Aminosäuresequenz

```
LysArgLysTyrGlyLysSerLeuTyrTyrTyrIleGlnGlnAspThrLysGly
AspTyrGlnLysAlaLeuLeuTyrLeuCysGlyGlyAspAsp.
```

besteht.

**14.** Menschliches Lipocortin-ähnliches Polypeptid, das von den rekombinanten DNA-Molekülen nach Anspruch 1 oder 2 codiert wird.

**15.** Polypeptid nach Anspruch 12, wobei das Polypeptid ausgewählt ist aus der Gruppe bestehend aus

(a)

```
GlyGlyProGlySerAlaValSerProTyrProThrPheAsnProSerSerAsp
ValAlaAlaLeuHisLysAlaIleMetValLysGlyValAspGluAlaThrIle
IleAspIleLeuThrLysArgAsnAsnAlaGlnArgGlnGlnIleLysAlaAla
TyrLeuGlnGluThrGlyLysProLeuAspGluThrLeuLysLysAlaLeuThr
GlyHisLeuGluGluValValLeuAlaLeuLeuLysThrProAlaGlnPheAsp
AlaAspGluLeuArgAlaAlaMetLysGlyLeuGlyThrAspGluAspThrLeu
IleGluIleLeuAlaSerArgThrAsnLysGluIleArgAspIleAsnArgVal
TyrArgGluGluLeuLysArgAspLeuAlaLysAspIleThrSerAspThrSer
GlyAspPheArgAsnAlaLeuLeuSerLeuAlaLysGlyAspArgSerGluAsp
PheGlyValAsnGluAspLeuAlaAspSerAspAlaArgAlaLeuTyrGluAla
GlyGluArgArgLysGlyThrAspValAsnValPheAsnThrIleLeuThrThr
ArgSerTyrProGlnLeuArgArgValPheGlnLysTyrThrLysTyrSerLys
HisAspMetAsnLysValLeuAspLeuGluLeuLysGlyAspIleGluLysCys
LeuThrAlaIleValLysCysAlaThrSerLysProAlaPhePheAlaGluLys
LeuHisGlnAlaMetLysGlyValGlyThrArgHisLysAlaLeuIleArgIle
MetValSerArgSerGluIleAspMetAsnAspIleLysAlaPheTyrGlnLys
MetTyrGlyIleSerLeuCysGlnAlaIleLeuAspGluThrLysGlyAspTyr
GluLysIleLeuValAlaLeuCysGlyGly
```

(b)

```
GlyGlyProGlySerAlaValSerProTyrProThrPheAsnProSerSerAsp
ValAlaAlaLeuHisLysAlaIleMetValLysGlyValAspGluAlaThrIle
IleAspIleLeuThrLysArgAsnAsnAlaGlnArgGlnGlnIleLysAlaAla
TyrLeuGlnGluThrGlyLysProLeuAspGluThrLeuLysLysAlaLeuThr
GlyHisLeuGluGluValValLeuAlaLeuLeuLysThrProAlaGlnPheAsp
AlaAspGluLeuArgAlaAlaMetLysGlyLeuGlyThrAspGluAspThrLeu
IleGluIleLeuAlaSerArgThrAsnLysGluIleArgAspIleAsnArgVal
TyrArgGluGluLeuLysArgAspLeuAlaLysAspIleThrSerAspThrSer
GlyAspPheArgAsnAlaLeuLeuSerLeuAlaLysGlyAspArgSerGluAsp
PheGlyValAsnGluAspLeuAlaAspSerAspAlaArgAlaLeuTyrGluAla
GlyGluArgArgLysGlyThrAspValAsnValPheAsnThrIleLeuThrThr;
```

50

(c)

GlyGlyProGlySerAlaValSerProTyrProThrPheAsnProSerSerAsp
ValAlaAlaLeuHisLysAlaIleMetValLysGlyValAspGluAlaThrIle
IleAspIleLeuThrLysArgAsnAsnAlaGlnArgGlnGlnIleLysAlaAla
TyrLeuGlnGluThrGlyLysProLeuAspGluThrLeuLysLysAlaLeuThr
GlyHisLeuGluGluValValLeuAlaLeuLeuLysThrProAlaGlnPheAsp
AlaAspGluLeuArgAlaAlaMetLysGlyLeuGlyThrAspGluAspThrLeu
IleGluIleLeuAlaSerArgThrAsnLysGluIleArgAspIleAsnArgVal
TyrArgGluGluLeuLysArgAspLeuAlaLysAspIleThrSerAspThrSer
GlyAspPheArgAsnAlaLeuLeuSerLeuAlaLysGlyAspArgSerGluAsp
PheGlyValAsnGluAspLeuAlaAspSerAspAlaArgAlaLeuTyrGluAla
GlyGlu

(d)

ValSerGluPheLeuLysGlnAlaTrpPheIleGluAsnGluGluGlnGluTyr
ValGlnThrValLysSerSerLysGlyGlyProGlySerAlaValSerProTyr
ProThrPheAsnProSerSerAspValAlaAlaLeuHisLysAlaIleLeuVal
LysGlyValAspGluAlaThrIleIleAspIleLeuThrLysArgAsnAsnAla
GlnArgGlnGlnIleLysAlaAlaTyrLeuGlnGluThrGlyLysProLeuAsp
GluThrLeuLysLysAlaLeuThrGlyHisLeuGluGluValValLeuAlaLeu
LeuLysThrProAlaGlnPheAspAlaAspGluLeuArgAlaAlaLeuLysGly
LeuGlyThrAspGluAspThrLeuIleGluIleLeuAlaSerArgThrAsnLys
GluIleArgAspIleAsnArgValTyrArgGluGluLeuLysArgAspLeuAla
LysAspIleThrSerAspThrSerGlyAspPheArgAsnAlaLeuLeuSerLeu
AlaLysGlyAspArgSerGluAspPheGlyValAsnGluAspLeuAlaAspSer
AspAlaArgAlaLeuTyrGluAlaGlyGluArgArgLysGlyThrAspValAsn
ValPheAsnThrIleLeuThrThrArgSerTyrProGlnLeuArgArgValPhe
GlnLysTyrThrLysTyrSerLysHisAsp

(e)

ValSerGluPheLeuLysGlnAlaTrpPheIleGluAsnGluGluGlnGluTyr
ValGlnThrValLysSerSerLysGlyGlyProGlySerAlaValSerProTyr
ProThrPheAsnProSerSerAspValAlaAlaLeuHisLysAlaIleLeuVal
LysGlyValAspGluAlaThrIleIleAspIleLeuThrLysArgAsnAsnAla
GlnArgGlnGlnIleLysAlaAlaTyrLeuGlnGluThrGlyLysProLeuAsp
GluThrLeuLysLysAlaLeuThrGlyHisLeuGluGluValVal;

EP 0 209 568 B1

(f)

```
ValSerGluPheLeuLysGlnAlaTrpPheIleGluAsnGluGluGlnGluTyr
ValGlnThrValLysSerSerLysGlyGlyProGlySerAlaValSerProTyr
ProThrPheAsnProSerSerAspValAlaAlaLeuHisLysAlaIleLeuVal
LysGlyValAspGluAlaThrIleIleAspIleLeuThrLysArgAsnAsnAla
GlnArgGlnGlnIleLysAlaAlaTyrLeuGlnGluThrGlyLysProLeuAsp
GluThrLeuLysLysAlaLeuThrGlyHisLeuGluGluValValLeuAlaLeu
LeuLysThrProAlaGlnPheAspAlaAspGluLeuArgAlaAlaLeuLysGly
LeuGlyThrAspGluAspThrLeuIleGluIleLeuAlaSerArgThrAsnLys
GluIleArgAspIleAsnArgValTyrArgGluGluLeuLysArgAspLeuAla
LysAspIleThrSerAspThrSerGlyAspPheArgAsnAlaLeuLeuSerLeu
AlaLysGlyAspArgSerGluAspPheGlyValAsnGluAsp
```

16. Polypeptid nach Anspruch 12, wobei das Polypeptid die Aminosäuresequenz

```
GlyGlyProGlySerAlaValSerProTyrProThrPheAsnProSerSerAsp
ValAlaAlaLeuHisLysAlaIleMetValLysGlyValAspGluAlaThrIle
IleAspIleLeuThrLysArgAsnAsnAlaGlnArgGlnGlnIleLysAlaAla
TyrLeuGlnGluThrGlyLysProLeuAspGluThrLeuLysLysAlaLeuThr
GlyHisLeuGluGluValValLeuAlaLeuLeuLysThrProAlaGlnPheAsp
AlaAspGluLeuArgAlaAlaMetLysGlyLeuGlyThrAspGluAspThrLeu
IleGluIleLeuAlaSerArgThrAsnLysGluIleArgAspIleAsnArgVal
TyrArgGluGluLeuLysArgAspLeuAlaLysAspIleThrSerAspThrSer
GlyAspPheArgAsnAlaLeuLeuSerLeuAlaLysGlyAspArgSerGluAsp
PheGlyValAsnGluAspLeuAlaAspSerAspAlaArgAlaLeuTyrGluAla
GlyGluArgArgLysGlyThrAspValAsnValPheAsnThrIleLeuThrThr
ArgSerTyrProGlnLeuArgArgValPheGlnLysTyrThrLysTyrSerLys
HisAspMetAsnLysValLeuAspLeuGluLeuLysGlyAspIleGluLysCys
LeuThrAlaIleValLysCysAlaThrSerLysProAlaPhePheAlaGluLys
LeuHisGlnAlaMetLysGlyValGlyThrArgHisLysAlaLeuIleArgIle
MetValSerArgSerGluIleAspMetAsnAspIleLysAlaPheTyrGlnLys
MetTyrGlyIleSerLeuCysGlnAlaIleLeuAspGluThrLysGlyAspTyr
GluLysIleLeuValAlaLeuCysGlyGlyAsn
```

aufweist.

52

EP 0 209 568 B1

**17.** DNA-Sequenz, bestehend aus

```
ATGGCAATGGTATCAGAATTCCTCAAGCAGGCCTGGTTTATTGAAAATGAAGAGC
AGGAATATGTTCAAACTGTGAAGTCATCCAAAGGTGGTCCCGGATCAGCGGTGAG
CCCCTATCCTACCTTCAATCCATCCTCGGATGTCGCTGCCTTGCATAAGGCCATA
ATGGTTAAAGGTGTGGATGAAGCAACCATCATTGACATTCTAACTAAGCGAAACA
ATGCACAGCGTCAACAGATCAAAGCAGCATATCTCCAGGAAACAGGAAAGCCCCT
GGATGAAACACTTAAGAAAGCCCTTACAGGTCACCTTGAGGAGGTTGTTTTAGCT
CTGCTAAAAACTCCAGCGCAATTTGATGCTGATGAACTTCGTGCTGCCATGAAGG
GCCTTGGAACTGATGAAGATACTCTAATTGAGATTTTGGCATCAAGAACTAACAA
AGAAATCAGAGACATTAACAGGGTCTACAGAGAGGAACTGAAGAGAGATCTGGCC
AAAGACATAACCTCAGACACATCTGGAGATTTTCGGAACGCTTTGCTTTCTCTTG
CTAAGGGTGACCGATCTGAGGACTTTGGTGTGAATGAAGACTTGGCTGATTCAGA
TGCCAGGGCCTTGTATGAAGCAGGAGAAAGGAGAAAGGGGACAGACGTAAACGTG
TTCAATACCATCCTTACCACCAGAAGCTATCCACAACTTCGCAGAGTGTTTCAGA
AATACACCAAGTACAGTAAGCATGACATGAACAAAGTTCTGGACCTGGAGTTGAA
AGGTGACATTGAGAAATGCCTCACAGCTATCGTGAAGTGCGCCACAAGCAAACCA
GCTTTCTTTGCAGAGAAGCTTCATCAAGCCATGAAAGGTGTTGGAACTCGCCATA
AGGCATTGATCAGGATTATGGTTTCCCGTTCTGAAATTGACATGAATGATATCAA
AGCATTCTATCAGAAGATGTATGGTATCTCCCTTTGCCAAGCCATCCTGGATGAA
ACCAAAGGAGATTATGAGAAATCCTGGTGGCTCTTTGTGGAGGAAACTAA.
```

**18.** DNA-Sequenz, bestehend aus

```
GAATTCAAGAGAAAGTACGGCAAGTCCCTGTACTATTATATCCAGCAAGACACTA
AGGGCGACTACCAGAAAGCGCTGCTGTACCTGTGTGGTGGAGATGACTGA.
```

**19.** Verfahren zur Herstellung eines menschlichen Polypeptids, das eine Hemmwirkung auf die Phospholipase $A_2$ und die immunologische Aktivität eines menschlichen Lipocortins aufweist, umfassend die Schritte der Züchtung eines mit einem rekombinanten DNA-Molekül nach Anspruch 5 transformierten Wirts und Gewinnung des Polypeptids.

**20.** Pharmazeutisch verträgliche Zusammensetzung, die zur Behandlung von arthritischen, allergischen, dermatologischen, ophthalmischen und Kollagen-Erkrankungen und anderen Störungen nützlich ist, einschließlich entzündlicher Vorgänge, umfassend eine pharmazeutisch wirksame Menge von mindestens einem Polypeptid, das ausgewählt ist aus der Gruppe bestehend aus Polypeptiden nach Anspruch 12, 14, 15 oder 16.

**21.** Pharmazeutisch verträgliche Zusammensetzung, die zur Behandlung von arthritischen, allergischen, dermatologischen, ophthalmischen und Kollagen-Erkrankungen und anderen Störungen nützlich ist,

53

einschließlich entzündlicher Vorgänge, umfassend eine pharmazeutisch wirksame Menge von mindestens einem Polypeptid nach Anspruch 13.

**Patentansprüche für folgenden Vertragsstaat : AT**

1. Ein einzelliger Wirt, der mit wenigstens einem rekombinanten DNA-Molekül transformiert ist, das durch eine DNA-Sequenz gekennzeichnet ist, die für ein humanes Polypeptid kodiert, das eine Phospholipase $A_2$ hemmende Aktivität und die immunologische Aktivität eines humanen Lipocortins hat und das ausgewählt ist aus der Gruppe bestehend aus:

   (a) der cDNA-Insertion von λ LC (ATCC Hinterlegungs Nr. 68812), das an beiden Enden durch EcoRI-Restriktionsstellen gebunden ist,

   (b) DNA-Sequenzen, die zu der DNA-Insertion von Anspruch 1 (a) hybridisieren und die für ein humanes Polypeptid kodieren, das eine Phospholiphase $A_2$ hemmende Aktivität und die immunologische Aktivität des humanen Lipocortins besitzt, das durch die cDNA-Insertion von Teil (a) oben kodiert wird, und

   (c) DNA-Sequenzen, die als Folge des genetisches Codes zu der DNA-Insertion und Sequenzen von (a) und (b) oben degeneriert sind,

   wobei die DNA-Sequenz in dem Molekül operativ mit einer Expressionssteuersequenz verbunden ist.

2. Der transformierte Wirt nach Anspruch 1, wobei die DNA-Sequenz besteht aus:

ATGGCAATGGTATCAGAATTCCTCAAGCAGGCCTGGTTTATTGAAAATGAAGAGC

AGGAATATGTTCAAACTGTGAAGTCATCCAAAGGTGGTCCCGGATCAGCGGTGAG

CCCCTATCCTACCTTCAATCCATCCTCGGATGTCGCTGCCTTGCATAAGGCCATA

ATGGTTAAAGGTGTGGATGAAGCAACCATCATTGACATTCTAACTAAGCGAAACA

ATGCACAGCGTCAACAGATCAAAGCAGCATATCTCCAGGAAACAGGAAAGCCCCT

GGATGAAACACTTAAGAAAGCCCTTACAGGTCACCTTGAGGAGGTTGTTTTAGCT

CTGCTAAAAACTCCAGCGCAATTTGATGCTGATGAACTTCGTGCTGCCATGAAGG

GCCTTGGAACTGATGAAGATACTCTAATTGAGATTTTGGCATCAAGAACTAACAA

AGAAATCAGAGACATTAACAGGGTCTACAGAGAGGAACTGAAGAGAGATCTGGCC

AAAGACATAACCTCAGACACATCTGGAGATTTTCGGAACGCTTTGCTTTCTCTTG

CTAAGGGTGACCGATCTGAGGACTTTGGTGTGAATGAAGACTTGGCTGATTCAGA

TGCCAGGGCCTTGTATGAAGCAGGAGAAAGGAGAAAGGGGACAGACGTAAACGTG

TTCAATACCATCCTTACCACCAGAAGCTATCCACAACTTCGCAGAGTGTTTCAGA

AATACACCAAGTACAGTAAGCATGACATGAACAAAGTTCTGGACCTGGAGTTGAA


AGGTGACATTGAGAAATGCCTCACAGCTATCGTGAAGTGCGCCACAAGCAAACCA

GCTTTCTTTGCAGAGAAGCTTCATCAAGCCATGAAAGGTGTTGGAACTCGCCATA

AGGCATTGATCAGGATTATGGTTTCCCGTTCTGAAATTGACATGAATGATATCAA

AGCATTCTATCAGAAGATGTATGGTATCTCCCTTTGCCAAGCCATCCTGGATGAA

ACCAAAGGAGATTATGAGAAATCCTGGTGGCTCTTTGTGGAGGAAACTAA**.**


3. Ein einzelliger Wirt, der mit wenigstens einem rekombinanten DNA-Molekül transformiert ist, ausgewählt aus der Gruppe bestehend aus:

(a) der cDNA-Insertion von pNLipl (ATCC Hinterlegungs Nr. 68811), das an beiden Enden durch EcoRI Restriktionsstellen gebunden ist,

(b) DNA-Sequenzen, die zu der DNA-Insertion von Anspruch 3(a) hybridisieren und die für ein humanes Polypeptid, das eine Phospholipase $A_2$ hemmende Aktivität und eine immunologische Aktivität des humanen Lipocortins hat, das durch die cDNA-Insertion von Teil (a) oben kodiert wird, und

(c) DNA-Sequenzen, die als Folge des genetischen Codes zu DNA-Insertionen und Sequenzen von (a) und (b) oben degeneriert sind,

wobei die DNA-Sequenz in dem Molekül operativ mit einer Expressionssteuersequenz verbunden ist.

4. Ein einzelliger Wirt, der mit wenigstens einem rekombinanten DNA-Molekül, gekennzeichnet durch die Sequenz:

GAATTCAAGAGAAAGTACGGCAAGTCCCTGTACTATTATATCCAGCAAGACACTA

AGGGCGACTACCAGAAAGCGCTGCTGTACCTGTGTGGTGGAGATGACTGA,

transformiert ist,

wobei die DNA-Sequenz in dem Molekül operativ mit einer Expressionssteuersequenz verbunden ist.

5. Der transformierte Wirt nach einem der Ansprüche 1 bis 4, wobei das rekombinante DNA-Molekül ausgewählt ist aus der Gruppe bestehend aus pLipPLT4A, pLipPLT4T und pSVL9109.

6. Ein einzelliger Wirt, der mit wenigstens einem rekombinanten DNA-Molekül pBg120 (ATCC Hinterlegungs Nr. 74106), transformiert ist,

wobei die DNA-Sequenz in dem Molekül operativ mit einer Expressionssteuersequenz verbunden ist.

7. Ein einzelliger Wirt, der mit wenigstens einem rekombinanten DNA-Molekül pNLipl (ATCC Hinterlegungs Nr. 68811), transformiert ist,

wobei die DNA-Sequenz in dem Molekül operativ mit einer Expressionssteuersequenz verbunden ist.

**8.** Ein einzelliger Wirt, der mit wenigstens einem rekombinanten DNA-Molekül bestehend aus:

```
ATGGCAATGGTATCAGAATTCCTCAAGCAGGCCTGGTTTATTGAAAATGAAGAGC
AGGAATATGTTCAAACTGTGAAGTCATCCAAAGGTGGTCCCGGATCAGCGGTGAG
CCCCTATCCTACCTTCAATCCATCCTCGGATGTCGCTGCCTTGCATAAGGCCATA
ATGGTTAAAGGTGTGGATGAAGCAACCATCATTGACATTCTAACTAAGCGAAACA
ATGCACAGCGTCAACAGATCAAAGCAGCATATCTCCAGGAAACAGGAAAGCCCCT
GGATGAAACACTTAAGAAAGCCCTTACAGGTCACCTTGAGGAGGTTGTTTTAGCT
CTGCTAAAAACTCCAGCGCAATTTGATGCTGATGAACTTCGTGCTGCCATGAAGG
GCCTTGGAACTGATGAAGATACTCTAATTGAGATTTTGGCATCAAGAACTAACAA
AGAAATCAGAGACATTAACAGGGTCTACAGAGAGGAACTGAAGAGAGATCTGGCC
AAAGACATAACCTCAGACACATCTGGAGATTTTCGGAACGCTTTGCTTTCTCTTG
CTAAGGGTGACCGATCTGAGGACTTTGGTGTGAATGAAGACTTGGCTGATTCAGA
TGCCAGGGCCTTGTATGAAGCAGGAGAAAGGAGAAAGGGGACAGACGTAAACGTG
TTCAATACCATCCTTACCACCAGAAGCTATCCACAACTTCGCAGAGTGTTTCAGA
AATACACCAAGTACAGTAAGCATGACATGAACAAAGTTCTGGACCTGGAGTTGAA
AGGTGACATTGAGAAATGCCTCACAGCTATCGTGAAGTGCGCCACAAGCAAACCA
GCTTTCTTTGCAGAGAAGCTTCATCAAGCCATGAAAGGTGTTGGAACTCGCCATA
AGGCATTGATCAGGATTATGGTTTCCCGTTCTGAAATTGACATGAATGATATCAA
AGCATTCTATCAGAAGATGTATGGTATCTCCCTTTGCCAAGCCATCCTGGATGAA
ACCAAAGGAGATTATGAGAAAATCCTGGTGGCTCTTTGTGGAGGAAACTAA
```

transformiert ist,

wobei die DNA-Sequenz in dem Molekül operativ mit einer Expressionssteuersequenz verbunden ist.

**9.** Ein einzelliger Wirt, der mit wenigstens einer rekombinanten DNA-Molekül, bestehend aus:

```
GAATTCAAGAGAAAGTACGGCAAGTCCCTGTACTATTATATCCAGCAAGACACTA
AGGGCGACTACCAGAAAGCGCTGCTGTACCTGTGTGGTGGAGATGACTGA
```

transformiert ist,

wobei die DNA-Sequenz in dem Molekül operativ mit einer Expressionssteuersequenz verbunden ist.

**10.** Der transformierte Wirt nach einem der Ansprüche 1 bis 9, wobei die Expressionskontrollsequenz ausgewählt ist aus der Gruppe bestehend aus dem lac-System, dem β-Lactamase-System, dem trp-System, dem tac-System, dem trc-System, dem Hauptoperator- und Promotorbereichen des Phagen λ , dem Kontrollbereich von fd Hüllprotein, dem Promotor für 3-Phosphoglyceratkinase oder anderen glykolytischen Enzymen, den Promotoren von Säurephosphaten, den Promotoren von Hefe-α-Paarung-Faktoren, den Promotoren von Säugetierzellen, den SV40 Früh- und Spätpromotoren, den Adenovirusspätpromotoren und den Metallthioninpromotoren und anderen Sequenzen, welche die Expression von Genen von prokaryotischen oder eukaryotischen Zellen und ihren Viren in Kombination derselben kontrollieren.

**11.** Ein Verfahren zum Herstellen eines humanen Polypeptids, das eine Phospholipase A$_2$ hemmende Aktivität und die immunologische Aktivität eines humanen Lipocortins aufweist, umfassend die Stufe des Kultivierens eines transformierten Wirten nach einem der Ansprüche 1 bis 10 und und Sammeln des Polypeptids.

**12.** Das Verfahren nach Anspruch 11 zum Herstellen eines humanen Polypeptides, das Phospholipase A$_2$ hemmende Aktivität und die immunologische Aktivität eines humanen Lipocortins hat, wobei das Polypeptid ausgewählt ist aus der Gruppe bestehend aus:

(a)

```
MetAlaMetValSerGluPheLeuLysGlnAlaTrpPheIleGluAsnGluGlu
GlnGluTyrValGlnThrValLysSerSerLysGlyGlyProGlySerAlaVal
SerProTyrProThrPheAsnProSerSerAspValAlaAlaLeuHisLysAla
IleMetValLysGlyValAspGluAlaThrIleIleAspIleLeuThrLysArg
AsnAsnAlaGlnArgGlnGlnIleLysAlaAlaTyrLeuGlnGluThrGlyLys
ProLeuAspGluThrLeuLysLysAlaLeuThrGlyHisLeuGluGluValVal
LeuAlaLeuLeuLysThrProAlaGlnPheAspAlaAspGluLeuArgAlaAla
MetLysGlyLeuGlyThrAspGluAspThrLeuIleGluIleLeuAlaSerArg
ThrAsnLysGluIleArgAspIleAsnArgValTyrArgGluGluLeuLysArg
AspLeuAlaLysAspIleThrSerAspThrSerGlyAspPheArgAsnAlaLeu
LeuSerLeuAlaLysGlyAspArgSerGluAspPheGlyValAsnGluAspLeu
AlaAspSerAspAlaArgAlaLeuTyrGluAlaGlyGluArgArgLysGlyThr
AspValAsnValPheAsnThrIleLeuThrThrArgSerTyrProGlnLeuArg
ArgValPheGlnLysTyrThrLysTyrSerLysHisAspMetAsnLysValLeu
AspLeuGluLeuLysGlyAspIleGluLysCysLeuThrAlaIleValLysCys
```

```
AlaThrSerLysProAlaPhePheAlaGluLysLeuHisGlnAlaMetLysGly
ValGlyThrArgHisLysAlaLeuIleArgIleMetValSerArgSerGluIle
AspMetAsnAspIleLysAlaPheTyrGlnLysMetTyrGlyIleSerLeuCys
GlnAlaIleLeuAspGluThrLysGlyAspTyrGluLysIleLeuValAlaLeu
CysGlyGlyAsn;
```

(b)

AlaMetValSerGluPheLeuLysGlnAlaTrpPheIleGluAsnGluGluGln
GluTyrValGlnThrValLysSerSerLysGlyGlyProGlySerAlaValSer
ProTyrProThrPheAsnProSerSerAspValAlaAlaLeuHisLysAlaIle
MetValLysGlyValAspGluAlaThrIleIleAspIleLeuThrLysArgAsn
AsnAlaGlnArgGlnGlnIleLysAlaAlaTyrLeuGlnGluThrGlyLysPro
LeuAspGluThrLeuLysLysAlaLeuThrGlyHisLeuGluGluValValLeu
AlaLeuLeuLysThrProAlaGlnPheAspAlaAspGluLeuArgAlaAlaMet
LysGlyLeuGlyThrAspGluAspThrLeuIleGluIleLeuAlaSerArgThr
AsnLysGluIleArgAspIleAsnArgValTyrArgGluGluLeuLysArgAsp
LeuAlaLysAspIleThrSerAspThrSerGlyAspPheArgAsnAlaLeuLeu
SerLeuAlaLysGlyAspArgSerGluAspPheGlyValAsnGluAspLeuAla
AspSerAspAlaArgAlaLeuTyrGluAlaGlyGluArgArgLysGlyThrAsp
ValAsnValPheAsnThrIleLeuThrThrArgSerTyrProGlnLeuArgArg
ValPheGlnLysTyrThrLysTyrSerLysHisAspMetAsnLysValLeuAsp
LeuGluLeuLysGlyAspIleGluLysCysLeuThrAlaIleValLysCysAla
ThrSerLysProAlaPhePheAlaGluLysLeuHisGlnAlaMetLysGlyVal
GlyThrArgHisLysAlaLeuIleArgIleMetValSerArgSerGluIleAsp
MetAsnAspIleLysAlaPheTyrGlnLysMetTyrGlyIleSerLeuCysGln
AlaIleLeuAspGluThrLysGlyAspTyrGluLysIleLeuValAlaLeuCys
GlyGlyAsn;

und

(c) Polypeptid-Fragmenten der Polypeptide von Teil (a) oder (b) oben, die eine biologische oder immunologische Aktivität des Lipocortins aufweisen, das oben in Teil (a) oder (b) definiert ist.

**13.** Das Verfahren nach Anspruch 11 zum Herstellen eines N-Lipocortinpolypeptids, gekennzeichnet durch die folgenden Eigenschaften:

(a) es hat die Fähigkeit das Enzym Phospholipase $A_2$ zu hemmen,

(b) es weist die tryptischen Fragmente auf:

   (1) LeuTyrAspSerMetLys;

   (2) LeuSerLeuAsnGlyAspThrSer ThrProProSerAlaTyrGly;

   (3) SerLeuTyrTyrTyrIleGln GlnAspThrLys;

   (4) TrpIleSerIleMetThrGluArg;

   (5) SerTyrSerProTyrAspMet LeuGluSerIle;

   (6) LeuLeuValValTyrProX ThrGlnIleLeu; worin x irgendeine Aminosäure ist, und

(c) es hat ein Carboxy-Ende bestehend aus der Aminosäuresequenz,

LysArgLysTyrGlyLysSerLeuTyrTyrTyr-
IleGlnGlnAspThrLysGly AspTyrGlnLysAlaLeuLeuTyrLeuCysGly-
GlyAspAsp.

**14.** Das Verfahren nach Anspruch 11, zum Herstellen eines humanlipocortinähnlichen Polypeptids, das durch die rekombinanten DNA-Moleküle der Ansprüche 1 oder 2 kodiert wird.

**15.** Das Verfahren gemäß Anspruch 11 zum Herstellen eines Polypeptides, worin das Polypeptid ausgewählt ist aus der Gruppe bestehend aus:

(a)

GlyGlyProGlySerAlaValSerProTyrProThrPheAsnProSerSerAsp

ValAlaAlaLeuHisLysAlaIleMetValLysGlyValAspGluAlaThrIle

IleAspIleLeuThrLysArgAsnAsnAlaGlnArgGlnGlnIleLysAlaAla

TyrLeuGlnGluThrGlyLysProLeuAspGluThrLeuLysLysAlaLeuThr

GlyHisLeuGluGluValValLeuAlaLeuLeuLysThrProAlaGlnPheAsp

AlaAspGluLeuArgAlaAlaMetLysGlyLeuGlyThrAspGluAspThrLeu

IleGluIleLeuAlaSerArgThrAsnLysGluIleArgAspIleAsnArgVal

TyrArgGluGluLeuLysArgAspLeuAlaLysAspIleThrSerAspThrSer

GlyAspPheArgAsnAlaLeuLeuSerLeuAlaLysGlyAspArgSerGluAsp

PheGlyValAsnGluAspLeuAlaAspSerAspAlaArgAlaLeuTyrGluAla

GlyGluArgArgLysGlyThrAspValAsnValPheAsnThrIleLeuThrThr

ArgSerTyrProGlnLeuArgArgValPheGlnLysTyrThrLysTyrSerLys

HisAspMetAsnLysValLeuAspLeuGluLeuLysGlyAspIleGluLysCys

LeuThrAlaIleValLysCysAlaThrSerLysProAlaPhePheAlaGluLys

LeuHisGlnAlaMetLysGlyValGlyThrArgHisLysAlaLeuIleArgIle

MetValSerArgSerGluIleAspMetAsnAspIleLysAlaPheTyrGlnLys

MetTyrGlyIleSerLeuCysGlnAlaIleLeuAspGluThrLysGlyAspTyr

GluLysIleLeuValAlaLeuCysGlyGly;

(b)

GlyGlyProGlySerAlaValSerProTyrProThrPheAsnProSerSerAsp

ValAlaAlaLeuHisLysAlaIleMetValLysGlyValAspGluAlaThrIle

IleAspIleLeuThrLysArgAsnAsnAlaGlnArgGlnGlnIleLysAlaAla

TyrLeuGlnGluThrGlyLysProLeuAspGluThrLeuLysLysAlaLeuThr

GlyHisLeuGluGluValValLeuAlaLeuLeuLysThrProAlaGlnPheAsp

AlaAspGluLeuArgAlaAlaMetLysGlyLeuGlyThrAspGluAspThrLeu

IleGluIleLeuAlaSerArgThrAsnLysGluIleArgAspIleAsnArgVal

TyrArgGluGluLeuLysArgAspLeuAlaLysAspIleThrSerAspThrSer

GlyAspPheArgAsnAlaLeuLeuSerLeuAlaLysGlyAspArgSerGluAsp

PheGlyValAsnGluAspLeuAlaAspSerAspAlaArgAlaLeuTyrGluAla

GlyGluArgArgLysGlyThrAspValAsnValPheAsnThrIleLeuThrThr;

(c)

GlyGlyProGlySerAlaValSerProTyrProThrPheAsnProSerSerAsp
ValAlaAlaLeuHisLysAlaIleMetValLysGlyValAspGluAlaThrIle
IleAspIleLeuThrLysArgAsnAsnAlaGlnArgGlnGlnIleLysAlaAla
TyrLeuGlnGluThrGlyLysProLeuAspGluThrLeuLysLysAlaLeuThr
GlyHisLeuGluGluValValLeuAlaLeuLeuLysThrProAlaGlnPheAsp
AlaAspGluLeuArgAlaAlaMetLysGlyLeuGlyThrAspGluAspThrLeu
IleGluIleLeuAlaSerArgThrAsnLysGluIleArgAspIleAsnArgVal
TyrArgGluGluLeuLysArgAspLeuAlaLysAspIleThrSerAspThrSer
GlyAspPheArgAsnAlaLeuLeuSerLeuAlaLysGlyAspArgSerGluAsp
PheGlyValAsnGluAspLeuAlaAspSerAspAlaArgAlaLeuTyrGluAla
GlyGlu;

(d)

ValSerGluPheLeuLysGlnAlaTrpPheIleGluAsnGluGluGlnGluTyr
ValGlnThrValLysSerSerLysGlyGlyProGlySerAlaValSerProTyr
ProThrPheAsnProSerSerAspValAlaAlaLeuHisLysAlaIleLeuVal
LysGlyValAspGluAlaThrIleIleAspIleLeuThrLysArgAsnAsnAla
GlnArgGlnGlnIleLysAlaAlaTyrLeuGlnGluThrGlyLysProLeuAsp
GluThrLeuLysLysAlaLeuThrGlyHisLeuGluGluValValLeuAlaLeu
LeuLysThrProAlaGlnPheAspAlaAspGluLeuArgAlaAlaLeuLysGly
LeuGlyThrAspGluAspThrLeuIleGluIleLeuAlaSerArgThrAsnLys
GluIleArgAspIleAsnArgValTyrArgGluGluLeuLysArgAspLeuAla
LysAspIleThrSerAspThrSerGlyAspPheArgAsnAlaLeuLeuSerLeu
AlaLysGlyAspArgSerGluAspPheGlyValAsnGluAspLeuAlaAspSer
AspAlaArgAlaLeuTyrGluAlaGlyGluArgArgLysGlyThrAspValAsn
ValPheAsnThrIleLeuThrThrArgSerTyrProGlnLeuArgArgValPhe
GlnLysTyrThrLysTyrSerLysHisAsp;

(e)

ValSerGluPheLeuLysGlnAlaTrpPheIleGluAsnGluGluGlnGluTyr
ValGlnThrValLysSerSerLysGlyGlyProGlySerAlaValSerProTyr
ProThrPheAsnProSerSerAspValAlaAlaLeuHisLysAlaIleLeuVal
LysGlyValAspGluAlaThrIleIleAspIleLeuThrLysArgAsnAsnAla

60

GlnArgGlnGlnIleLysAlaAlaTyrLeuGlnGluThrGlyLysProLeuAsp
GluThrLeuLysLysAlaLeuThrGlyHisLeuGluGluValVal;

und
(f)

ValSerGluPheLeuLysGlnAlaTrpPheIleGluAsnGluGluGlnGluTyr
ValGlnThrValLysSerSerLysGlyGlyProGlySerAlaValSerProTyr
ProThrPheAsnProSerSerAspValAlaAlaLeuHisLysAlaIleLeuVal
LysGlyValAspGluAlaThrIleIleAspIleLeuThrLysArgAsnAsnAla
GlnArgGlnGlnIleLysAlaAlaTyrLeuGlnGluThrGlyLysProLeuAsp
GluThrLeuLysLysAlaLeuThrGlyHisLeuGluGluValValLeuAlaLeu
LeuLysThrProAlaGlnPheAspAlaAspGluLeuArgAlaAlaLeuLysGly
LeuGlyThrAspGluAspThrLeuIleGluIleLeuAlaSerArgThrAsnLys
GluIleArgAspIleAsnArgValTyrArgGluGluLeuLysArgAspLeuAla
LysAspIleThrSerAspThrSerGlyAspPheArgAsnAlaLeuLeuSerLeu
AlaLysGlyAspArgSerGluAspPheGlyValAsnGluAsp.

16. Das Verfahren nach Anspruch 11, zum Herstellen eines Polypeptides, wobei das Polypeptid die Aminosäuresequenz:

GlyGlyProGlySerAlaValSerProTyrProThrPheAsnProSerSerAsp
ValAlaAlaLeuHisLysAlaIleMetValLysGlyValAspGluAlaThrIle
IleAspIleLeuThrLysArgAsnAsnAlaGlnArgGlnGlnIleLysAlaAla
TyrLeuGlnGluThrGlyLysProLeuAspGluThrLeuLysLysAlaLeuThr
GlyHisLeuGluGluValValLeuAlaLeuLeuLysThrProAlaGlnPheAsp
AlaAspGluLeuArgAlaAlaMetLysGlyLeuGlyThrAspGluAspThrLeu
IleGluIleLeuAlaSerArgThrAsnLysGluIleArgAspIleAsnArgVal
TyrArgGluGluLeuLysArgAspLeuAlaLysAspIleThrSerAspThrSer
GlyAspPheArgAsnAlaLeuLeuSerLeuAlaLysGlyAspArgSerGluAsp
PheGlyValAsnGluAspLeuAlaAspSerAspAlaArgAlaLeuTyrGluAla
GlyGluArgArgLysGlyThrAspValAsnValPheAsnThrIleLeuThrThr
ArgSerTyrProGlnLeuArgArgValPheGlnLysTyrThrLysTyrSerLys
HisAspMetAsnLysValLeuAspLeuGluLeuLysGlyAspIleGluLysCys
LeuThrAlaIleValLysCysAlaThrSerLysProAlaPhePheAlaGluLys
LeuHisGlnAlaMetLysGlyValGlyThrArgHisLysAlaLeuIleArgIle
MetValSerArgSerGluIleAspMetAsnAspIleLysAlaPheTyrGlnLys
MetTyrGlyIleSerLeuCysGlnAlaIleLeuAspGluThrLysGlyAspTyr
GluLysIleLeuValAlaLeuCysGlyGlyAsn

hat.

17. Der transformierte Wirt nach einem der Ansprüche 1 bis 10, ausgewählt aus der Gruppe bestehend aus den Stämmen von E. coli, Pseudomonas, Bacillus, Streptomyces, Hefezellen, anderen Fungi, Mäusezellen oder anderen tierischen Zellwirten, Pflanzenzellwirten und humanen Gewebezellen.

**Revendications**
**Revendications pour les Etats contractants suivants : BE, CH, DE, FR, GB, IT, LI, LU, NL, SE**

1. Une molécule d'ADN recombinant caractérisée par une séquence d'ADN qui code pour un polypeptide humain ayant une activité inhibitrice de phospholipase A2 et l'activité immunologique d'une lipocortine humaine et qui est choisie dans le groupe qui est formé de :
   a) l'insert d'ADNc de λ LC (ATCC N° de dépôt 68812) relié aux deux extrémités par des sites de restriction Eco RI,
   b) les séquences d'ADN qui hybrident pour l'insert d'ADN de la revendication la et qui encodent un polypeptide humain qui a une action inhibitrice sur la phospholipase A2 et l'activité immunologique de la lipocortine humaine encodée par l'insert d' ADNc de la partie a) ci-dessus et
   c) les séquences d'ADN qui sont dégénérées en conséquence du code génétique pour l'insert d'ADN et les séquences de a) et de b) ci-dessus.

2. La molécule d'ADN recombinant selon la revendication 1, caractérisée en ce que la séquence d'ADN consiste en :

ATGGCAATGGTATCAGAATTCCTCAAGCAGGCCTGGTTTATTGAAAATGAAGAGC
AGGAATATGTTCAAACTGTGAAGTCATCCAAAGGTGGTCCCGGATCAGCGGTGAG
CCCCTATCCTACCTTCAATCCATCCTCGGATGTCGCTGCCTTGCATAAGGCCATA
ATGGTTAAAGGTGTGGATGAAGCAACCATCATTCACATTCTAACTAACCCAAACA
ATGCACACCCTCAACACATCAAAGCAGCATATCTCCAGGAAACAGGAAAGCCCCT
CGATGAAACACTTAAGAAAGCCCTTACAGGTCACCTTGAGGAGGTTGTTTTAGCT
CTGCTAAAAACTCCAGCGCAATTTGATGCTGATGAACTTCGTGCTGCCATGAAGG
GCCTTGGAACTGATGAAGATACTCTAATTGAGATTTTGGCATCAAGAACTAACAA
AGAAATCAGAGACATTAACAGGGTCTACAGAGAGGAACTGAAGAGAGATCTGGCC
AAAGACATAACCTCAGACACATCTGGAGATTTTCGGAACGCTTTGCTTTCTCTTG
CTAAGGGTGACCGATCTGAGGACTTTGGTGTGAATGAAGACTTGGCTGATTCAGA

TGCCAGGGCCTTGTATGAAGCAGGAGAAAGGAGAAAGGGGACAGACGTAAACGTG
TTCAATACCATCCTTACCACCAGAAGCTATCCACAACTTCGCAGAGTGTTTCAGA
AATACACCAAGTACAGTAAGCATGACATGAACAAAGTTCTGGACCTGGAGTTGAA
AGGTGACATTGAGAAATGCCTCACAGCTATCGTGAAGTGCGCCACAAGCAAACCA
GCTTTCTTTGCAGAGAAGCTTCATCAAGCCATGAAAGGTGTTGGAACTCGCCATA
AGGCATTGATCAGGATTATGGTTTCCCGTTCTGAAATTGACATGAATGATATCAA
AGCATTCTATCAGAAGATGTATGGTATCTCCCTTTGCCAAGCCATCCTGGATGAA
ACCAAGGATTATGAGAAAATCCTGGTGGCTCTTTGTGGAGGAAACTAA.

EP 0 209 568 B1

3. Une molécule d'ADN recombinant choisie dans le groupe formé de :

a) l'insert d'ADNc de pNLIp1 (ATCC n° de dépôt 68811) limitée aux deux extrémités par des sites de restriction Eco RI

b) des séquences d'ADN qui hybrident pour l'insert d'ADN de la revendication 3a) et qui encodent un polypeptide humain qui a une action inhibitrice de la phospholipase A2 et l'activité immunologique de la lipocortine humaine encodée par l'insert d'ADNc de la partie a) ci-dessus, et

c) des séquences d'ADN qui sont dégénérées par suite du code génétique pour l'insert d'ADN et des séquences de a) et de b) ci-dessus.

4. Une molécule d'ADN recombinant, caractérisée par la séquence :

GAATTCAAGAGAAAGTACGGCAAGTCCCTGTACTTATATATCCAGCAAGACACTA
AGGGCGACTACCAGAAAGCCCTGCTGTACCGTGTGGTGGAGATGACTGA.

5. La molécule d'ADN recombinant selon la revendication 1 ou 2, caractérisée en ce que cette séquence d'ADN est reliée fonctionnellement à une séquence de contrôle de l'expression dans la molécule.

6. La molécule d'ADN recombinant selon la revendication 5, caractérisée en ce que cette séquence de contrôle de l'expression est choisie dans le groupe formé du système lac, du système β-lactamase, du système trp, du système tac, du système trc, de l'opérateur principal et des régions du promoteur du phageλ de la région de contrôle de la protéine de couverture fd, du promoteur pour la 3-phosphoglycérate kinase ou d'autres enzymes glycolytiques, des promoteurs des phosphases acides, des promoteurs des facteurs de couplage en des promoteurs pour les cellules de mammifères, des promoteurs précoces et tardifs SV 40, du promoteur tardif d'adenovirus et du promoteur de métallothionine et d'autres séquences qui contrôlent l'expression des gènes des cellules procaryotes ou eucaryotes et de leurs virus et des combinaisons de ceux-ci.

7. La molécule d'ADN recombinant selon la revendication 5 qui est choisie dans le groupe qui consiste en pLipPLT 4A, pLIpPLT 4T et en pSVL 9109.

8. La molécule d'ADN recombinant pBgl20 (ATCC n° de dépôt 74106).

9. La molécule d'ADN recombinant pNLipl (ATCC n° de dépôt n° 68811).

10. Un hôte unicellulaire transformé par au moins une molécule d'ADN recombinant selon la revendication 5.

11. L'hôte transformé selon la revendication 10 choisi dans le groupe formé des souches d'E. coli, de Pseudomonas, de Bacillus, de Streptomyces, de cellules de levure, d'autres champignons, des cellules de souris ou d'autres hôtes de cellules animales des hôtes de cellules végétales, et des cellules de tissu humain.

12. Un polypeptide humain qui a l'action inhibitrice sur la phospholipase A2 et l'activité immunologique d'une lipocortine humaine, ce polypeptide étant choisi dans le groupe qui consiste en :

a)

MetAlaMetValSerGluPheLeuLysGlnAlaTrpPheIleGluAsnGluGlu
GlnGluTyrValGlnThrValLysSerSerLysGlyGlyProGlySerAlaVal
SerProTyrProThrPheAsnProSerSerAspValAlaAlaLeuHisLysAla
IleMetValLysGlyValAspGluAlaThrIleIleAspIleLeuThrLysArg
AsnAsnAlaGlnArgGlnGlnIleLysAlaAlaTyrLeuGlnGluThrGlyLys
ProLeuAspGluThrLeuLysLysAlaLeuThrGlyHisLeuGluGluValVal
LeuAlaLeuLeuLysThrProAlaGlnPheAspAlaAspGluLeuArgAlaAla
MetLysGlyLeuGlyThrAspGluAspThrLeuIleGluIleLeuAlaSerArg
ThrAsnLysGluIleArgAspIleAsnArgValTyrArgGluGluLeuLysArg
AlaAspSerAspAlaArgAlaLeuTyrGluAlaGlyGluArgArgLysGlyThr
AspValAsnValPheAsnThrIleLeuThrThrArgSerTyrProGlnLeuArg
ArgValPheGlnLysTyrThrLysTyrSerLysHisAspMetAsnLysValLeu
AspLeuGluLeuLysGlyAspIleGlyLysCysLeuThrAlaIleValLysCys
AlaThrSerLysProAlaPhePheAlaGluLysLeuHisGlnAlaMetLysGly
ValGlyThrArgHisLysAlaLeuIleArgIleMetValSerArgSerGluIle
AspMetAsnAspIleLysAlaPheTyrGlnLysMetTyrGlyIleSerLeuCys
GlnAlaIleLeuAspGluThrLysGlyAspTyrGluLysIleLeuValAlaLeu
CysGlyGlyAsn ;

b)

AlaMetValSerGluPheLeuLysGlnAlaTrpPheIleGluAsnGluGluGln
GluTyrValGlnThrValLysSerSerLysGlyGlyProGlySerAlaValSer
ProTyrProThrPheAsnProSerSerAspValAlaAlaLeuHisLysAlaIle
MetValLysGlyValAspGluAlaThrIleIleAspIleLeuThrLysArgAsn
AsnAlaGlnArgGlnGlnIleLysAlaAlaTyrLeuGlnGluThrGlyLysPro
LeuAspGluThrLeuLysLysAlaLeuThrGlyHisLeuGluGluValValLeu
AlaLeuLeuLysThrProAlaGlnPheAspAlaAspGluLeuArgAlaAlaMet
LysGlyLeuGlyThrAspGluAspThrLeuIleGluIleLeuAlaSerArgThr
AsnLysGluIleArgAspIleAsnArgValTyrArgGluGluLeuLysArgAsp
LeuAlaLysAspIleThrSerAspThrSerGlyAspPheArgAsnAlaLeuLeu
SerLeuAlaLysGlyAspArgSerGluAspPheGlyValAsnGluAspLeuAla
AspSerAspAlaArgAlaLeuTyrGluAlaGlyGluArgArgLysGlyThrAsp

```
ValAsnValPheAsnThrIleLeuThrThrArgSerTyrProGlnLeuArgArg
ValPheGlnLysTyrThrLysTyrSerLysHisAspMetAsnLysValLeuAsp
LeuGluLeuLysGlyAspIleGluLysCysLeuThrAlaIleValLysCysAla
ThrSerLysProAlaPhePheAlaGluLysLeuHisGlnAlaMetLysGlyVal
GlyThrArgHisLysAlaLeuIleArgIleMetValSerArgSerGluIleAsp
MetAsnAspIleLysAlaPheTyrGlnLysMetTyrGlyIleSerLeuCysGln
AlaIleLeuAspGluThrLysGlyAspTyrGluLysIleLeuValAlaLeuCys
GlyclyAsn,
```

et

c) des fragments de polypeptide du polypeptide de la partie a) ou b) ci-dessus qui manifeste une activité biologique ou immunologique de la lipocortine définie à la partie a) ou b) ci-dessus.

13. Un polypeptide de N-lipocortine, caractérisé par les propriétés suivantes :

a) il a la capacité d'inhiber l'enzyme phospholipase A2,

b) il comprend les fragments tryptiques :

(1) LeuTyrAspSerMelLys ;

(2) LeuSerLeuAsnGlyAspThrSer ThrProProSerAlaTyrGly ;

(3) SerLeuTyrTyrIleGlnGlnAspThrLys ;

(4) TrpIleSerIleMetGluArg ;

(5) SerTyrSerProTyrAspMetLeuGluSerIle ;

(6) LeuLeuValValTyrProXThrGlnIleLeu ;

dans lesquels X est n'importe quel amino-acide et

c) il a une extrémité carboxylique formée de la sequence d'amino-acide :

```
LysArgLysTyrGlyLysSerLeuTyrTyrTyrIleGlnGlnAspThrLysGly
AspTyrGlnLysAlaLeuLeuTyrLeuCysGlyGlyAspAsp.
```

14. Un polypeptide du type lipocortine humain encodé par des molécules d'ADN recombinant de la revendication 1 ou 2.

15. Le polypeptide selon la revendication 12, caractérisé en ce que le polypeptide est choisi dans le groupe formé de :

a)

```
GlyGlyProGlySerAlaValSorProTyrProThrPheAsnProSerSerAsp
ValAlaAlaLeuIleLysAlaIleMetValLysClyValAspCluAlaThrIle
IleAspIleLeuThrLysArgAsnAsnAlaGlnArgGlnGlnIleLysAlaAla
TyrLeuGlnGluThrGlyLysProLeuAspGluThrLeuLysLysAlaLeuThr
GlyHisLeuGluGluValValLeuAlaLeuLeuLysThrProAlaGlnPheAsp
AlaAspGluLeuArgAlaAlaMetLysGlyLeuGlyThrAspGluAspThrLeu
ileGluIleLeuAlaSerArgThrAsnLysGluIleArgAspIleAsnArgVal
TyrargGluGluLeuLysArgAspLeuAlaLysAspIleThrSerAspThrSer
GlyAspPheArgAsnAlaLeuLeuSerLeuAlaLysGlyAspArgSerGluAsp
PheGlyValAsnGluAspLeuAlaAspSerAspAlaArgAlaLeutyrGluAla
GlyGluArgArgLysGlyThrAspValAsnValPheAsnThrIleLeuThrThr
ArgSerTyrProGlnLeuArgArgValPheGlnLysTyrThrLysTyrSerLys
HisAspMetAsnLysValLeuAspLeuGluLeuLysGlyAspIleGluLysCys
LeuThrAlaIleValLysCysAlaThrSerLysProAlaPhePheAlaGluGly
LeuHisGlnAlaMetLysGlyValGlyThrArgHisLysAlaLeuIleArgIle
MetValSerArgSerGluIleAspMetAsnAspIleLysAlaPheTyrGlnLys
MetTyrGlyIleSerLeuCysGlnAlsIleLeuAspGluThrLysGlyAspTyr
GluLysIleLeuValAlaLeuCysGlyGly ;
```

(b)

```
GlyGlyProGlySerAlaValSerProTyrProThrPheAsnProSerSerAsp
ValAlaAlaLeuHisLysAlaIleMetValLysGlyValAspGluAlaThrIle
IleAspIleLeuThrLysArgAsnAsnAlaGlnArgGlnGlnIleLysAlaAla
TyrLeuGlnGluThrGlyLysProLeuAspGluThrLeuLysLysAlaLeuThr
GlyHisLeuGluGluValVAlLeuAlaLeuLeuLysThrProAlaGlnPheAsp
AlaAspGluLeuArgAlaAlaMetLysGlyLeuGlyThrAspGluAspThrLeu
IleGluIleLeuAlaSerArgThrAsnLysGluIleArgAspIleAsnArgVal
TyrArgGluGluLeuLysArgAspLeuAleLysAspIleThrSerAspThrSer
GlyAspPheArgAsnAlaLeuLeuSerLeuAlaLysGlyAspArgSerGluAsp
PheGlyValAsnGluAspLeuAlaAspSerAspAlaArgAlaLeuTyrGluAla
GlyGluArgArgLysGlyThrAspValAsnValPheAsnThrIleLeuThrThr
```

c)

```
GlyGlyProGlySerAlaValSerProTyrProThrPheAsnProSerSerAsp
ValAlaAlaLeuHisLysAlaIleMetValLysGlyValAspGluAlaThrIle
```

66

IleAspIleleuThrLysArgAsnAsnAlaGlnArgGlnGlnIleLysAlaAla
TyrLeuGlnGluThrGlyLysProLeuAspGluThrLeuLysLysAlaLeuThr
GlyHisLeuGluGluValValLeuAlaLeuLeuLysThrProAlaGlnPheAsp
AlaAspGluLeuArgAlaAlaMetLysGlyLeuGlyThrAspGluAspThrLeu
IleGluIleLeuAlaSerArgThrAsnLysGluIleArgAspIleAsnArgVal
TyrArgglugluLeuLysArgAspLeuAlaLysAspIleThrSerAspThrSer
GlyAspPheArgAsnAlaLeuLeuSerLeuAlaLysGlyAspArgSerGluAsp
PheGlyValAsnGluAspLeuAlaAspSerAspAlaLeuTyrGluAlaglyglu


d)


ValSerGluPheLeuLysGlnAlaTrhPheIleGluAsnGluGluGlnGluTyr
ValGlnThrValLysSerSerLysGlyGlyProGlySerAlaValSerProTyr
ProThrPheAsnProSerSerAspValAlaAlaLeuHisLysAlaIleLeuVal
LysGlyValAspGluAlaThrIleIleAspIleLeuThrLysArgAsnasnAla
GlnArgGlnGlnIleLysAlaAlaTyrLeuGlnGluThrGlyLysProLeuAsp
GluThrLeuLysLysAlaLeuThrGlyHisLeuGluGluValValLeuAlaLeu
LeuLysThrProAlaGlnPheAspAlaAspGluLeuArgAlaAlaLeuLysGly
LeuGlyThrAspGluAspThrLeuIleGluIleLeuAlaSerArgThrAsnLys
GluIleArfAspIleAsnArgValTyrArgGluGluLeuLysArgAspLeuAla
LysAspIleThrSerAspThrSerGlyAspPheArgAsnAlaLeuLeuSerLeu
AlaLysGlyAspArgSerGluAspPheClyValAsnGluAspLeuAlaAspSer
AspAlaArgAlaLeuTyrGluAlaGlyGluArgArgLysGlyThrAspValAsn
ValPheAsnThrIleLeuThrThrArgSerTyrProGlnLeuArgArgValPhe
GlnLysTyrThrLysTyrSerLYSHisAsp ;


e)


ValSerGluPheLeuLysGlnAlaTrpPheIleGluAsnGluGluGlnGluTyr
ValGlnThrValLysSerSerLysGlyGlyProGlySerAlaValSerProTyr
ProThrPheAsnProSerSerAspValAlaAlaLeuHisLysAlaIleLeuVal
LysGlyValAspGluAlaThrIleIleAspIleLeuthrLysArgAsnAsnAla
GlnArgGlnGlnIleLysAlaAlaTyrLeuGlnGluThrGlyLysProLeuAsp
GluThrLeuLysLysAlaLeuThrGlyHisLeuGluGluValVal ;


et
f)


ValSerGluPheLeuLysGlnAlaTrpPheIleGluAsnGluGluGlnGluTyr
ValGlnThrValLysSerSerLysGlyGlyProGlySerAlaValSerProTyr
ProTyrPheAsnProSerSerAspValAlaAlaLeuHisLysAlaIleLeuVal

```
LysGlyValAspGluAlaThrIleIleAspIleLeuThrLysArgAsnAsnAla
GlnArgGlnGlnIleLusAlaAlaTyrLeuGlnGluThrGlyLysProLeuAsp
GluThrLeuLysLysAlaLeuThrGlyHisLeuGluGluValValLeuAlaLeu
LeuLysThrProAlaGlnPheAspAlaAspGluLeuArgAlaAlaLeuLysGly
LeuGlyThrAspGluAspThrLeuIleGluIleLeuAlaSerArgThrAsnLys
GluIleArgAspIleAsnArgValTyrArgGluGluLeuLysArgAspLeuAla
LysAspIleThrSerAspThrSerGlyAspPheArgAsnAlaLeuLeuSerLeu
AlaLysGlyAspArgSerGluAspPheGlyValAsnGluAsp.
```

16. Le polypeptide selon la revendication 12, caractérisé en ce que le polypeptide a la séquence d'amino-acide :

```
GlyGlyProGlySerAlaValSerProTyrProThrPheAsnProSerSerAsp
ValAlaAlaLeuHisLysAlaIleMetValLysGlyValAspGluAlaThrIle
IleAspIleLeuThrLysArgAsnAsnAlaGlnArgGlnGlnIleLysAlaAla
tyrLeuGlnGluThrGlyLysProLeuAspGluThrLeuLysLysAlaLeuThr
GlyHisLeuGluGluValValLeuAlaLeuLeuLysThrProAlaGlnPheAsp
AlaAspGluLeuArgAlaAlaMetLysGlyLeuGlyThrAspGluAspThrLeu
IleGluIleLeuAlaSerArgThrAsnLysGluIleArgAspIleAsnArgVal
TyrArgGluGluLeuLysArgAspLeuAlaLysAspIleThrSerAspThrSer
GlyAspPheArgAsnAlaLeuLeuSerLeuAlaLysGlyAspArgSerGluAsp
PheGlyValAsnGluAspLeuAlaAspSerAspAlaArgAlaLeuTyrGluAla
GlyGluArgArgLysGlyThrAspValAsnValPheAsnThrIleLeuThrThr
ArgSerTyrProGlnLeuArgArgValPheGlnLysTyrThrLysTyrSerLys
HisAspMetAsnLysValLeuAspLeuGluLeuLysGlyAspIleGluLysCys
LeuTrgAlaIleValLysCysAlaThrSerLysProAlaPhePheAlaGluLys
LeuHisGlnAlaMetLysGlyValGlyThrArgHisLysAlaLeuIleArgIle
MetValSerArgSerGluIleAspMetAsnAspIleLysAlaPheTyrGlnLys
MetTyrGlyIleSerLeuCysGlnAlaIleLeuAspGluThrLysGlyAspTyr
GlyLysIleLeuValAlaLeuCysGlyGlyAsn.
```

17. Une séquence d'ADN consistant en :

```
ATGGCAATGGTATCAGAATTCCTCAAGCAGGCCTGGTTTATTGAAAATGAAGAGC
AGGAATATGTTCAAACTGTGAAGTCATCCAAAGGTGGTCCCGGATCAGCGGTGAG
```

```
CCCCTATCCTACCTTCAATCCATCCTCGGATGTCGCTGCCTTGCATAAGGCCATA

ATGGTTAAAGGTGTGGATGAAGCAACCATCATTCACATTCTAACTAACCCAAACA

ATGCACACCCTCAACACATCAAAGCAGCATATCTCCAGGAAACAGGAAAGCCCCT

CGATGAAACACTTAAGAAAGCCCTTACAGGTCACCTTGAGGAGGTTGTTTTAGCT

CTGCTAAAAACTCCAGCGCAATTTGATGCTGATGAACTTCGTGCTGCCATGAAGG

GCCTTGGAACTGATGAAGATACTCTAATTGAGATTTTGGCATCAAGAACTAACAA

AGAAATCAGAGACATTAACAGGGTCTACAGAGAGGAACTGAAGAGAGATCTGGCC

AAAGACATAACCTCAGACACATCTGGAGATTTTCGGAACGCTTTGCTTTCTCTTG

CTAAGGGTGACCGATCTGAGGACTTTGGTGTGAATGAAGACTTGGCTGATTCAGA

TGCCAGGGCCTTGTATGAAGCAGGAGAAAGGAGAAAGGGGACAGACGTAAACGTG

TTCAATACCATCCTTACCACCAGAAGCTATCCACAACTTCGCAGAGTGTTTCAGA

AATACACCAAGTACAGTAAGCATGACATGAACAAAGTTCTGGACCTGGAGTTGAA

AGGTGACATTGAGAAATGCCTCACAGCTATCGTGAAGTGCGCCACAAGCAAACCA

GCTTTCTTTGCAGAGAAGCTTCATCAAGCCATGAAAGGTGTTGGAACTCGCCATA

AGGCATTGATCAGGATTATGGTTTCCCGTTCTGAAATTGACATGAATGATATCAA

AGCATTCTATCAGAAGATGTATGGTATCTCCCTTTGCCAAGCCATCCTGGATGAA

ACCAAGGATTATGAGAAAATCCTGGTGGCTCTTTGTGGAGGAAACTAA.
```

**18.** Une séquence d'ADN qui consiste en :

```
GAATTCAAGAGAAAGTACCCCAAGTCCCTGTACTATTATATCCAGCAAGACACTA

AGGGCGACTACCAGAAAGCGCTGCTGTACCTGTCTGGTGGAGATGACTGA.
```

**19.** Un procédé de production d'un polypeptide humain qui a une activité inhibitrice sur la phospholipase A2 et l'activité immunologique d'une lipocortine humaine qui comprend les étapes de mise en culture d'un hôte transformé par une molécule d'ADN recombinant selon la revendication 5 et de recueil du polypeptide.

**20.** Une composition acceptable pharmaceutiquement, utile dans le traitement des maladies arthritiques, allergiques, dermatologiques, ophtalmiques et des maladies du collagène et d'autres désordres qui impliquent des processus inflammatoires, qui comprend une quantité pharmaceutiquement efficace d'au moins un polypeptide choisi dans le groupe qui consiste en des polypeptides selon les revendications 12, 14, 15 ou 16.

**21.** Une composition aceptable pharmaceutiquement utile dans le traitement des maladies arthritiques, allergiques, dermatologiques, optalmiques et des maladies du collagène ainsi que d'autres désordres impliquant des processus inflammatoires, qui comprend une quantité pharmaceutiquement efficace d'au moins un polypeptide selon la revendication 13.

**Revendications pour l'Etat contractant suivant : AT**

**1.** Un hôte unicellulaire transformé par au moins une molécule d'ADN recombinant caractérisé par une séquence d'ADN codant pour un polypeptide humain ayant une activité inhibitrice sur la phospholipase A2 et l'activité immunologique d'une lipocortine humaine et qui est choisie dans le groupe qui consiste en :

a) l'insert d'ADNc de ( λ LC(ATCC n° de dépôt 68812) limité aux deux extrémités par des sites de restriction <u>Eco RI</u>,

b) des séquences d'ADN qui hybrident pour l'insert d'ADN de la revendication la et qui encodent un polypeptide humain ayant une activité inhibitrice sur la phospholipase A2 et une activité immunologique de la lipocortine humaine encodée par l'insert d'ADNc de la partie a) ci-dessus et,

c) des séquences d'ADN qui sont dégénérées par suite du code génétique pour l'insert d'ADN et les séquences de a) et de b) ci-dessus,

caractérisé en ce que cette séquence d'ADN est reliée fonctionnellement à une séquence de contrôle de l'expression dans la molécule.

2. L'hôte transformé selon la revendication 1, caractérisé en ce que la séquence d'ADN est formé de :

```
ATGGCAATGGTATCAGAATTCCTCAAGCAGGCCTGGTTTATTGAAAATGAAGAGC

AGGAATATGTTCAAACTGTGAAGTCATCCAAAGGTGGTCCCGGATCAGCGGTGAG

CCCCTATCCTACCTTCAATCCATCCTCGGATGTCGCTGCCTTGCATAAGGCCATA

ATGGTTAAAGGTGTGGATGAAGCAACCATCATTCACATTCTAACTAACCCAAACA

ATGCACACCCTCAACACATCAAAGCAGCATATCTCCAGGAAACAGGAAAGCCCCT

CGATGAAACACTTAAGAAAGCCCTTACAGGTCACCTTGAGGAGGTTGTTTTAGCT

CTGCTAAAAACTCCAGCGCAATTTGATGCTGATGAACTTCGTGCTGCCATGAAGG
```

```
GCCTTGGAACTGATGAAGATACTCTAATTGAGATTTTGGCATCAAGAACTAACAA

AGAAATCAGAGACATTAACAGGGTCTACAGAGAGGAACTGAAGAGAGATCTGGCC

AAAGACATAACCTCAGACACATCTGGAGATTTTCGGAACGCTTTGCTTTCTCTTG

CTAAGGGTGACCGATCTGAGGACTTTGGTGTGAATGAAGACTTGGCTGATTCAGA

TGCCAGGGCCTTGTATGAAGCAGGAGAAAGGAGAAAGGGGACAGACGTAAACGTG

TTCAATACCATCCTTACCACCAGAAGCTATCCACAACTTCGCAGAGTGTTTCAGA

AATACACCAAGTACAGTAAGCATGACATGAACAAAGTTCTGGACCTGGAGTTGAA

AGGTGACATTGAGAAATGCCTCACAGCTATCGTGAAGTGCGCCACAAGCAAACCA

GCTTTCTTTGCAGAGAAGCTTCATCAAGCCATGAAAGGTGTTGGAACTCGCCATA

AGGCATTGATCAGGATTATGGTTTCCCGTTCTGAAATTGACATGAATGATATCAA

AGCATTCTATCAGAAGATGTATGGTATCTCCCTTTGCCAAGCCATCCTGGATGAA

ACCAAGGATTATGAGAAAATCCTGGTGGCTCTTTGTGGAGGAAACTAA.
```

3. Un hôte unicellulaire transformé par au moins un molécule d'ADN recombinant choisie dans le groupe formé de :

a) l'insert d'ADNc de pNLip1 (ATCC n° de dépôt 68811) limitée aux extrémités par des sites de restriction Eco RI

b) des séquences d'ADN qui hybrident pour l'insert d'ADN de la revendication 3a) et qui encodent un polypeptide humain ayant une activité inhibitrice de la phospholipase A2 et une activité immunologique de la lipocortine humaine encodée par l'insert d'ADNc de la partie a) ci-dessus, et

c) des séquences d'ADN qui sont dégénérées par suite du code génétique pour l'insert d'ADN et des séquences de a) et de b) ci-dessus,

caractérisé en ce que ces séquences d'ADN sont reliées fonctionnellement à une séquence de contrôle de l'expression dans la molécule.

4. Un hôte unicellulaire transformé par au moins une molécule d'ADN recombinant, caractérisé en ce que la séquence :

GAATTCAAGAGAAAGTACGGCAAGTCCCTGTACTATTATATCCAGCAAGACACTA

AGGGCGACTACCAGAAAGCGCTGCTGTACCTGTGTGGTGGAGATGACTGA

caractérisé en ce que cette séquence d'ADN est reliée fonctionnellement à une séquence de contrôle de l'expression dans la molécule.

5. L'hôte transformé selon l'une des revendications 1 à 4, caractérisé en ce que la molécule d'ADN recombinant est choisie dans le groupe formé de pLipPLT4A, pLipPLT4T et pSVL9109.

6. Un hôte unicellulaire transformé par au moins une molécule d'ADN recombinant pBG120 (ATCC n° de dépôt 74106) caractérisé en ce que cette séquence d'ADN est reliée fonctionnellement à une séquence de contrôle de l'expression dans la molécule

7. Un hôte unicellulaire transformé par au moins une molécule d'ADN recombinant pNLip1 (ATCC n° de dépôt 68811), caractérisé en ce que ceette séquence d'ADN est reliée fonctionnellement à une séquence de contrôle de l'expression dans la molécule.

8. Un hôte unicellulaire tranformé par au moins une molécule d'ADN recombinant formée de :

ATGGCAATGGTATCAGAATTCCTCAAGCAGGCCTGGTTTATTGAAAATGAAGAGC

AGGAATATGTTCAAACTGTGAAGTCATCCAAAGGTGGTCCCGGATCAGCGGTGAG

CCCCTATCCTACCTTCAATCCATCCTCGGATGTCGCTGCCTTGCATAAGGCCATA

ATGGTTAAAGGTGTGGATGAAGCAACCATCATTGACATTCTAACTAAGCGAAACA

ATGCACAGCGTCAACAGATCAAAGCAGCATATCTCCAGGAAACAGGAAAGCCCCT

GGATGAAACACTTAAGAAAGCCCTTACAGGTCACCTTGAGGAGGTTGTTTTAGCT

CTGCTAAAAACTCCAGCGCAATTTGATGCTGATGAACTTCGTGCTGCCATGAAGG

GCCTTGGAACTGATGAAGATACTCTAATTGAGATTTTGGCATCAAGAACTAACAA

AGAAATCAGAGACATTAACAGGGTCTACAGAGAGGAACTGAAGAGAGATCTGGCC

AAAGACATAACCTCAGACACATCTGGAGATTTTCGGAACGCTTTGCTTTCTCTTG

CTAAGGGTGACCGATCTGAGGACTTTGGGTGAATGAAGACCTTGGCTGATTCAGA

TGCCAGGGCCTTGTATGAAGCAGGAGAAAGGAGAAAGGGGACAGACGTAAACGTG

TTCAATACCATCCTTACCACCAGAAGCTATCCACAACTTCGCAGAGTGTTTCAGA

AATACACCAAGTACAGTAAGCATGACATGAACAAAGTTCTGGACCTGGAGTTGAA

AGGTGACATTGAGAAATGCCTCACAGCTATCGTGAAGTGCGCCACAAGCAAACCA

GCTTTCTTTGCAGAGAAGCTTCATCAAGCCATGAAAGGTGTTGGAACTCGCCATA

AGGCATTGATCAGGATTATGGTTTCCCGTTCTGAAATTGACATGAATGATATCAA

AGCATTCTATCAGAAGATGTATGGTATCTCCCTTTGCCAAGCCATCCTGGATGAA

ACCAAAGGAGATTATGAGAAAATCCTGGTGGCTCTTTGTGGAGGAAACTAA

caractérisé en ce que cette séquence d'ADN est reliée fonctionnellement à une séquence de contrôle de l'expression dans la molécule.

9. Un hôte unicellulaire transformé par au moins une molécule d'ADN recombinant formée de :

```
CAATTCAAGAGAAAGTACGGCAACTCCCTCTACTATTATATCCAGCAACACACTA
AGGGCGACTACCACAAAGCCCTGCTGTACCTGTGTGGTGGAGATGACTGA
```

caractérisé en ce que cette séquence d'ADN est reliée fonctionnellement à une séquence de contrôle de l'expression dans la molécule.

10. L'hôte transformé selon l'une des revendications 1 à 9, caractérisé en ce que cette séquence de contrôle de l'expression est choisie dans le groupe formé du système lac, du système de la β-lactamase, du système trp, du sytème tac, du système trc, de l'opérateur principal, et des régions du promoteur du phage (λ), la région de contrôle de la protéine de couverture fd, le promoteur pour la 3-phosphoglycérate kinase ou d'autres enzymes glycolytiques, les promoteurs des phosphates acides, les promoteurs des facteurs de couplage en (α) de la levure, les promoteurs pour les cellules de mammifères, les promoteurs précoces et tardifs SV40, le promoteur tardif d'adenovirus et le promoteur de la métallothionine et d'autres séquences qui contrôlent l'expression des gènes des cellules procaryotes ou eucaryotes ainsi que leurs virus et les combinaisons de ceux-ci.

11. Un procédé de production d'un polypeptide humain ayant l'activité inhibitrice sur la phospholipase A2 et l'activité immunologique d'une lipocortine humaine, qui comprend les étapes de mise en culture d'un hôte transformé selon les revendications 1 à 10 et de recueil dudit polypeptide.

12. Le procédé selon la revendication 11 d'obtention d'un polypeptide humain qui a une activité inhibitrice sur la phospholipase A2 et l'activité immunologique d'une lipocortine humaine, ce polypeptide étant choisi dans le groupe formé de :

a)

MetAlaMetValSerGluPheLeuLysGlnAlaTrpPheIleGluAsnGluGlu
GlnGluTyrValGlnThrValLysSerSerLysGlyGlyProGlySerAlaVal
SerProTyrProThrPheAsnProSerSerAspValAlaAlaLeuHisLysAla
IleMetValLysGlyValAspGluAlaThrIleIleAspIleLeuThrLysArg
AsnAsnAlaGlnArgGlnGlnIleLysAlaAlaTyrLeuGlnGluThrGlyLys
ProLeuAspGluThrLeuLysLysAlaLeuThrGlyHisLeuGluGluValVal
LeuAlaLeuLeuLysThrProAlaGlnPheAspAlaAspGluLeuArgAlaAla
MetLysGlyLeuGlyThrAspGluAspThrLeuIleGluIleLeuAlaSerArg
ThrAsnLysGluIleArgAspIleAsnArgValTyrArgGluGluLeuLysArg
AlaAspSerAspAlaArgAlaLeuTyrGluAlaGlyGluArgArgLysGlyThr
AspValAsnValPheAsnThrIleLeuThrThrArgSerTyrProGlnLeuArg
ArgValPheGlnLysTyrThrLysTyrSerLysHisAspMetAsnLysValLeu
AspLeuGluLeuLysGlyAspIleGlyLysCysLeuThrAlaIleValLysCys
AlaThrSerLysProAlaPhePheAlaGluLysLeuHisGlnAlaMetLysGly
ValGlyThrArgHisLysAlaLeuIleArgIleMetValSerArgSerGluIle
AspMetAsnAspIleLysAlaPheTyrGlnLysMetTyrGlyIleSerLeuCys


GlnAlaIleLeuAspGluThrLysGlyAspTyrGluLysIleLeuValAlaLeu
CysGlyGlyAsn ;

73

b)

```
AlaMetValSerGluPheLeuLysGlnAlaTrpPheIleGluAsnGluGluGln
GluTyrValGlnThrValLysSerSerLysGlyGlyProGlySerAlaValSer
ProTyrProThrPheAsnProSerSerAspValAlaAlaLeuHisLysAlaIle
MetValLysGlyValAspGluAlaThrIleIleAspIleLeuThrLysArgAsn
AsnAlaGlnArgGlnGlnIleLysAlaAlaTyrLeuGlnGluThrGlyLysPro
LeuAspGluThrLeuLysLysAlaLeuThrGlyHisLeuGluGluValValLeu
AlaLeuLeuLysThrProAlaGlnPheAspAlaAspGluLeuArgAlaAlaMet
LysGlyLeuGlyThrAspGluAspThrLeuIleGluIleLeuAlaSerArgThr
AsnLysGluIleArgAspIleAsnArgValTyrArgGlugluLeuLysArgAsp
LeuAlaLysAspIleThrSerAspThrSerGlyAspPheArgAsnAlaLeuLeu
SerLeuAlaLysGlyAspArgSerGluAspPheGlyValAsnGluAspLeuAla
AspSerAspAlaArgAlaLeuTyrGluAlaGlyGluArgArgLysGlyThrAsp
ValAsnValPheAsnThrIleLeuThrThrArgSerTyrProGlnLeuArgArg
ValPheGlnLysTyrThrLysTyrSerLysHisAspMetAsnLysValLeuAsp
LeuGluLeuLysGlyAspIleGluLysCysLeuThrAlaIleValLysCysAla
ThrSerLysProAlaPhePheAlaGluLysLeuHisGlnAlaMetLysGlyVal
GlyThrArgHisLysAlaLeuIleArgIleMetValSerArgSerGluIleAsp
MetAsnAspIleLysAlaPheTyrGlnLysMetTyrGlyIleSerLeuCysGln
AlaIleLeuAspGluThrLysGlyAspTyrGluLysIleLeuValAlaLeuCys
GlyclyAsn,
```

et

c) des fragments de polypeptide du polypeptide de la partie a) ou b) ci-dessus qui manifeste une activité biologique ou immunologique de la lipocortine définie en a) ou b) ci-dessus.

**13.** Le procédé selon la revendication 11 de production d'un polypeptide de N-lipocortine, caractérisé par les propriétés suivantes :

a) il a l'aptitude à inhiber l'enzyme phospholipase A2

b) il comprend les fragments tryptiques :

(1) LeuTyrAspSerMetLys ;

(2) LeuSerLeuAsnGlyAspThrSer ThrProProserAlaTyrGly ;

(3) SerLeuTyrTyrTyrIleGin GlnAspThrLys ;

(4) TrpIleSerIleketThrGluArg ;

(5) SerTyrSerProTyrAspMet LeuGluSerIle ;

(6) LeuLeuValValTyrProX ThrGlnIleLeu ;

dans lesquels X est tout amino-acide et

c) qui a une extrémité portant un carboxyle consistant en la séquence d'amino-acides :

```
LysArgLysTyrGlyLysSerLeuTyrTyrTyrIleGlnGlnAspThrLysGly
AspTyrGlnLysAlaLeuLeuTyrLeuCysGlyGlyAspAsp.
```

**14.** Le procéde selon la revendication 11 d'obtention d'un polypeptide du type de la lipocortine humaine encodé par les molécules d'ADN recombinant de la revendication 1 ou de la revendication 2.

**15.** Le procédé selon la revendication 11 d'obtention d'un polypeptide dans lequel le polypeptide est choisi dans le groupe forme de :

a)

GlyClyProClySerAlaValSorProTyrProThrPheAsnProSerSerAsp
ValAlaAlaLeuHieLysAlaIleMetValLysClyValAspCluAlaThrIle
IleAspIleLeuThrLysArgAsnAsnAlaGlnArgGlnGlnIleLysAlaAla
TyrLeuGlnGluThrGlyLysProLeuAspGluThrLeuLysLysAlaLeuThr
GlyHisLeuGluGluValValLeuAlaLeuLeuLysThrProAlaGlnPheAsp
AlaAspGluLeuArgAlaAlaMetLysGlyLeuGlyThrAspGluAspThrLeu
ileGluIleLeuAlaSerArgThrAsnLysGluIleArgAspIleAsnArgVal
TyrargGluGluLeuLysArgAspLeuAlaLysAspIleThrSerAspThrSer
GlyAspPheArgAsnAlaLeuLeuSerLeuAlaLysGlyAspArgSerGluAsp
PheGlyValAsnGluAspLeuAlaAspSerAspAlaArgAlaLeutyrGluAla
GlyGluArgArgLysGlyThrAspValAsnValPheAsnThrIleLeuThrThr
ArgSerTyrProGlnLeuArgArgValPheGlnLysTyrThrLysTyrSerLys
HisAspMetAsnLysValLeuAspLeuGluLeuLysGlyAspIleGluLysCys
LeuThrAlaIleValLysCysAlaThrSerLysProAlaPhePheAlaGluGly
LeuHisGlnAlaMetLysGlyValGlyThrArgHisLysAlaLeuIleArgIle


MetValSerArgSerGluIleAspMetAsnAspIleLysAlaPheTyrGlnLys
MetTyrGlyIleSerLeuCysGlnAlsIleLeuAspGluThrLysGlyAspTyr
GluLysIleLeuValAlaLeuCysGlyGly ;

(b)


GlyGlyProGlySerAlaValSerProTyrProThrPheAsnProSerSerAsp
ValAlaAlaLeuHisLysAlaIleMetValLysGlyValAspGluAlaThrIle
IleAspIleLeuThrLysArgAsnAsnAlaGlnArgGlnGlnIleLysAlaAla
TyrLeuGlnGluThrGlyLysProLeuAspGluThrLeuLysLysAlaLeuThr
GlyHisLeuGluGluValVAlLeuAlaLeuLeuLysThrProAlaGlnPheAsp
AlaAspGluLeuArgAlaAlaMetLysGlyLeuGlyThrAspGluAspThrLeu
IleGluIleLeuAlaSerArgThrAsnLysGluIleArgAspIleAsnArgVal
TyrArgGluGluLeuLysArgAspLeuAleLysAspIleThrSerAspThrSer
GlyAspPheArgAsnAlaLeuLeuSerLeuAlaLysGlyAspArgSerGluAsp
PheGlyValAsnGluAspLeuAlaAspSerAspAlaArgAlaLeuTyrGluAla
GlyGluArgArgLysGlyThrAspValAsnValPheAsnThrIleLeuThrThr ;

c)

GlyGlyProClySerAlaValSerProTyrProThrPheAsnProSerSerAsp
ValAlaAlaLeuHisLysAlaIleMetValLysGlyValAspGluAlaThrIle
IleAspIleleuThrLysArgAsnAsnAlaGlnArgGlnGlnIleLysAlaAla
TyrLeuGlnGluThrGlyLysProLeuAspGluThrLeuLysLysAlaLeuThr
GlyHisLeuGluGluValValLeuAlaLeuLeuLysThrProAlaGlnPheAsp
AlaAspGluLeuArgAlaAlaMetLysGlyLeuGlyThrAspGluAspThrLeu
IleGluIleLeuAlaSerArgThrAsnLysGluIleArgAspIleAsnArgVal
TyrArgglugluLeuLysArgAspLeuAlaLysAspIleThrSerAspThrSer
GlyAspPheArgAsnAlaLeuLeuSerLeuAlaLysGlyAspArgSerGluAsp
PheGlyValAsnGluAspLeuAlaAspSerAspAlaLeuTyrGluAlaglyglu

d)

ValSerGluPheLeuLysGlnAlaTrhPheIleGluAsnGluGluGlnGluTyr
ValGlnThrValLysSerSerLysGlyGlyProGlySerAlaValSerProTyr
ProThrPheAsnProSerSerAspValAlaAlaLeuHisLysAlaIleLeuVal
LysGlyValAspGluAlaThrIleIleAspIleLeuThrLysArgAsnasnAla
GlnArgGlnGlnIleLysAlaAlaTyrLeuGlnGluThrGlyLysProLeuAsp
GluThrLeuLysLysAlaLeuThrGlyHisLeuGluGluValValLeuAlaLeu
LeuLysThrProAlaGlnPheAspAlaAspGluLeuArgAlaAlaLeuLysGly

LeuGlyThrAspGluAspThrLeuIleGluIleLeuAlaSerArgThrAsnLys
GluIleArfAspIleAsnArgValTyrArgGluGluLeuLysArgAspLeuAla
LysAspIleThrSerAspThrSerGlyAspPheArgAsnAlaLeuLeuSerLeu
AlaLysGlyAspArgSerGluAspPheClyValAsnGluAspLeuAlaAspSer
AspAlaArgAlaLeuTyrGluAlaGlyGluArgArgLysGlyThrAspValAsn
ValPheAsnThrIleLeuThrThrArgSerTyrProGlnLeuArgArgValPhe
GlnLysTyrThrLysTyrSerLYSHisAsp ;

e)

ValSerGluPheLeuLysGlnAlaTrpPheIleGluAsnGluGluGlnGluTyr
ValGlnThrValLysSerSerLysGlyGlyProGlySerAlaValSerProTyr
ProThrPheAsnProSerSerAspValAlaAlaLeuHisLysAlaIleLeuVal
LysGlyValAspGluAlaThrIleIleAspIleLeuthrLysArgAsnAsnAla
GlnArgGlnGlnIleLysAlaAlaTyrLeuGlnGluThrGlyLysProLeuAsp
GluThrLeuLysLysAlaLeuThrGlyHisLeuGluGluValVal ;

et
f)

```
ValSerGluPheLeuLysGlnAlaTrpPheIleGluAsnGluGluGlnGluTyr
ValGlnThrValLysSerSerLysGlyGlyProGlySerAlaValSerProTyr
ProTyrPheAsnProSerSerAspValAlaAlaLeuHisLysAlaIleLeuVal
LysGlyValAspGluAlaThrIleIleAspIleLeuThrLysArgAsnAsnAla
GlnArgGlnGlnIleLusAlaAlaTyrLeuGlnGluThrGlyLysProLeuAsp
GluThrLeuLysLysAlaLeuThrGlyHisLeuGluGluValValLeuAlaLeu
LeuLysThrProAlaGlnPheAspAlaAspGluLeuArgAlaAlaLeuLysGly
LeuGlyThrAspGluAspThrLeuIleGluIleLeuAlaSerArgThrAsnLys
GluIleArgAspIleAsnArgValTyrArgGluGluLeuLysArgAspLeuAla
LysAspIleThrSerAspThrSerGlyAspPheArgAsnAlaLeuLeuSerLeu
AlaLysGlyAspArgSerGluAspPheGlyValAsnGluAsp.
```

16. Le procédé selon la revendication 11 de production d'un polypeptide,caractérisé en ce que le polypeptide a la séquence d'amino-acides :

```
GlyGlyProGlySerAlaValSerProTyrProThrPheAsnProSerSerAsp
ValAlaAlaLeuHisLysAlaIleMetValLysGlyValAspGluAlaThrIle
IleAspIleLeuThrLysArgAsnAsnAlaGlnArgGlnGlnIleLysAlaAla
```

```
tyrLeuGlnGluThrGlyLysProLeuAspGluThrLeuLysLysAlaLeuThr
GlyHisLeuGluGluValValLeuAlaLeuLeuLysThrProAlaGlnPheAsp
AlaAspGluLeuArgAlaAlaMetLysGlyLeuGlyThrAspGluAspThrLeu
IleGluIleLeuAlaSerArgThrAsnLysGluIleArgAspIleAsnArgVal
TyrArgGluGluLeuLysArgAspLeuAlaLysAspIleThrSerAspThrSer
GlyAspPheArgAsnAlaLeuLeuSerLeuAlaLysGlyAspArgSerGluAsp
PheGlyValAsnGluAspLeuAlaAspSerAspAlaArgAlaLeuTyrGluAla
GlyGluArgArgLysGlyThrAspValAsnValPheAsnThrIleLeuThrThr
ArgSerTyrProGlnLeuArgArgValPheGlnLysTyrThrLysTyrSerLys
HisAspMetAsnLysValLeuAspLeuGluLeuLysGlyAspIleGluLysCys
LeuTrgAlaIleValLysCysAlaThrSerLysProAlaPhePheAlaGluLys
LeuHisGlnAlaMetLysGlyValGlyThrArgHisLysAlaLeuIleArgIle
MetValSerArgSerGluIleAspMetAsnAspIleLysAlaPheTyrGlnLys
MetTyrGlyIleSerLeuCysGlnAlaIleLeuAspGluThrLysGlyAspTyr
GlyLysIleLeuValAlaLeuCysGlyGlyAsn.
```

**17.** L'hôte transformé selon l'une des revendications 1 à 10 choisi dans le groupe qui consiste en des souches d'E. Coli, de Pseudomonas, de Bacillus, des streptomyces, de cellules de levure, d'autres champignons, de cellules de souris ou d'autres hôtes de cellule animales, des hôtes de cellules végétales, et des cellules de tissus humains.

FIG.1

CNBr 1: (less than 2Kd based on amino acid analysis)

- Met - lys - gly - ala - gly - thr - arg - arg - lys - thr -
  1     2     3     4     5     6     7     8     9     10

  leu - ile
  11    12

CNBr 2: (less than 2Kd based on amino acid analysis)

- met - leu - lys - thr - pro - ala - gln - phe - asp - ala -
  1     2     3     4     5     6     7     8     9     10

  asp - glu - leu - ile(?) - arg(?)
  11    12    13    14        15

CNBr 3: (7Kd based on SDS-polyacrylamide gel electrophoresis)

- met - $\frac{tyr}{lys}$ - $\frac{val}{ala}$ - asn - gln - asp - $\frac{leu}{trp}$ - ala - $\frac{ala}{gln}$ -
  1         2              3          4     5     6        7          8        9

CNBr 4: (no protein detected by SDS-PAGE)

CNBr 5: (mixture of peptides, less than 5Kd based on SDS-PAGE)

CNBr 6: (mixture consisting of two 12Kd fragments based on SDS-PAGE)

# FIG.2

T17(a+b): - $\frac{ala}{tyr}$ - $\frac{leu}{phe}$ - $\frac{gln}{val}$ - $\frac{gln}{gln}$ - $\frac{ala}{lys}$ - $\frac{tyr}{gly}$ - gln - $\frac{arg}{gly}$

        1     2    3    4    5    6    7    8

T22(a): - ser - glu - ile - asp - met - asn - glu - ile - lys
        1    2    3    4    5    6    7    8    9

T22(b): - lys - val - phe - gln - asn - tyr - arg
        1    2    3    4    5    6    7

T23: - ser - tyr - pro - his (arg) - leu
        1    2    3    4    5

T24: - thr - pro - ala - gln - phe - asp - ala - asp - glu -
        1    2    3    4    5    6    7    8    9

        leu - leu - arg
        10    11    12

T29: - ala - ala - tyr - leu - gln - glu - thr - gly - lys -
        1    2    3    4    5    6    7    8    9

        pro - leu - asp - glu - thr - leu - lys
        10   11   12   13   14   15   16

T31: - gly - gly - pro - gly - ser - ala - val - ser - pro -
        1    2    3    4    5    6    7    8    9

        tyr - pro - ser(thr) - phe - asn - ser(thr) - ser
        10   11    12    13   14    15    16

        ser - thr - val - ala - trp - ala
        17   18   19   20   21   22

T35: - lys - gly - thr - asp - val - asn - val - phe (?) -
        1    2    3    4    5    6    7    8

        asn - thr - x - leu
        9    10   11   12

T38: - gly - leu - gly - thr - asp - glu -
        1    2    3    4    5    6

FIGURE 3

Sequence T22a:    GluIleAspMetAsnGluIle
    20-mer        GARATHGAYATGAAYGARAT

Sequence T22b:    LysValPheGlnAsnTyr
    17-mer        AARGTNTTYCARAAYTA

Sequence T24[1]:   AlaGlnPheAspAlaAspGlu
    20-mer        GCNCARTTYGAYGCNGAYGA

Sequence T29:     GlnGluThrGlyLysPro
    17-mer        CARGARACNGGNAARCC


[1]The sequence shown for T24 is also
contained within CNBr fragment 2.

Coding redundancies are:

N = C, T, A or G

R = A or G

Y = C or T

H = A, C or T

Figure 4

```
1    TTTCTTCTTTAGTTCTTTTGCAAGAAGGTAGAGATAAAGACACTTTTCAAAAATGGCAATGGTATCAGAATTCCTCAAGCAGGCCTGGTTTATTGAAAATG    100
     ----------------------------------------------------------------------------------------------------
     AAAGAGAAATCAAGAAACGTTCTTCCATCTCTATTTCTGTGAAAAAGTTTTTACCGTTACCATAGTCTTAAGGAGTTCGTCCGACCAAATAACTTTTAC
                                                        MetAlaMetValSerGluLysLeuLysGlnAlaTrpPheIleGluAsnG
                                                          1

101  AAGAGCAGGAATATGTTCAAACTGTGAAGTCATCCAAAGGTGGTCCCGGATCAGCGGTGAGCCCTCATCCATCCTCCGATGTCGCTGC                 200
     ----------------------------------------------------------------------------------------------------
     TTCTCGTCCTTATACAAGTTTGACACTTCAGTAGGTTCCACCAGGGCCTAGTCGCCACTCGGGAGTGAGGTAGGTAGGAGCCTACAGCGACG
     luGluGlnTyrValGlnThrValLysSerSerLysGlyGlyProGlySerAlaValSerProTyrProThrPheAsnProSerSerAspValAlaAl
                                 30

201  CTTGCATAAGGCCCATAATGGTTAAAGCTGTGGATGAAGCAACCATCATTGACATTCTAACTAAGGCGAAACAATGCACACAGGTCAACAGATCAAAGCAGCA   300
     ----------------------------------------------------------------------------------------------------
     GAACGTATTCCGGGTATTACCAATTTCCACACCTACTTCGTTGGTAGTAACTTCGTAAGATTGATTCGCTTGTTACGTGTCGCAGTTGTCTAGTTTCGTCGT
     aLeuHisLysAlaIleMetValLysGlyValAlaspGluAlaThrIleIleAspIleLeuThrLysAlaLysGlnIleIleLysAlaAla

301  TATCTCCAGGAAACAGGAAAGCCCCTGGATGAAACACTTAAGAAAGCCCTTACAGGTCACCTTGAGGAGGTTGTTTTTAGCTCTGCTAAAAACTCCAGCGC    400
     ----------------------------------------------------------------------------------------------------
     ATAGAGGTCCTTTGTCCTTTCGGGGACCTACTTTGTGAATTCTTTCGGGAATGTCCAGTGGAACTCCTCCAACAAAAATCGAGACGATTTTTGAGGTCGCG
     TyrLeuGlnGluThrGlyLysProLeuAspGluThrLeuLysLysAlaLeuThrGlyHisLeuGluGluValValLeuAlaLeuLeuLysThrProAlaG

401  AATTTGATGCTGATGAACTTCGTGTCGCCATCAAGGGGCCTTGGAACTGATGATGAAGATACTCTAATTGAGATTTTGGCATCAAGAACTAACAAAGAAATCAG   500
     ----------------------------------------------------------------------------------------------------
     TTAAACTACGACTACTTGAAGCAGCACGGTACTTCCCGGAACCTTGACTACTTCTATGAGATTAACTCTAAAACCGTAGTTCTTGATTGTTCTTTAGTC
     lnPheAspAlaAspGluLeuArgLeuArgAlaAlaMetLysGlyLeuTyrAspThrLeuIleGluIleLeuAlaSerArgThrAsnLysGluIleAr

501  AGACATTAACAGGGTCTCTACAGAGGACAACTGAAGAGAGATCTGGCCAAAGACACATAACCTCAGACACCTGCTTTCGGAACGCGCTTTGCTTTCTCTT    600
     ----------------------------------------------------------------------------------------------------
     TCTGTAATTGTCCCAGATGTCTCCTCTTGACTTCTCCTCTAGACCGGTTTCTGTGTATTGGAGTCGTCTGTGGTGATTATTGGAAGCCTTTGCGCGAAAGAGAA
     gAspIleAsnArgValTyrArgGluLeuLysArgGluAspPheArgSerGlySerAspThrSerAspPheArgAsnAlaLeuLeuSerLeu

601  GCTAAGGGTGACCGATCTGAGGACTTTGGTGTGTGAATGAAGACTTGGCTGATTCAGATTCAGATGCCAGGGCCTTGATTGAAGCAGGAAGGGACAG      700
     ----------------------------------------------------------------------------------------------------
     CGATTCCCACTGGCTAGACTCCTGAAACCACACACTTACTTCTGAACCGACTAAGTCTACGGTCCCGGAACATATTCGTCCTCTTTCCCCTGTC
     AlaLysGlyAspArgSerGluAspPheGlyValAspGluAspLeuAlaAspSerAspAlaArgAlaLeuIleGluAlaGlyArgGlyThrA
```

82

```
ACGTAAACGTGTTCAATACCATCCTTACCACCAGAAGCTATCCACAACTTCGCAGAGTGTTTCAGAAATACACCAAGTACAGTAAGCATGACATGAACAA
----------------------------------------------------------------------------------------------------
TGCATTTGCACAAGTTATGGTAGGAATGGTGGTCTTCGATAGGTGTTGAAGCGTCTCACAAAGTCTTTATGTGGTTCATGTCATTCGTACTGTACTTGTT
spValAsnValPheAsnThrIleLeuThrThrArgSerTyrProGlnLeuArgArgValPheGlnLysTyrThrLysTyrSerLysHisAspMetAsnLy

AGTTCTGGACCTGGAGTTGAAAGGTGACATTGAGAAATGCCTCACAGCTATCGTGAAGTGCGCCACAAGCAAACCAGCTTTCTTTGCAGAGAAGCTTCAT
----------------------------------------------------------------------------------------------------
TCAAGACCTGGACCTCAACTTTCCACTGTAACTCTTTACGGAGTGTCGATAGCACTTCACGCGGTGTTCGTTTGGTCGAAAGAAACGTCTCTTCGAAGTA
sValLeuAspLeuGluLeuLysGlyAspIleGluLysCysLeuThrAlaIleValLysCysAlaThrSerLysProAlaPhePheAlaGluLysLeuHis

CAAGCCATGAAAGGTGTTGGAACTCGCCATAAGGCATTGATCAGGATTATGGTTTCCCGTTCTGAAATTGACATGAATGATATCAAAGCATTCTATCAGA
----------------------------------------------------------------------------------------------------
GTTCGGTACTTTCCACAACCTTGAGCGGTATTCCGTAACTAGTCCTAATACCAAAGGGCAAGACTTTAACTGTACTTACTATAGTTTCGTAAGATAGTCT
GlnAlaMetLysGlyValGlyThrArgHisLysAlaLeuIleArgIleMetValSerArgSerGluIleAspMetAsnAspIleLysAlaPheTyrGlnL

AGATGTATGGTATCTCCCTTTGCCAAGCCATCCTGGATGAAACCAAAGGAGATTATGAGAAATCCTGGTGGCTCTTTGTGGAGGAAACTAAACATTCCC
----------------------------------------------------------------------------------------------------
TCTACATACCATAGAGGGAAACGGTTCGGTAGGACCTACTTTGGTTTCCTCTAATACTCTTTTAGGACCACCGAGAAACACCTCCTTTGATTTGTAAGGG
ysMetTyrGlyIleSerLeuCysGlnAlaIleLeuLeuAspGluThrLysGlyAspTyrGluLysIleLeuValAlaLeuCysGlyGlyAsnEnd
                                                                                         346
TTGATGGTCTCAAGCTATGATCAGAAGACTTTAATTATATATTTTCATCCTATAAGCTTAAATAGGAAAGTTTCTTCAACAGGATTACAGTGTAGCTACC
----------------------------------------------------------------------------------------------------
AACTACCAGAGTTCGATACTAGTCTTCTGAAATTAATATATAAAAGTAGGATATTCGAATTTATCCTTTCAAAGAAGTTGTCCTAATGTCACATCGATGG

TACATGCTGAAAAATATAGCCTTTAAATCATTTTTATATTATAACTCTGTATAATAGAGATAAGTCCATTTTTTAAAAATGTTTTCCCCAAACCATAAAA
----------------------------------------------------------------------------------------------------
ATGTACGACTTTTTATATCGGAAATTTAGTAAAAATATAATATTGAGACATATTATCTCTATTCAGGTAAAAAATTTTTACAAAAGGGGTTTGGTATTTT

CCCTATACAAGTTGTTCTAGTAACAATACATGAGAAAGATGTCTATGTAGCTGAAAATAAAATGACGTCACAAGAC
-----------------------------------------------------------------------------
GGGATATGTTCAACAAGATCATTGTTATGTACTCTTTCTACAGATACATCGACTTTTATTTTACTGCAGTGTTCTG
```

FIGURE 4 A

EP 0 209 568 B1

FIG.5

FIG. 6

FIG. 7

# FIG.8

# FIG.9

FIG.10

DIGESTION TIME, MIN.

FIG.14

# FIG. 11

# FIG.12

# FIG. 13

FIG.15

# FIG.16

# FIG.17

LIPO-S              LIPO-L

# FIG. 18

ELASTASE DIGEST

% OF INHIBITION

# FIG. 19

e-5          e-4          e-1

FIGURE 20

LIST OF OLIGONUCLEOTIDES

```
Lipo-60    CGTGCTGCCCTGAAAGGCCTTGG    Met 127→ Leu
Lipo-61    AAGGCAATATTGGTTAAAGGT      Met 56→   Leu
Lipo-68    GGAGGTTGTTATGGCTCTGCTA     Leu 109→ Met
Lipo-75    GATCTGGCCAAAGATATCATG      Thr 169→ Met by
Lipo-76    GATCCATGATATCTTTGGCCA               insertion
Lipo-77    GTCATCCACCATGGGTCCCGGA     Gly 30→   Met
Lipo-78    TCTGAATCGGCCATGTCTTCAT     Leu 198→ Met
Lipo-79    ATACCATCATGACCACCAG        Leu 225→ Met
```

1 3 [30] 56 [109] 127 [169] [198] [225] 248

Lipo 8    26Kd

Lipo 11    14.6Kd

Lipo 15    20.7Kd

Lipo 9    17.7Kd

FIG. 21

# FIG.22

UNCUT

26K

# F/G.23

FIG. 24

FIG. 25

```
        1       2       3       4       5       6
T9:   - Leu - Tyr - Asp - Ser - Met - Lys -


        1       2       3       4       5       6       7
T14:  - Ser - Glu - Ile - Asp - Met - Leu - Lys -
        Val   Leu   Val


        1       2       3       4       5       6       7       8       9       10      11
T19:  - Leu - Ser - Leu - Asn - Gly - Asp - Thr - Ser - Thr - Pro - Pro -

              12      13      14      15
              Ser - Ala - Tyr - Gly -


        1       2       3       4       5       6       7       8       9       10      11
T20:  - Ser - Leu - Tyr - Tyr - Tyr - Ile - Gln - Gln - Asp - Thr - Lys -


        1       2       3       4       5       6       7       8       9       10      11
T21:  - x  -  x  -  x  -  x  - Leu - Val - Lys - Gln - Tyr - Glu - Leu -
                                Gly   Ala   Gln   Asp
                                Ser   Lys   Val   Lys

              12      13      14      15
              Lys - Lys - Gln - Gln
                    Leu


        1       2       3       4       5       6       7       8       9       10      11
T22:  - Lys - Ala - Glu - Asp - Gly - Leu - Val - Ile - Asp - Tyr - Glu -
        Arg   Leu   Met   Val   Ala   Ser   Ala   Lys

              12      13      14      15
              Leu - Ile - Asp - Gln -


        1       2       3       4       5       6       7       8
T24:  - Trp - Ile - Ser - Ile - Met - Thr - Glu - Arg -


        1       2       3       4       5       6       7       8       9       10      11
T25:  - Ser - Tyr - Ser - Pro - Tyr - Asp - Met - Leu - Glu - Ser - Ile -


        1       2       3       4       5       6       7       8       9       10      11
T28:  - Leu - Leu - Val - Val - Tyr - Pro - x  - Thr - Gln - Ile - Leu -


        1       2       3       4       5       6       7       8       9       10      11
T29:  - Gly - Val - Gly - Glu - Ala - Thr - Ile - Ile - Asp - Ile - Leu -
        Ala   Leu   Asp   Thr   Asp   Glu   Asp   Gly   Leu   Ile   Glu
        Asp         Leu   Tyr   Leu         Gly   Ser         Leu

              12      13
              Thr - Lys -
              Ile
              Glu


        1       2       3       4       5       6       7       8       9
T32:  - Ala - Leu - Leu - Tyr - Leu - X  - Gly - Gly - Asp -
        Gly
```

103

## FIGURE 26

Sequence T20:   SerLeuTyrTyrTyrIleGlnGlnAspThrLys

    NLip1
    RTC YTG YTG GAT RTA RTA RTA YTT
    NLip2
    RTC YTG YTG AAT RTA RTA RTA YTT
    NLip3
    RTCYTG YTG TAT RTA RTA RTA YTT


Sequence T25:   SerTyrSerProTyrAspMetLeuGluSerIle

    NLip4
    YTC NAR CAT RTC RTC CGG
    NLip5
    YTC NAR CAT RTC RTA AGG
    NLip6
    YTC NAR CAT RTC RTA TGG
    NLip7
    YTC NAR CAT RTC RTA GGG


Sequence T24:   TrpIleSerIleMetThrGluArg

    NLip8
    TCA GTC ATX ATN PNX ATC CA
    NLip9
    TCG GTC ATX ATN PNX ATC CA
    NLip10
    TCC GTC ATX ATN PNX ATC CA
    NLip11
    TCT GTC ATX ATN PNX ATC CA


Sequence T9:   LeuTyrAspSerMetLys

    NLip12
    YTT CAT NPZ RTC RTA AA
    NLip13
    YTT CAT NPZ RTC RTA GA
    NLip14
    YTT CAT NPZ RTC RTA CA
    NLip15
    YTT CAT NPZ RTC RTA TA

FIGURE 27

cDNA insert of pNlipl

```
  1  GAATTCAAGA GAAAGTACGG CAAGTCCCTG TACTATTATA TCCAGCAAGA

 51  CACTAAGGGC GACTACCAGA AAGCGCTGCT GTACCTGTGT GGTGGAGATG

101  ACTGAAGCCC GACACGGCCT GAGCGTCCAG AAATGGTGCT CACCATGCTT

151  CCAGCTAACA GGTCTAGAAA ACCAGCTTGC GAATAACAGT CCCCGTGGCC

201  ATCCCTGTGA GGGTGACGTT AGCATTACCC CCAACCTCAT TTTAGTTGCC

251  TAAGCATTGC CTGGCCTTCC TGTCTAGTCT CTCCTGTAAG CCAAAGAAAT

301  GAACATTCCA AGGAGTTGGA AGTGAAGTCT ATGATGTGAA ACACTTTGCC

351  TCCTGTGTAC TGTGTCATAA ACAGATGAAT AAACTGAATT TGTACTTAA

     AAAAAAAAAA .......
```

Figure 28

```
N-lipocortin:    1 ..................................GAATTCAAGA   10
                                                         |   |||
lipocortin  :  951 GGTTTCCCGTTCTGAAATTGACATGAATGATATCAAAGCATTCTATCAGA 1000

              11 GAAAGTACGGCAAGTCCCTGTACTATTATATCCAGCAAGACACTAAGGGC   60
                 | ||||| || ||||||| |  |   | ||||  || || |||| ||||
            1001 AGATGTATGGTATCTCCCTTTGCCAAGCCATCCTGGATGAAACCAAAGGA 1050

              61 GACTACCAGAAAGCGCTGCTGTACCTGTGTGGTGGAGATGACTGAAGCCC  110
                 || ||   || || ||  |||| |||||| |||||| |||||| || |||
            1051 GATTATGAGAAAATCCTGGTGGCTCTTTGTGGAGGAAACTAAACATTCCC 1100

             111 GACACGGCCTGA....GCGTCCAGAAATGGTGCTCACCATGCTTCCAGCT  156
                 ||  |  | |     ||  ||| |||||  ||| |  | || ||  | ||
            1101 TTGATGGTCTCAAGCTATGATCAGAAGACTTTAATTATATATTTTCATCC 1150

             157 AACAGGTCTAGAAAACCAGCTTGCGAATAACAG...TCCCCGTGGCCATC  203
                 | || || ||| ||  || ||| |  ||  ||   | || ||||| || |
            1151 TATAAGCTTAAATAGGAAAGTTTCTTCAACAGGATTACAGTGTAGCTACC 1200

             204 CCTGTGAGGGTGACGTTAGCATTACCCCCAACCTCATTTTAGTTGCCTAA  253
                 | ||| ||   ||  |||||||| |||  | | |||||  ||  |   |
            1201 TACATGCTGAAAAATATAGCCTTTAAATCATTTTTATATTATAACTCTGT 1250

             254 GCATTGCCTGGCCTTCCTGTCTAGTCTCTCCTGTAAGCCAAAGAAATGAA  303
                 ||| | |  |  |  ||||     | |||| | | |||||| ||| ||
            1251 ATAATAGAGATAAGTCCATTTTTTAAAAATGTTTTCCCCAAACCATAAAA 1300

             304 CATTCCAAGGAGTTGGAAGTGAAGTCTATGATGTGAAACACTTTGCCTCC  353
                 |  |   | || ||||| |  || ||| || |||  |||||  || | |
            1301 CCCT.ATACAAGTTGTTCTAGTAACAATACATGAGAAAGATGT......C 1343

             354 TGTGTACTGTGTCATAAACAGATGAATAAACTGAATTTGTACTTT  398
                 ||| | ||| || |||||  |  || |||||| || ||
            1344 TATGTAGCTGAAAATAAAATGACGTCACAAGAC............ 1376
```